# EUROPEAN PATENT SPECIFICATION

(11) **EP 1 173 440 B1**
(45) Date of publication and mention of the grant of the patent: **15.09.2004**
(21) Application number: 00923532.6
(22) Date of filing: 20.04.2000
(51) Int. Cl.: C07D 471/16, A61K 31/395, A61P 43/00, C07D 498/16, C07D 513/16

(54) **TETRACYCLIC AZEPINOINDOLE COMPOUNDS AS 5-HT RECEPTOR LIGANDS**
TETRACYCLISCHE AZEPINOINDOL VERBINDUNGEN ALS 5-HT REZEPTOR LIGANDEN
COMPOSES D'AZEPINOINDOLE TETRACYCLIQUE UTILISES COMME LIGANDS RECEPTEURS DE 5-HT

(30) Priority: 23.04.1999 US 130811 P
(43) Date of publication of application: 23.01.2002
(73) Proprietor: PHARMACIA & UPJOHN COMPANY, Kalamazoo, Michigan 49001 (US)
(72) Inventor: HESTER, Jackson, B., Jr., Galesburg, MI 49053 (US); ROGERS, Bruce, N., Portage, MI 49024 (US); JACOBSEN, E., Jon, Kalamazoo, MI 49008 (US); ENNIS, Michael, Dalton, Portage, MI 49002 (US); ACKER, Brad, A., Kalamazoo, MI 49009 (US); VANDER VELDE, Susan, L., Kalamazoo, MI 49009 (US); FRANK, Kristine, E., Portage, MI 49002-5880 (US)
(74) Representative: Perry, Robert Edward
(86) International application number: PCT/US2000/010600
(87) International publication number: WO 2000/064899

(56) References cited:
- EP-A- 0 377 238
- US-A- 3 676 558
- US-A- 3 839 357

## Description

### PRIORITY OF INVENTION

This application claims priority from United States Provisional Application Number 60/130,881, filed 23 April 1999.

### FIELD OF THE INVENTION

The present invention provides tetracyclic 1,2,3,4,5,6-hexahydroazepino-[4,5-b]indole derivatives having a ring connecting position 6 (N-6) and position 7 (C-7), and more specifically, provides compounds of formula (I) described hereinbelow. These compounds are 5-HT ligands, and are useful for treating diseases wherein modulation of 5-HT activity is desired.

### BACKGROUND OF THE INVENTION

Serotonin has been implicated in a number of diseases and conditions which originate in the central nervous system. These include diseases and conditions related to sleeping, eating, perceiving pain, controlling body temperature, controlling blood pressure, depression, anxiety, schizophrenia, and other bodily states. R. W. Fuller, Biology of Serotonergic Transmission, 221 (1982); D. J. Boullin, Serotonin in Mental Abnormalities 1:316 (1978); J. Barchas, et al., Serotonin and Behavior, (1973). Serotonin also plays an important role in peripheral systems, such as the gastrointestinal system, where it has been found to mediate a variety of contractile, secretory, and electrophysiologic effects.

As a result of the broad distribution of serotonin within the body, there is a tremendous interest in drugs that affect serotonergic systems. In particular, receptor-specific agonists and antagonists are of interest for the treatment of a wide range of disorders, including anxiety, depression, hypertension, migraine, obesity, compulsive disorders, schizophrenia, autism, neurodegenerative disorders (e.g. Alzheimer's disease, Parkinsonism, and Huntington's chorea), and chemotherapy-induced vomiting. M. D. Gershon, et al., The Peripheral Actions of 5-Hydroxytryptamine, 246 (1989); P. R. Saxena, et al., Journal of Cardiovascular Pharmacology, 15:Supplement 7 (1990).

The major classes of serotonin receptors (5-HT₁₋₇) contain fourteen to eighteen separate receptors that have been formally classified. See Glennon, et al., *Neuroscience and Behavioral Reviews*, 1990, *14*, 35; and D. Hoyer, et al. *Pharmacol. Rev*. 1994, *46*, 157-203. Recently discovered information regarding subtype identity, distribution, structure, and function suggests that it is possible to identify novel, subtype specific agents, having improved therapeutic profiles (e.g. fewer side effects).

For example, the 5-HT₂ family of receptors is comprised of 5-HT_{2A}, 5-HT_{2B}, and 5-HT_{2C} subtypes, which have been grouped together on the basis of primary structure, secondary messenger system, and operational profile. All three subtypes are G-protein coupled, activate phospholipase C as a principal transduction mechanism, and contain a seven-transmembrane domain structure. There are distinct differences in the distribution of the three 5-HT₂ subtypes. The 5-HT_{2B} and 5-HT_{2A} receptors are widely distributed in the periphery, while the 5-HT_{2C} receptor has been found only in the central nervous system, being highly expressed in many regions of the human brain. See G. Baxter, et al. *Trends in Pharmacol*. *Sci*. 1995, *16*, 105-110.

Subtype 5-HT_{2A} has been associated with effects including vasoconstriction, platelet aggregation, and bronchoconstriction, while subtype 5-HT_{2C} has been associated with diseases that include depression, anxiety, obsessive compulsive disorder, panic disorders, phobias, psychiatric syndromes, and obesity. Very little is known about the pharmacologic role of the 5-HT_{2B} receptor. See F. Jenck, et al., *Exp*. *Opin. Invest. Drugs,* 1998, *7*, 1587-1599; M. Bos, et al., *J*. *Med*. *Chem*., 1997, *40*, 2762-2769; J.R. Martin, et al., *The Journal of Pharmacology and Experimental Therapeutics,* 1998, *286*, 913-924; S.M. Bromidge, et al., *J*. *Med*. *Chem*., 1998, *41*, 1598-1612; G.A. Kennett, *Drugs*, 1998, *1*, 4, 456-470; and A. Dekeyne, et al., *Neuropharmacology*, 1999, *38*, 415-423.

US-A-3,676,558, discloses compositions comprising specific 6-alkyl-1,2,3,4,5,6-hexahydroazepino[4,5-b]indole compounds. The compositions are reported to be useful to suppress hunger in mammals. This patent also discloses a method for inducing anorexia in obese subjects to produce weight loss. The azepino[4,5-b]indole compounds disclosed in this patent lack the ring connecting the 6-position and the 7-position that is present in the compounds of the instant invention.

US-A-3,839,357, discloses specific 1,2,3,4,5,6-hexahydroazepino[4,5-b]indole compounds, which are reported to have sedative or tranquilizing action. The azepino[4,5-b]indole compounds disclosed in this patent also lack the ring connecting the 6-position and the 7-position that is present in the compounds of the instant invention.

EP-A-0377238 discloses indolo-lactams which differ from the compounds of this invention by containing an oxo group on the hetero ring.

There is currently a need for pharmaceutical agents that are useful to treat diseases and conditions that are associated with 5-HT receptors.

### SUMMARY OF THE INVENTION

In accordance with the present invention, novel compounds which demonstrate useful biological activity, and particularly activity as 5-HT receptor ligands, are provided. Thus, the present invention provides a compound of formula I: 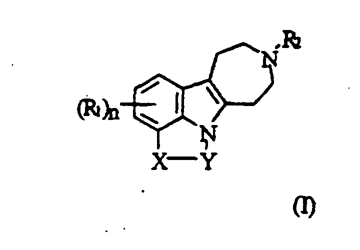 wherein,
each R₁ is independently hydroxy, nitro, halo, cyano, trifluoromethyl, trifluoromethoxy, C₁₋₇alkyl, C₁₋₇alkoxy, C₁₋₇alkanoyl, C₁₋ ₇alkoxycarbonyl, C₁₋₇alkanoyloxy, aryl, heteroaryl, -S(O)ₘNRₐR_{b}, NR_{c}R_{d}, -S(O)ₘRₑ, or -C(=O)NRₐR_{b}, wherein any C₁₋₇alkyl, C₁₋₇alkoxy, C₁₋₇alkanoyl, C₁₋₇alkoxycarbonyl, or C₁₋₇alkanoyloxy of R₁ is optionally partially unsaturated and is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋ ₇alkanoyloxy, -S(O)ₘRₑ, -S(O)ₘNRₐR_{b}, NR_{c}R_{d}, or -C(=O)NRₐR_{b};
R₂ is hydrogen, C₁₋₇alkyl, C₁₋₇alkanoyl, arylcarbonyl, aryl, (aryl)C₁₋₇alkyl, C₁₋₇alkoxycarbonyl, aryloxycarbonyl, arylsulfonyl, or (aryl)C₁₋ ₇alkoxycarbonyl;
X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo (=O), thioxo (=S), -NR_{q}Rᵣ, -S(O)ₚRₛ, or -ORₜ, or with one or two substituents independently selected from the group consisting of C₁₋₇alkyl, (C₁₋₇alkoxy)C₁₋ ₇alkyl, aryl, (aryl)C₁₋₇alkyl, heteroaryl, (heteroaryl)C₁₋₇alkyl, and (aryl)oxyC₁₋₇alkyl; or wherein the chain is optionally substituted on a carbon with a 4, 5, or 6 membered spirocyclic carbon ring; or wherein the chain is optionally substituted on two adjacent atoms with a 2, 3, or 4 membered alkylene chain (e.g. -CH₂CH₂-, -CH₂CH₂CH₂-, or -CH₂CH₂CH₂CH₂-) forming a ring that is fused to the ring comprising X and Y;
each m is independently 0, 1, or 2;
n is 0, 1, 2, or 3;
p is 0, 1, or 2;
each Rₐ and R_{b} is independently hydrogen, C₁₋₇alkyl, aryl, (aryl)C₁₋₇alkyl, heteroaryl, or (heteroaryl)C₁₋₇alkyl; or Rₐ and R_{b} together with the nitrogen to which they are attached form a pyrrolidino, piperidino, morpholino, or thiomorpholino ring;
each R_{c} and R_{d} is independently hydrogen, C₁₋₇alkyl, C₁₋₇alkanoyl, C₁₋₇alkoxycarbonyl, aryl, (aryl)C₁₋₇alkyl, heteroaryl, (heteroaryl)C₁₋₇alkyl, arylcarbonyl, heteroarylcarbonyl, aryloxycarbonyl, or heteroaryloxycarbonyl; or R_{c} and R_{d} together with the nitrogen to which they are attached form a pyrrolidino, piperidino, morpholino, or thiomorpholino ring;
each Rₑ is independently hydrogen, C₁₋₇alkyl, aryl, (aryl)C₁₋₇alkyl, heteroaryl, or (heteroaryl)C₁₋₇alkyl;
R_{f} is hydrogen, C₁₋₇alkyl, aryl, (aryl)C₁₋₇alkyl, heteroaryl, (heteroaryl)C₁₋₇alkyl, or is a bond to a 2, 3, or 4 membered alkylene chain that forms a ring that is fused to the ring comprising X and Y;
each R_{q} and Rᵣ is independently hydrogen, C₁₋₇alkyl, aryl, (aryl)C₁₋₇alkyl, heteroaryl, or (heteroaryl)C₁₋₇alkyl; or R_{q} and Rᵣ together with the nitrogen to which they are attached form a pyrrolidino, piperidino, morpholino, or thiomorpholino ring;
Rₛ is C₁₋₇alkyl, aryl, (aryl)C₁₋₇alkyl, heteroaryl, or (heteroaryl)C₁₋ ₇alkyl; and
Rₜ is hydrogen, C₁₋₇alkyl, aryl, (aryl)C₁₋₇alkyl, heteroaryl, or (heteroaryl)C₁₋₇alkyl;
wherein any aryl or heteroaryl ring of R₁, R₂, X, Y, Rₐ-R_{f}, or R_{q}-Rₜ is optionally substituted with one or more (e.g. 1, 2, 3, or 4) substituents independently selected from halo, hydroxy, cyano, nitro, trifluoromethyl, trifluoromethoxy, C₁₋₇alkyl, C₁₋₇alkoxy, phenyl, sulfonyl, NRⱼRₖ, or-C(=O)NRⱼRₖ; wherein each Rⱼ and Rₖ is independently hydrogen, C₁₋₇alkyl, C₁₋₇alkanoyl, C₁₋₇alkoxycarbonyl, aryl, (aryl)C₁₋₇alkyl, arylcarbonyl, or aryloxycarbonyl; or Rⱼ and Rₖ together with the nitrogen to which they are attached form a pyrrolidino, piperidino, morpholino, or thiomorpholino ring;
or a pharmaceutically acceptable salt thereof;
provided Y is not oxy, thio, sulfinyl, or NR_{f}; and
provided X and Y together are not a 2-membered unsaturated chain; and
provided no carbon of X and Y is bonded to more than one oxy, thio, sulfinyl, or NR_{f}.

In another aspect, the present invention also provides:
a pharmaceutical composition comprising a compound of formula I, or a pharmaceutically acceptable salt thereof, and a pharmaceutically acceptable excipient (the composition preferably comprises a therapeutically effective amount of the compound or salt),
a method for treating a disease or condition in a mammal (e.g. a human) wherein a 5-HT receptor is implicated and modulation of a 5-HT function is desired comprising administering a therapeutically effective amount of a compound of formula I, or a pharmaceutically acceptable salt thereof to the mammal,
a method for treating or preventing a disease or disorder of the central nervous system in a mammal comprising administering a therapeutically effective amount of a compound of formula I, or a pharmaceutically acceptable salt thereof to the mammal,
a compound of formula (I) or a pharmaceutically acceptable salt thereof for use in medical diagnosis or therapy (e.g. the treatment or prevention of 5-HT related disease such as anxiety, obesity, depression, or a stress related disease),
the use of a compound of formula I, or a pharmaceutically acceptable salt thereof to prepare a medicament useful for treating or preventing a disease or disorder of the central nervous system in a mammal, and
a method for modulating 5-HT receptor function, comprising administering an effective modulatory amount of a compound of formula I, or a pharmaceutically acceptable salt thereof.

The invention also provides novel intermediates and processes disclosed herein that are useful for preparing compounds of formula I.

### DESCRIPTION OF THE FIGURES

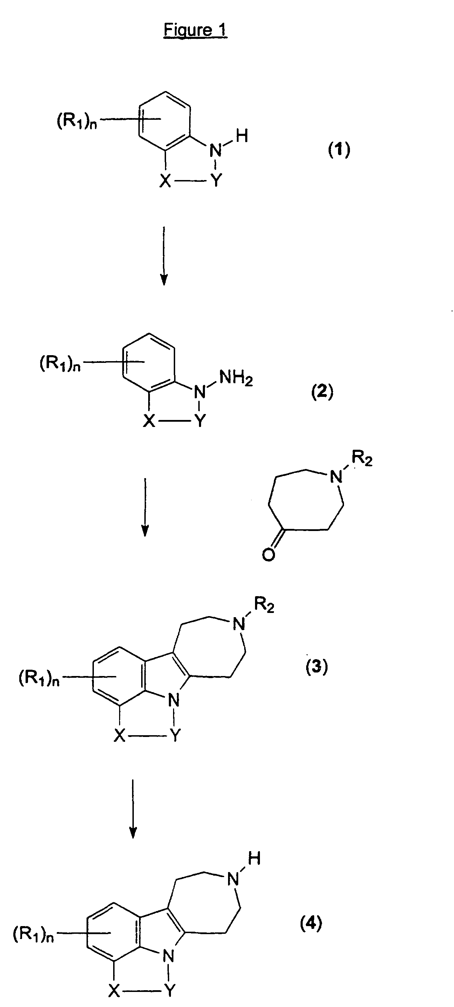

Figures 1-6 illustrate synthetic processes and intermediates useful for preparing compounds of the invention.

### DETAILED DESCRIPTION OF THE INVENTION

The compounds of the invention are useful for treating or preventing diseases or disorders of the central nervous system. Specific diseases or disorders of the central nervous system for which a compound of formula I may have activity include, but are not limited to: obesity, depression, schizophrenia, schizophreniform disorder, schizoaffective disorder, delusional disorder, a stress related disease (e.g. general anxiety disorder), panic disorder, a phobia, obsessive compulsive disorder, post-traumatic-stress syndrome, immune system depression, a stress induced problem with the urinary, gastrointestinal or cardiovascular system (e.g., stress incontinence), neurodegenerative disorders, autism, chemotherapy-induced vomiting, hypertension, migraine, headaches, cluster headaches, sexual dysfunction in a mammal (e.g. a human), addictive disorder and withdrawal syndrome, an adjustment disorder, an age-associated learning and mental disorder, anorexia nervosa, apathy, an attention-deficit disorder due to general medical conditions, attention-deficit hyperactivity disorder, behavioral disturbance (including agitation in conditions associated with diminished cognition (e.g., dementia, mental retardation or delirium)), bipolar disorder, bulimia nervosa, chronic fatigue syndrome, conduct disorder, cyclothymic disorder, dysthymic disorder, fibromyalgia and other somatoform disorders, generalized anxiety disorder, an inhalation disorder, an intoxication disorder, movement disorder (e.g., Huntington's disease or Tardive Dyskinesia), oppositional defiant disorder, peripheral neuropathy, post-traumatic stress disorder, premenstrual dysphoric disorder, a psychotic disorder (brief and long duration disorders, psychotic disorder due to medical condition, psychotic disorder NOS), mood disorder (major depressive or bipolar disorder with psychotic features) seasonal affective disorder, a sleep disorder, a specific development disorder, agitation disorder, selective serotonin reuptake inhibition (SSRI) "poop out" syndrome or a Tic disorder (e.g., Tourette's syndrome).

The following definitions are used, unless otherwise described: halo is fluoro, chloro, bromo, or iodo. Alkyl, alkoxy, etc. denote both straight and branched groups; but reference to an individual radical such as "propyl" embraces only the straight chain radical, a branched chain isomer such as "isopropyl" being specifically referred to. When alkyl can be partially unsaturated, the alkyl chain may comprise one or more (e.g. 1, 2, 3, or 4) double or triple bonds in the chain.

Aryl denotes a phenyl radical or an ortho-fused bicyclic carbocyclic radical having about nine to ten ring atoms in which at least one ring is aromatic. Heteroaryl denotes a radical of a monocyclic aromatic ring containing five or six ring atoms consisting of carbon and 1, 2, 3, or 4 heteroatoms each selected from the group consisting of non-peroxide oxygen, sulfur, and N(X) wherein X is absent or is H, O, C₁₋₄alkyl, phenyl or benzyl, as well as a radical of an ortho-fused bicyclic heterocycle of about eight to ten ring atoms derived therefrom, particularly a benz-derivative or one derived by fusing a propylene, trimethylene, or tetramethylene diradical thereto.

It will be appreciated by those skilled in the art that compounds of the invention having a chiral center may exist in and be isolated in optically active and racemic forms. Some compounds may exhibit polymorphism. It is to be understood that the present invention encompasses any racemic, optically-active, polymorphic, tautomeric, or stereoisomeric form, or mixture thereof, of a compound of the invention, which possesses the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine 5-HT activity using the standard tests which are well known in the art.

The carbon atom content of various hydrocarbon-containing moieties is indicated by a prefix designating the minimum and maximum number of carbon atoms in the moiety, i.e., the prefix C ᵢ₋ⱼ indicates a moiety of the integer "i" to the integer "j" carbon atoms, inclusive. Thus, for example, C₁₋ ₇alkyl refers to alkyl of one to seven carbon atoms, inclusive.

The compounds of the present invention are generally named according to the IUPAC or CAS nomenclature system. Abbreviations which are well known to one of ordinary skill in the art may be used (e.g. "Ph" for phenyl, "Me" for methyl, "Et" for ethyl, "h" for hour or hours and "rt" for room temperature).

Specific and preferred values listed below for radicals, substituents, and ranges, are for illustration only; they do not exclude other defined values or other values within defined ranges for the radicals and substituents

Specifically, C₁₋₇alkyl can be methyl, ethyl, propyl, isopropyl, butyl, iso-butyl, sec-butyl, pentyl, 3-pentyl, hexyl, or heptyl; C₁₋₇alkoxy can be methoxy, ethoxy, propoxy, isopropoxy, butoxy, iso-butoxy, sec-butoxy, pentoxy, 3-pentoxy, hexyloxy, 1-methylhexyloxy, or heptyloxy; C₁₋₇alkanoyl can be acetyl, propanoyl, butanoyl, pentanoyl, 4-methylpentanoyl, hexanoyl, or heptanoyl; C₁₋₇alkoxycarbonyl can be methoxycarbonyl, ethoxycarbonyl, propoxycarbonyl, isopropoxycarbonyl, butoxycarbonyl, pentoxycarbonyl, hexyloxycarbonyl, or heptyloxycarbonyl; C₁₋₇alkanoyloxy can be acetoxy, propanoyloxy, butanoyloxy, isobutanoyloxy, pentanoyloxy, hexanoyloxy, or heptanoyloxy; aryl can be phenyl, indenyl, or naphthyl; and heteroaryl can be furyl, imidazolyl, triazolyl, triazinyl, oxazoyl, isoxazoyl, thiazolyl, isothiazoyl, pyrazolyl, pyrrolyl, pyrazinyl, tetrazolyl, pyridyl, (or its N-oxide), thienyl, pyrimidinyl (or its N-oxide), indolyl, isoquinolyl (or its N-oxide) or quinolyl (or its N-oxide).

A specific value for R₁ is hydroxy, nitro, halo, cyano, trifluoromethyl, trifluoromethoxy, C₁₋₇alkyl, C₁₋₇alkoxy, C₁₋₇alkanoyl, C₁₋ ₇alkoxycarbonyl, C₁₋₇alkanoyloxy, aryl, heteroaryl, -S(O)ₘNRₐR_{b}, NR_{c}R_{d}, -S(O)ₘRₑ, or -C(=O)NRₐR_{b}, wherein any C₁₋₇alkyl, C₁₋₇alkoxy, C₁₋₇alkanoyl, C₁₋₇alkoxycarbonyl, or C₁₋₇alkanoyloxy of R₁ is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋₇alkanoyloxy, -S(O)ₘRₑ, -S(O)ₘNRₐR_{b}, NR_{c}R_{d}, or -C(=O)NRₐR_{b}.

A specific value for R₁ is hydroxy, nitro, halo, cyano, trifluoromethyl, trifluoromethoxy, C₁₋₇alkyl, C₁₋₇alkoxy, C₁₋₇alkanoyl, C₁₋ ₇alkoxycarbonyl, C₁₋₇alkanoyloxy, aryl, heteroaryl, -S(O)ₘNRₐR_{b}, NR_{c}R_{d}, - S(O)ₘRₑ, or -C(=O)NRₐR_{b}, wherein any C₁₋₇alkyl or C₁₋₇alkoxy of R₁ is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋₇alkanoyloxy, -S(O)ₘRₑ, -S(O)ₘNRₐR_{b}, NR_{c}R_{d}, or -C(=O)NRₐR_{b}.

A specific value for R₁ is hydroxy, nitro, halo, cyano, trifluoromethyl, trifluoromethoxy, C₁₋₇alkyl, C₁₋₇alkoxy, C₁₋₇alkanoyl, C₁₋ ₇alkoxycarbonyl, C₁₋₇alkanoyloxy, aryl, heteroaryl, -S(O)ₘNRₐR_{b}, NR_{c}R_{d}, -S(O)ₘRₑ, or -C(=O)NRₐR_{b}, wherein any C₁₋₇alkyl is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋₇alkanoyloxy, -S(O)ₘRₑ, -S(O)ₘNRₐR_{b}, NR_{c}R_{d}, or -C(=O)NRₐR_{b}.

A specific value for R₁ is hydroxy, nitro, halo, cyano, trifluoromethyl, trifluoromethoxy, C₁₋₇alkyl, C₁₋₇alkoxy, C₁₋₇alkanoyl, C₁₋ ₇alkoxycarbonyl, C₁₋₇alkanoyloxy, aryl, heteroaryl, -S(O)ₘNRₐR_{b}, NR_{c}R_{d}, - S(O)ₘRₑ, or -C(=O)NRₐR_{b}.

A specific value for R₁ is independently C₁₋₇alkyl, C₁₋₇alkoxy, trifluoromethyl, or halo.

A specific value for R₂ is hydrogen.

A specific value for R₂ is C₁₋₄alkyl, C₁₋₄alkanoyl, arylcarbonyl, (aryl)C₁₋₂alkyl, C₁₋₄alkoxycarbonyl, aryloxycarbonyl, arylsulfonyl, or (aryl)methoxycarbonyl, wherein any aryl is optionally substituted with 1, 2, or 3 substituents independently selected from C₁₋₄alkyl and trifluoromethyl.

A specific value for R₂ is methyl, ethyl, propyl, isopropyl, acetyl, *tert*-butoxycarbonyl, benzyloxycarbonyl, benzyl, or *p*-toluenesulfonyl.

A specific value for n is 1, 2, or 3.

A specific value for n is 0.

A specific group of compounds are compounds of formula (I) wherein n is 0. It will be clear to one skilled in the art that when n is 0, the benz ring of the indole in formula (I) is substituted with hydrogens.

A specific group of compounds are compounds of formula I wherein X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo in (=O), thioxo (=S), -NR_{q}Rᵣ, -S(O)ₚRₛ, or -ORₜ, or with one or two substituents independently selected from the group consisting of C₁₋₇alkyl, (C₁₋₇alkoxy)C₁₋ ₇alkyl, aryl, (aryl)C₁₋₇alkyl, heteroaryl, (heteroaryl)C₁₋₇alkyl, and (aryl)oxyC₁₋₇alkyl.

A specific group of compounds are compounds of formula I wherein X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo (=O), thioxo (=S), -NR_{q}Rᵣ, -S(O)ₚRₛ, or -ORₜ.

A specific group of compounds are compounds of formula I wherein X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl.

A specific group of compounds are compounds of formula I wherein X and Y together are a 2, 3, or 4 membered chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo (=O), hydroxy, or C₁₋₇alkoxy, or with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl and (aryl)oxyC₁₋₇alkyl; and wherein the chain is optionally substituted on two adjacent atoms with a 2, 3, or 4 membered alkylene chain forming a ring that is fused to the ring comprising X and Y.

A specific group of compounds are compounds of formula I wherein X and Y together are a 2, 3, or 4 membered chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo (=O), hydroxy, (aryl)oxy, heteroaryl(oxy), or C₁₋₇alkoxy, or with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl; and wherein the chain is optionally substituted on two adjacent atoms with a 2, 3, or 4 membered alkylene chain forming a ring that is fused to the ring comprising X and Y.

A specific group of compounds are compounds of formula I wherein X and Y together are a 2, 3, or 4 membered carbon chain wherein the chain is optionally substituted on each carbon with oxo (=O), hydroxy, or C₁₋₇alkoxy, or with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl and (aryl)oxyC₁₋₇alkyl.

A specific group of compounds are compounds of formula I wherein X and Y together are a 2, 3, or 4 membered carbon chain wherein the chain is optionally substituted on each carbon with oxo (=O), hydroxy, (aryl)oxy, heteroaryl(oxy) or C₁₋₇alkoxy, or with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl.

A specific group of compounds are compounds of formula I wherein X and Y together are a 2 or 3 membered carbon chain optionally substituted on each carbon with oxo or hydroxy, or with one or two C₁₋₇alkyl.

A specific group of compounds are compounds of formula I wherein X is -O-, -S-, or -C(R_{g})(Rₕ)-, wherein R_{g} and Rₕ are each independently hydrogen, C₁₋₇alkyl, (C₁₋₇alkoxy)C₁₋₇alkyl, aryl, (aryl)C₁₋₇alkyl, heteroaryl, (heteroaryl)C₁₋₇alkyl or (aryl)oxyC₁₋₇alkyl, or R_{g} and Rₕ together are oxo.

A specific group of compounds are compounds of formula I wherein Y is -C(R_{g})(Rₕ)-, -C(R_{g})(Rₕ)C(R_{g})(Rₕ)-, -C(R_{g})(Rₕ)C(R_{g})(Rₕ)C(R_{g})(Rₕ)-, -C(R_{g})(Rₕ)C(=O)-, -C(R_{g})(Rₕ)C(R_{g})(Rₕ)C(=O)-, -C(=O)C(R_{g})(Rₕ)-, or -C(=O)C(R_{g})(Rₕ)C(R_{g})(Rₕ)-, and each R_{g} and Rₕ is independently hydrogen or C₁₋₇alkyl.

A specific group of compounds are compounds wherein X is -O-, - S-, or -C(R_{g})(Rₕ)-; and Y is -C(R_{g})(Rₕ)C(=O)-, or -C(R_{g})(Rₕ)C(R_{g})(Rₕ)-, wherein each R_{g} and Rₕ is independently hydrogen or C₁₋₇alkyl.

A specific group of compounds are compounds wherein X is -O- or -S-; and Y is -C(R_{g})(Rₕ)C(=O)-, -C(=O)C(R_{g})(Rₕ)-, or -C(R_{g})(Rₕ)C(R_{g})(Rₕ)-, wherein each R_{g} and Rₕ is independently hydrogen or C₁₋₇alkyl.

A specific group of compounds are compounds wherein X and Y together are -CH(R_{g})CH(R_{g})-, -CH(R_{g})CH(R_{g})CH(R_{g})-,--CH(R_{g})CH(R_{g})CH(R_{g})CH(R_{g})-, -C(R_{g})=C(R_{g})CH(R_{g})-, -C(R_{g})=C(R_{g})CH(R_{g})CH(R_{g})-, -CH(R_{g})C(R_{g})=C(R_{g})CH(R_{g})-, -O-CH(R_{g})CH(R_{g})-, -O-CH(R_{g})CH(R_{g})CH(R_{g})-, -S-CH(R_{g})CH(R_{g})-, -S-CH(R_{g})CH(R_{g})CH(R_{g})-, -S(O)-CH(R_{g})CH(R_{g})-, -S(O)-CH(R_{g})CH(R_{g})CH(R_{g})-, -S(O)₂-CH(R_{g})CH(R_{g})-, -S(O)₂-CH(R_{g})CH(R_{g})CH(R_{g})-, -NR_{f}-CH(R_{g})CH(R_{g})-, -NR_{f}-CH(R_{g})CH(R_{g})CH(R_{g})-, -CH(R_{g})C(=O)-, -CH(R_{g})CH(R_{g})C(=O)-, -CH(R_{g})CH(R_{g})CH(R_{g})C(=O)-, -CH(R_{g})OC(=O)-, - CH(R_{g})CH(R_{g})OC(=O)-, -OCH(R_{g})C(=O)-, or -OCH(R_{g})CH(R_{g})C(=O)-; wherein each R_{g} is independently hydrogen, C₁₋₇alkyl, aryl, (aryl)C₁₋₇alkyl or (aryl)oxyC₁₋₇alkyl.

A specific group of compounds are compounds wherein X and Y together are -CH(R_{g})CH₂-, -CH(R_{g})CH(R_{g})CH₂-, -CH(R_{g})CH(R_{g})CH(R_{g})CH₂-, -CH=CHCH₂-, -CH=CHCH(R_{g})CH₂-, -CH(R_{g})CH=CHCH₂-, -O-CH₂CH₂-, -O-CH₂CH(R_{g})CH₂-, -S-CH₂CH₂-, -S-CH₂CH(R_{g})CH₂-, -S(O)-CH₂CH₂-, -S(O)-CH₂CH(R_{g})CH₂-, -S(O)₂-CH₂CH₂-, -S(O)₂-CH₂CH(R_{g})CH₂-, -NR_{f}-CH₂CH₂-, -NR_{f}-CH₂CH(R_{g})CH₂-, -CH₂C(=O)-, -CH(R_{g})CH₂C(=O)-, -CH(R_{g})CH(R_{g})CH₂C(=O)-, -CH₂OC(=O)-, -CH(R_{g})CH₂OC(=O)-, -OCH₂C(=O)-, or -OCH₂CH₂C(=O)-; wherein each R_{g} is independently -NR_{q}Rᵣ, -S(O)ₚRₛ, or -ORₜ.

A specific group of compounds are compounds wherein X and Y together are -CH(R_{g})CH(R_{g})-, -CH(R_{g})CH(R_{g})CH(R_{g})-, -C(R_{g})=C(R_{g})CH(R_{g})-, -O-CH(R_{g})CH(R_{g})-, -S-CH(R_{g})CH(R_{g})-, -S(O)-CH(R_{g})CH(R_{g})-, -S(O)₂-CH(R_{g})CH(R_{g})-, -NR_{f}-CH(R_{g})CH(R_{g})-, -CH(R_{g})C(=O)-, -CH(R_{g})CH(R_{g})C(=O)-, -CH(R_{g})OC(=O)-, -OCH(R_{g})C(=O)-; wherein each R_{g} is independently hydrogen or C₁₋₇alkyl.

A specific group of compounds are compounds wherein X and Y together are -CH(R_{g})CH(R_{g})-, -CH(R_{g})CH(R_{g})CH(R_{g})-, -C(R_{g})=C(R_{g})CH(R_{g})-, -O-CH(R_{g})CH(R_{g})-, -S-CH(R_{g})CH(R_{g})-, -S(O)-CH(R_{g})CH(R_{g})-, -S(O)₂-CH(R_{g})CH(R_{g})-, -NR_{f}-CH(R_{g})CH(R_{g})-, or -CH(R_{g})CH(R_{g})C(=O)-; wherein each R_{g} is independently hydrogen, C₁₋₇alkyl, or together with an R_{g} on an adjacent carbon atom forms a fused 4, 5, or 6, membered carbocyclic ring.

A specific group of compounds are compounds wherein X and Y together are -CH(R_{g})CH(R_{g})-, -CH(R_{g})CH(R_{g})CH(R_{g})-, -O-CH(R_{g})CH(R_{g})-, -S-CH(R_{g})CH(R_{g})-; wherein each R_{g} is independently hydrogen, C₁₋₇alkyl, aryl, or (aryl)C₁₋₇alkyl.

A specific group of compounds are compounds wherein X and Y together are -CH₂CH₂CH₂-, -CH₂CH₂C(CH₃)H-, -CH₂C(CH₃)HCH₂-, - C(CH₃)HCH₂CH₂-, -CH₂CH₂-, -CH₂C(CH₃)H-, -C(CH₃)HC(CH₃)H-,-- CH(R_{g})CH(R_{g})-, -O-CH₂CH₂-, -O-C(CH₃)HCH₂-, or -S-CH₂CH₂-.

A specific group of compounds are compounds wherein X and Y together are -CH₂CH₂-, -CH₂CH₂CH₂-, -CH=CHCH₂-, -O-CH₂CH₂-, - S-CH₂CH₂-, -S(O)-CH₂CH₂-, -S(O)₂-CH₂CH₂-, -NR_{f}-CH₂CH₂-, -CH₂C(=O)-, - CH₂CH₂C(=O)-, -CH₂OC(=O)-, or -OCH₂C(=O)-.

A specific group of compounds are compounds of formula (I) wherein X and Y together are -CH(R_{g})CH₂-, -CH(R_{g})CH(R_{g})CH₂-, or - O-CH₂CH₂-, wherein each R_{g} is independently -NR_{q}Rᵣ, -S(O)ₚRₛ, -ORₜ, C₁₋₇alkyl, (C₁₋₇alkoxy)C₁₋₇alkyl, aryl, (aryl)C₁₋₇alkyl, heteroaryl, (heteroaryl)C₁₋₇alkyl, or (aryl)oxyC₁₋₇alkyl.

A specific group of compounds are compounds of formula (I) wherein X and Y together are -C(=O)CH₂-, -CH₂C(=O)-, -C(=S)CH₂-, - CH₂C(=S)-, -C(=O)CH₂CH₂-, -CH₂C(=O)CH₂-, -CH₂CH₂C(=O)-, - C(=S)CH₂CH₂-, -CH₂C(=S)CH₂-, or -CH₂CH₂C(=S)-.

A specific group of compounds are compounds wherein n is 1 and R₁ is C₁₋₇alkyl, C₁₋₇alkoxy, or halo.

A specific group of compounds are compounds wherein n is 1 and R₁ is methyl, methoxy, chloro, or fluoro.

A specific group of compounds are compounds wherein X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo (=O), hydroxy, or C₁₋₇alkoxy, or with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl; or wherein the chain is optionally substituted on a carbon with a 4, 5, or 6 membered spirocyclic carbon ring; or wherein the chain is optionally substituted on two adjacent atoms with a 2, 3, or 4 membered alkylene chain (e.g. -CH₂CH₂-, -CH₂CH₂CH₂-, or - CH₂CH₂CH₂CH₂-) forming a ring that is fused to the ring comprising X and Y;

When X and Y together, or R₁, is a "partially unsaturated" group, such group may comprise one or more (e.g. 1 or 2) carbon-carbon double or triple bonds. For example, when R¹ is a partially unsaturated C₁₋₇alkyl, it can be vinyl, allyl, 1-propenyl, 2-propenyl, 1-butenyl, 2-butenyl, 3-butenyl, 1,3-butadienyl, 1-pentenyl, 2-pentenyl, 3-pentenyl, 4-pentenyl, 1-hexenyl, 2-hexenyl, 3-hexenyl, 4-hexenyl, 2,4-hexadienyl, 5-hexenyl, ethynyl, 1-propynyl, 2-propynyl, 1-butynyl, 2-butynyl, 3-butynyl, 1-pentynyl, 2-pentynyl, 3-pentynyl, 4-pentynyl, 5-hexene-1-ynyl, 2-hexynyl, 3-hexynyl, 3-hexen-5-ynyl, 4-hexynyl, or 5-hexynyl.

Specifically, the invention also provides a method for treating or preventing anxiety, obesity, depression, schizophrenia, a stress related disease (e.g. general anxiety disorder), panic disorder, a phobia, obsessive compulsive disorder, post-traumatic-stress syndrome, immune system depression, a stress induced problem with the gastrointestinal or cardiovascular system, or sexual dysfunction in a mammal (e.g. a human) comprising administering a therapeutically effective amount of a compound of formula I, or a pharmaceutically acceptable salt thereof to the mammal.

Specifically, the invention also provides a method of treating or preventing anxiety, obesity, depression, or a stress related disease, comprising administering to a mammal (e.g. a human) in need of such treatment, a therapeutically effective amount of a compound of formula (I) or a pharmaceutically acceptable salt thereof.

Specifically, the invention also provides the use of a compound of formula (I) or a pharmaceutically acceptable salt thereof to prepare a medicament for treating or preventing anxiety, obesity, depression, schizophrenia, a stress related disease (e.g. general anxiety disorder), panic disorder, a phobia, obsessive compulsive disorder, post-traumatic-stress syndrome, immune system depression, a stress induced problem with the gastrointestinal or cardiovascular system, or sexual dysfunction in a mammal (e.g. a human),.

Specifically, the invention also provides the use of a compound of formula (I) or a pharmaceutically acceptable salt thereof to prepare a medicament for treating or preventing anxiety, obesity, depression, or a stress related disease in a mammal (e.g. a human).

The invention also provides processes and intermediates useful for preparing compounds of formula (I). For example, an intermediate useful for preparing a compound of formula (I) wherein R₂ is hydrogen, is a corresponding compound of formula (I) wherein R₂ is a suitable protecting group. Thus the invention provides a compound of formula (I) wherein R₂ is a suitable protecting group, and wherein R₁, X, Y, and n have any of the values, specific values or prefered values defined herein. Suitable amine protecting groups, as well as methods for their preparation and removal are well known in the art, for example, see Greene, T.W.; Wutz, P.G.M. "Protecting Groups In Organic Synthesis" third edition, 1999, New York, John Wiley & sons, Inc. Prefered protecting groups include benzyloxycarbonyl (CBZ) and benzoyl.

The invention also provides intermediate compounds of formula 3, 9, 10, 11, 13, 15, and 17-20 as shown in Figures, 1-6, wherein R₂ is a protecting group.

The invention also provides intermediate salts that are useful for preparing or purifying compounds of formula (I). Suitable methods for preparing salts are known in the art and are disclosed herein. For example, the preparation of an oxylate salt is shown in Example 41. As will be apparent to one skilled in the art, such salts can be converted to the corresponding free-base or to another salt using known methods.

For example, compounds of formula I wherein R₂ is C₁₋₄alkyl, C₁₋₄alkanoyl, arylcarbonyl, (aryl)C₁₋₂alkyl, C₁₋₄alkoxycarbonyl, aryloxycarbonyl, arylsulfonyl, or (aryl)methoxycarbonyl, wherein any aryl is optionaly substituted with 1, 2, or 3 substituents independently selected from C₁₋₄alkyl and trifluoromethyl, are particularly useful as intermediates for preparing corresponding compounds of formula I wherein R₂ is hydrogen. Preferred compounds of formula I that are useful for preparing compounds of formula I wherein R₂ is hydrogen are compounds wherein R₂ is methyl, ethyl, propyl, isopropyl, acetyl, *tert*-butoxycarbonyl, benzyloxycarbonyl, benzyl, or *p*toluenesulfonyl.

The invention also provides a method for preparing a compound of formula (I) wherein R₂ is hydrogen comprising deprotecting a corresponding compound of formula (I) wherein R₂ is a suitable nitrogen protecting group.

Compounds of the invention can generally be prepared using the synthetic schemes illustrated in Figures 1-6. Starting materials can be prepared by procedures described in these schemes or by procedures that would be well known to one of ordinary skill in organic chemistry. The variables used in the Schemes are as defined below or as in the claims.

Compounds of formula I can be prepared by reactions outlined in Figure 1. Step 1 involves formation of intermediate N-nitroso compounds by treatment with isoamylnitrite or other standard acid catalyzed N-nitrosation conditions. The resulting N-nitroso compounds are directly reduced to their corresponding hydrazines (2) by treatment with lithium aluminum hydride in a suitable solvent such as tetrahydrofuran. In step 2, hydrazines (2) are condensed by acid catalysis with 1-benzoylhexahydroazepine, which is available by the process described in *J. Org. Chem*., Vol. 33, pp 3187-95 (1968), or with benzyl 4-oxo-1-azepanecarboxylate, the synthesis of which is described in the experimental section. Fischer/Indole cyclization of the crude hydrazines provides the desired azepinoindoles (3). The Fischer/Indole cyclization can be effected with a variety of acids such as formic acid, acetic acid, trifluoroacetic acid, aqueous hydrochloric acid, aqueous sulfuric acid, or polyphosphoric acid. Step 3 is effected by either catalytic hydrogenolysis when R₂ is benzyl or benzyloxycarbonyl, or by base catalyzed hydrolysis in a suitable solvent such as ethylene glycol when R₂ is benzoyl. Azepinoindoles 4 (wherein when R₂ is hydrogen) can conveniently be isolated as their hydrochloride salts.

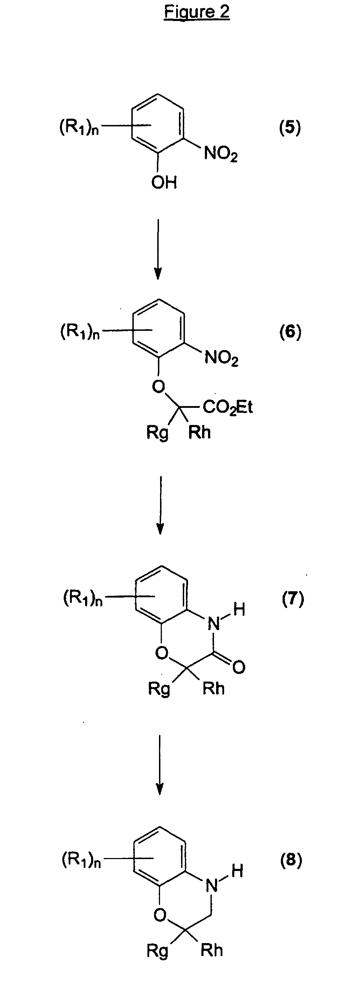

It will be apparent to those skilled in the art that many of the requisite amines (1) are commercially available or known in the literature. The necessary 3,4-dihydro-1(2H)-quinolinylamines required for Examples 1-13 are known compounds. Indolines required for the synthesis of Examples 14-18 are either commercially available or readily prepared from known indoles following the procedure described in *Synthesis* pp. 859-60 (1977). For the benzmorpholines and benzthiomorpholines required for Examples 19-25, the synthetic route shown in Figure 2 was followed. Nitrophenols 5 were alkylated with ethyl bromoacetate derivatives to afford 6. The nitro moiety is then reduced with Pd/C in the presence of hydrogen in a suitable solvent such as ethanol. In situ cyclization gives amide 7, which is then reduced with borane to provide the required amines 8.

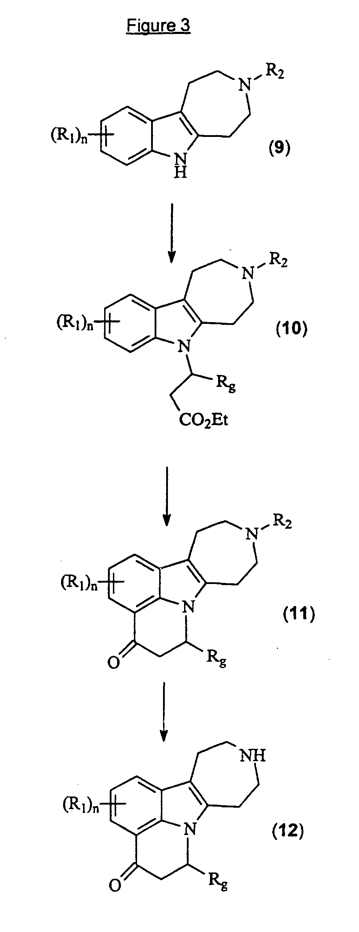

Compounds of formula I can also be prepared by the reactions outlined in Figure 3. Azapinoindoles 9 are known in the literature (*J Med Chem*., 1968, *11*, 101-106) and can participate in a Michael conjugate addition into ethyl acrylate, or a derivative thereof, in the presence of a suitable base such as cesium carbonate. Base-catalyzed hydrolysis of esters 10 gives crude acids which then undergo intramolecular Friedel-Crafts acylation in an acidic media (e.g. polyphosphoric acid or Eaton's reagent). When R₂ is benzoyl, azepinoindoles 12 can be obtained from base-catalyzed hydrolysis in tetrahydrofuran/methanol.

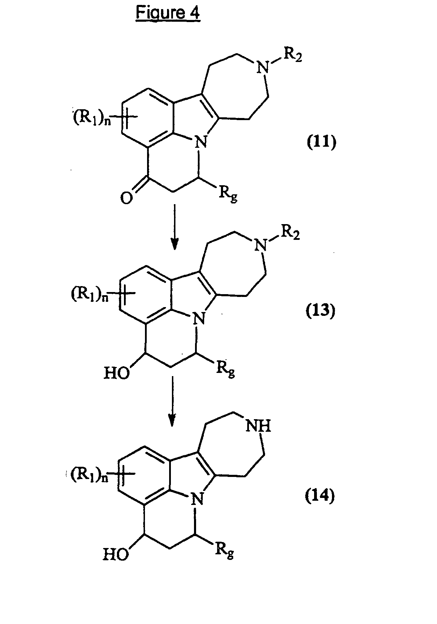

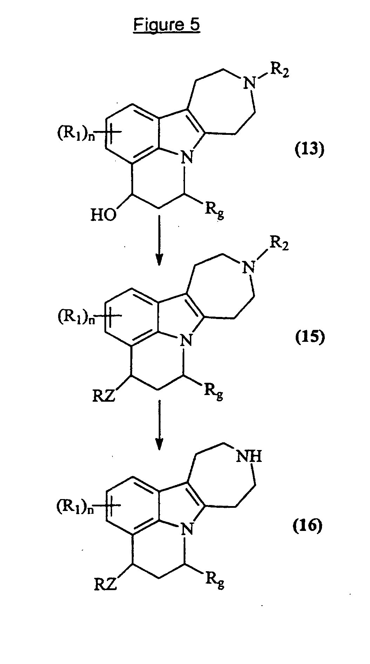

Aryl ketones 11 can be used as intermediates in the synthesis of additional compounds of formula I as shown in Figure 4. The ketone moiety is reduced with sodium borohydride to give alcohols 13. When R₂ is benzoyl, base-catalyzed hydrolysis of alcohols 13 gives azepinoindoles 14. Alternatively, alcohols 13 can also be used as intermediates in the synthesis of additional compounds of formula I as shown in Figure 5. Alcohols 13 can be alkylated with an alkyl halide in the presence of sodium hydride or with phenols via Mitsunobu reaction conditions. The use of thiols or amines in the Mitsunobu reaction can give additional derivatives. Removal of R₂ gives azepinoindoles 16.

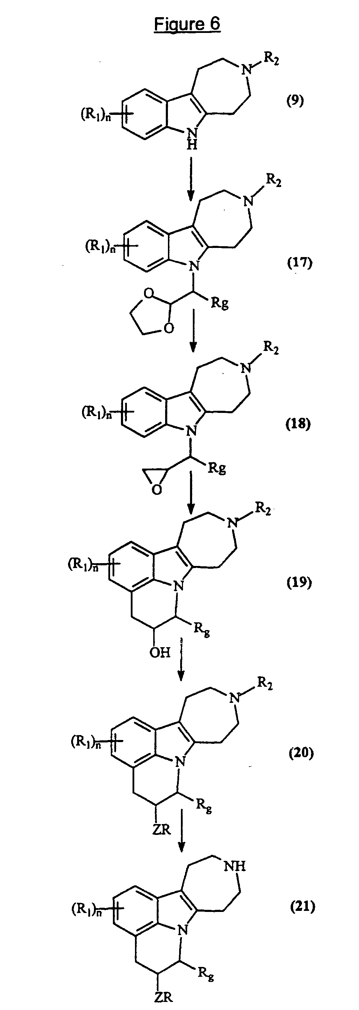

Azepinoindoles 9 can also be used to make compounds of formula I as shown in Figure 6. Alkylation of 9 with 2-bromomethyl-1,3-dioxolane, or a derivative thereof, gives compounds 17. Acid-catalyzed removal of the acetal group leads to an aldehyde that can be reacted with trimethylsulfoxonium iodide and sodium hydride to give epoxides 18. Use of a Lewis acid, such as boron trifluoride diethyl etherate, would lead to alcohols 19. These alcohols could be treated as above to give azepinoindoles 20 and 21.

In cases where compounds are sufficiently basic or acidic to form stable nontoxic acid or base salts, administration of the compounds as salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including hydrochloride, sulfate, nitrate, bicarbonate, and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

Compounds of the present invention can conveniently be administered in a pharmaceutical composition containing the compound in combination with a suitable excipient. Such pharmaceutical compositions can be prepared by methods and contain excipients which are well known in the art. A generally recognized compendium of such methods and ingredients is Remington's Pharmaceutical Sciences by E.W. Martin (Mark Publ. Co., 15th Ed., 1975). The compounds and compositions of the present invention can be administered parenterally (for example, by intravenous, intraperitoneal or intramuscular injection), topically, orally, or rectally.

For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The compounds or compositions can also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

Pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases; the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions can be prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers. Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Useful dosages of the compounds of formula I can be determined by comparing their *in vitro* activity, and *in vivo* activity in animal models. Methods for the extrapolation of effective dosages in mice, and other animals, to humans are known to the art; for example, see U.S. Pat. No. 4,938,949.

The compound is conveniently administered in unit dosage form; for example, containing about 0.05 mg to about 500 mg, conveniently about 0.1 mg to about 250 mg, most conveniently, about 1 mg to about 150 mg of active ingredient per unit dosage form. The desired dose may conveniently be presented in a single dose or as divided doses administered at appropriate intervals, for example, as two, three, four or more sub-doses per day. The sub-dose itself may be further divided, e.g., into a number of discrete loosely spaced administrations.

The compositions can conveniently be administered orally, sublingually, transdermally, or parenterally at dose levels of about 0.01 to about 150 mg/kg, preferably about 0.1 to about 50 mg/kg, and more preferably about 0.1 to about 10 mg/kg of mammal body weight.

For parenteral administration the compounds are presented in aqueous solution in a concentration of from about 0.1 to about 10%, more preferably about 0.1 to about 7%. The solution may contain other ingredients, such as emulsifiers, antioxidants or buffers.

The exact regimen for administration of the compounds and compositions disclosed herein will necessarily be dependent upon the needs of the individual subject being treated, the type of treatment and, of course, the judgment of the attending practitioner.

The ability of a compound of the invention to act as a 5-HT receptor agonist or antagonist can also be determined using *in vitro* and *in vivo* assays that are known in the art. The invention provides compounds of formula I that act as either agonists or as antagonists of one or more 5-HT receptor subtypes. The compounds Exemplified herein are 5-HT ligands, which typically displace >50% of a radiolabeled test ligand from one or more 5-HT receptor subtype at a concentration of 1 M. The procedures used for testing such displacement are well known and would be readily available to one skilled in the art.

### DESCRIPTION OF PREFERRED EMBODIMENTS

### PREPARATION 1.

### Preparation of 1-benzyl 4-ethyl 5-oxo-1,4-azepanedicarboxylate

A dry 500 ml 3-neck flask was charged with benzyl 4-oxo-1-piperidinecarboxylate (35.08 g, 150 mmol). It was dissolved in 130 ml Et₂O and cooled to -45°C. Ethyldiazoacetate (20.5 ml, 195 mmol) and boron trifluoride ethyl ether (19.4 ml, 158 mmol) were added simultaneously by syringe pump over 45 minutes. The temperature was kept below -25°C. The reaction was stirred for 30 minutes longer, and then quenched with sat. NaHCO₃. The ice bath was removed. The reaction was diluted with EtOAc (250 ml) and H₂O (150 ml). The layers were separated, and the organic phase was dried over MgSO₄. It was concentrated under reduced pressure to an orange oil. The product was purified by flash chromatography (silica gel, 40% EtOAc/hexane), yielding product as pale yellow oil (42.1 g, 88%). ¹H NMR (CDCl₃) δ 7.39-7.31, 5.15-5.12, 4.42-4.17, 3.96-3.83, 3.75-3.70, 3.65, 3.54-3.37, 2.08-2.03, 1.29-1.24; IR (liq.) 1743, 1702, 1476, 1455, 1443, 1425, 1371, 1318, 1295, 1238, 1213, 1178, 1098, 1068, 1028 cm ⁻¹.

### Preparation of benzyl 4-oxo-1-azepanecarboxylate

A solution of potassium hydroxide (24.6 g, 375 mmol) in H₂O (400 ml) was added to a solution of 1-benzyl 4-ethyl 5-oxo-1,4-azepanedicarboxylate (40.0 g, 125 mmol) in ethanol (400 ml). The resulting mixture was heated at reflux for 2.5 hours. Reaction was then cooled to rt., the ethanol was removed under reduced pressure, and was diluted with 200 ml brine and 300 ml ethyl acetate. The layers were separated, and the aqueous phase was extracted with ethyl acetate (2x 100 ml). The combined organic layers were washed with brine, dried over MgSO₄, and concentrated to an orange oil *in vacuo*. The product was isolated by flash chromatography (silica gel, 40%EtOAc/hexane) yielding a clear, colorless oil (22.6 g, 73%). ¹H NMR (CDCl₃) δ 7.31-7.30, 5.12, 3.65-3.63, 2.68-2.60, 1.81-1.78; IR (liq.) 1698, 1475, 1454, 1442, 1423, 1331, 1320, 1295, 1270, 1241, 1191, 1165, 1091, 900, 699 cm⁻¹.

### EXAMPLE 1 Preparation of 5,6,9,10,11,12-hexahydro-4H,8H-azepino[4',5':4,5]pyrrolo[3,2,1-ij]quinoline hydrochloride

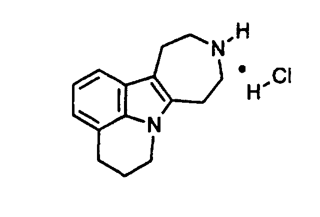

### Step 1. Preparation of 3,4-dihydro-1(2H)-quinolinylamine hydrochloride.

A neat reaction between 1,2,3,4-tetrahydroquinoline (3.71g, 27.9 mmol) and isoamylnitrite (8.72 g, 74.4 mmol) was allowed to stir for 1 hour. The residual isoamylnitrite was removed under reduced pressure and the N-nitroso intermediate was taken up in tetrahydrofuran (20 ml). This solution was added dropwise to a refluxing solution of lithium aluminum hydride in tetrahydrofuran (55 ml, 1M, 55.0 mmol). One hour after the addition was complete, the reaction was cooled to 0 °C and quenched. The reaction was filtered, concentrated under reduced pressure, and extracted into ether. The ethereal solution was washed with water, brine and dried over anhydrous potassium carbonate. The resulting brown oil (4.11 g) was trapped as the hydrochloride salt and recrystallized from methanol\ethyl acetate\hexanes to provide the title compound (mp 186-189 °C). ¹H NMR (CDCl₃) δ 10.55, 7.31, 7.10, 6.98, 6.87, 3.59, 2.74, 2.05;
IR (drift) 3053, 2956, 2940, 2868, 2841, 2835, 2724, 2667, 1603, 1586, 1580, 1567, 1545, 1499, 747 cm⁻¹.

### Step 2. Preparation of benzyl 5,6,8,9,11,12-hexahydro-4H,10H azepino[4',5':4,5]pyrrolo[3,2,1-ij]quinoline-10-carboxylate.

A solution of 3,4-dihydro-1(2H)-quinolinylamine (2.97 g, 20.1 mmol), benzyl 4-oxo-1-azepanecarboxylate (4.96 g, 20.1 mmol), and glacial acetic acid (0.2 ml) in ethanol (200 ml) was allowed to reflux for 2.5 hours. The reaction was then cooled and evaporated *in vacuo*. The hydrazone product was purified by flash chromatography (90g SiO₂, 1% MeOH/CH₂Cl₂) providing benzyl 4-[3,4-dihydro-1(2H)-quinolinylimino]-1-azepanecarboxylate (7.55 g) as an oil. To a solution of benzyl 4-[3,4-dihydro-1(2H)-quinolinylimino]-1-azepanecarboxylate (6.79g, 17.99 mmol) in ethanol (200 ml) was added trifluoroacetic acid (6.22 g, 53.96 mmol). The reaction was heated and allowed to stir at reflux for 2.5 hours, at which time it was cooled to rt., evaporated, and extracted into dichloromethane. This extract was washed with water, brine, dried with anhydrous sodium sulfate, and evaporated under reduced pressure. Crystallization from ethyl acetate/hexanes provided 2.93 g of the title compound (mp 131-133 °C). ¹H NMR (CDCl₃) δ 7.38, 7.29, 7.00, 6.86, 5.19, 3.98, 3.78, 2.97, 2.24;
IR (drift) 2947, 1699, 1473, 1420, 1358, 1260, 1250, 1235, 1216, 1101, 997, 764; 757, 746, 703 cm⁻¹.

### Step 3. Preparation of 5,6,9,10,11,12-hexahydro-4H,8H-azepino[4',5':4,5]pyaolo[3,2,1-ij]quinoline hydrochloride.

A mixture of benzyl 5,6,8,9,11,12-hexahydro-4H,10H-azepino[4',5':4,5]pyrrolo[3,2,1-ij]quinoline-10-carboxylate (2.42 g, 6.71 mmol) and 10 % Pd/C (0.15 g) in ethanol (110 ml) was hydrogenated under 40 psi for 1.5 hours. The mixture was filtered through celite, rinsed with methanol, dichloromethane and evaporated. Methanolic hydrochloric acid is added and evaporated. The resulting product is recrystallized from methanol/ethyl acetate to give 1.40 g (80%) of the title compound (mp 261-263 °C). ¹H NMR (CD₃OD) δ 7.25, 6.95, 6.84, 4.08, 3.48, 3.32, 3.28, 3.20, 2.95, 2.22; IR (drift) 2939, 2894, 2880, 2863, 2832, 2806, 2759, 2743, 2687, 2669, 2645, 2445, 1332, 1253, 743 cm⁻¹.

### EXAMPLE 2 Preparation of 2-methyl-5,6,9,10,11,12-hexahydro-4H,8H-azepino[4',5':4,5]pyrrolo[3,2,1-ij]quinoline hydrochloride

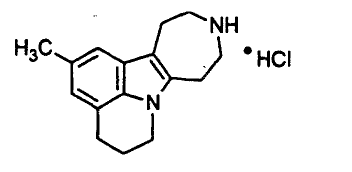

Following the general procedure of Example 1, making non-critical variations but starting with 6-methyl-1,2,3,4-tetrahydroquinoline, the title compound was obtained (mp 268-270 °C). ¹H NMR (DMSO-*d*_{*6*}) δ 9.6, 6.99, 6.61, 3.98, 3.30, 3.16, 3.03, 2.81, 2.32, 2.08; IR (drift) 2965, 2949, 2934, 2902, 2880, 2850, 2823, 2791, 2769, 2695, 2659, 2438,1458,1251, 839 cm⁻¹.

### EXAMPLE 3 Preparation of 1-methoxy-5,6,9,10,11,12-hexahydro-4H,8H-azepino[4',5':4,5]pyrrolo[3,2,1-ij]quinoline hydrochloride

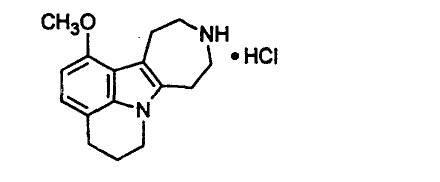

Following the general procedure of Example 1, making non-critical variations but starting with 7-methoxy-1,2,3,4-tetrahydroquinoline, the title compound was obtained (mp 277-279 °C). ¹H NMR (DMSO-*d*_{*6*}) δ 9.27, 6.65, 6.32, 3.96, 3.77, 3.29, 3.15, 2.78, 2.05; IR (drift) 2968, 2953, 2935, 2889, 2832, 2803, 2780, 2765, 2744, 2713, 1594, 1509, 1247, 1152, 781 cm⁻¹.

### EXAMPLE 4 Preparation of 2-fluoro-5,6,9,10,11,12-hexahydro-4H,8H-azepino[4',5':4,5]pyrrolo[3,2,1-ij]quinoline hydrochloride

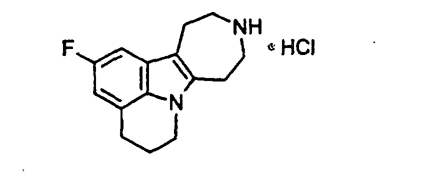

Following the general procedure of Example 1, making non-critical variations but starting with 6-fluoro-1,2,3,4-tetrabydroquinoline, the title compound was obtained (mp 273-276 °C). ¹H NMR (CD₃OD) δ 6.94, 6.64, 4.07, 3.50, 3.44, 3.32, 3.28, 3.15, 2.94, 2.22; IR (drift) 2957, 2926, 2894, 2843, 2814, 2799, 2742, 2708, 2665, 2561, 2545, 2440, 1496, 1419, 1129 cm⁻¹.

### EXAMPLE 5 Preparation of 6-methyl-5,6,9,10,11,12-hexahydro-4H,8H-azepino[4',5':4,5]pyrrolo[3,2,1-ij]quinoline hydrochloride

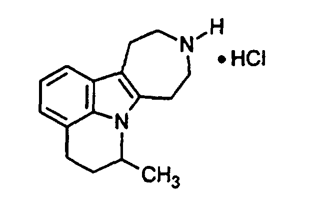

Following the general procedure of Example 1, making non-critical variations but starting with 2-methyl-1,2,3,4-tetrahydroquinoline, the title compound is obtained (mp 214-217 °C). ¹H NMR (CDCl₃) δ 9.61, 7.25, 6.93, 6.84, 4.62, 3.32, 3.11, 2.87, 2.12, 2.01, 1.20; IR (drift) 2968, 2933, 2893, 2814, 2742, 2711, 2669, 2560, 2437, 1463, 1415, 1377, 1335, 1324, 742 cm⁻¹.

### EXAMPLE 6 Preparation of 5-methyl-5,6,9,10,11,12-hexahydro-4H,8H-azepino[4',5':4,5]pyrrolo[3,2,1-ij]quinoline hydrochloride

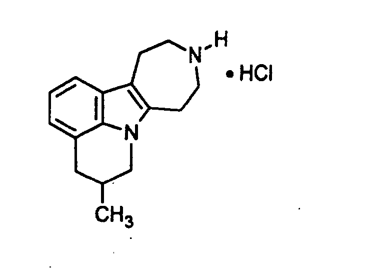

Following the general procedure of Example 1, making non-critical variations but starting with 3-methyl-1,2,3,4-tetrahydroquinoline, the title compound was obtained (mp 273-274 °C). ¹H NMR (CDCl₃) δ 10.10, 7.27, 7.02, 6.88, 4.07, 3.51, 3.37, 3.31, 3.00, 2.65, 2.37, 1,20; IR (drift) 2956, 2923, 2899, 2867, 2831, 2800, 2766, 2686, 2670, 2562, 2448, 1454, 1428, 1257, 740 cm⁻¹.

### EXAMPLE 7 Preparation of 4-methyl-5,6,9,10,11,12-hexahydro-4H,8H-azepino[4',5':4,5]pyrrolo[3,2,1-ij]quinoline hydrochloride

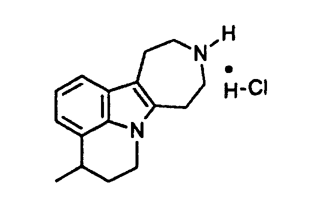

Following the general procedure of Example 1, making non-critical variations but starting with 4-methyl-1,2,3,4-tetrahydroquinoline, the title compound was obtained (mp 260-263 °C). ¹H NMR (DMSO-*d*_{*6*}) δ 9.79, 7.22, 6.92, 6.85, 4.10, 4.00, 3.28, 3.20, 3.08, 2.15, 1.80, 1.30; IR (drift) 2959, 2939, 2891, 2873, 2860, 2811, 2800, 2739, 2709, 2662, 2647, 2553, 2542, 2437, 734 cm⁻¹.

### EXAMPLE 8 Preparation of (+)-6-methyl-5,6,9,10,11,12-hexahydro-4H,8H-azepino[4',5':4,5]pyrrolo[3,2,1-ij]quinoline hydrochloride

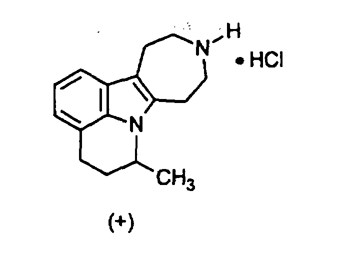

Following the general procedure of Example 5 making non-critical variations, preparative chiral HPLC was performed after step two on an EM ST 140R closed loop recycling prep HPLC system (EM Separations Technology). The column used was a 5x50 cm Chiralpak AD column at 30 °C. The mobile phase was 5% isopropanol/95% heptane at a flow rate of 75 ml/min. Peak collection was monitored by UV detection at 285 nm. Following step three, the title compound was obtained (mp 196-199 °C).

### EXAMPLE 9 Preparation of (-)-6-methyl-5,6,9,10,11,12-hexahydro-4H,8H-azepino[4',5':4,5]pyrrolo[3,2,1-ij]quinoline hydrochloride

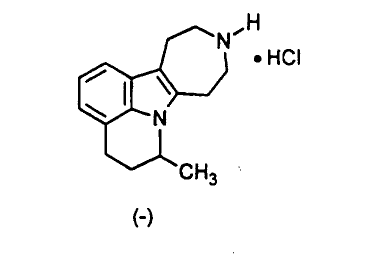

Following the general procedure of Example 5 making non-critical variations, preparative chiral HPLC was performed after step two on an EM ST 140R closed loop recycling prep HPLC system (EM Separations Technology). The column used was a 5x50 cm Chiralpak AD column at 30 °C. The mobile phase was 5% isopropanol/95% heptane at a flow rate of 75 ml/min. Peak collection was monitored by UV detection at 285 nm. Following step three, the title compound was obtained (mp 196-199 °C); [α]²⁵_{D} = -27 (*c* 0.88, DMSO).

### EXAMPLE 10 Preparation of (+)-5-methyl-5,6,9,10,11,12-hexahydro-4H,8H-azepino[4',5':4,5]pyrrolo[3,2,1-ij]quinoline hydrochloride

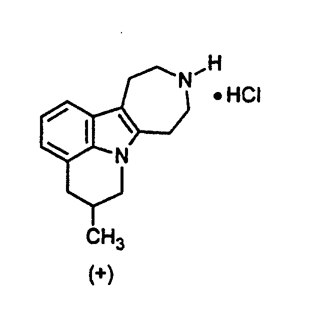

Following the general procedure of Example 6 making non-critical variations, preparative chiral HPLC was performed after step two on an EM ST 140R closed loop recycling prep HPLC system (EM Separations Technology). The column used was a 5x50 cm Chiralpak AD column at 30 °C. The mobile phase was 5% isopropanol/95% heptane at a flow rate of 75 ml/min. Peak collection was monitored by UV detection at 285 nm. Following step three, the title compound was obtained (mp 259-262 °C); [α]²⁵_{D} = + 20 (*c* 0.28, DMSO).

### EXAMPLE 11 Preparation of (-)-5-methyl-5,6,9,10,11,12-hexahydro-4H,8H-azepino[4',5':4,5]pyrrolo[3,2,1-ij]quinoline hydrochloride

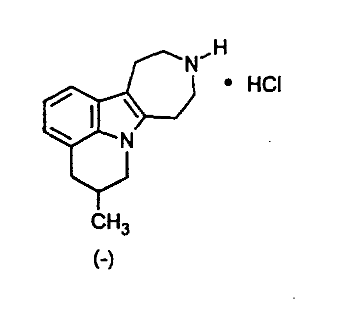

Following the general procedure of Example 6 making non-critical variations, preparative chiral HPLC was performed after step two on an EM ST 140R closed loop recycling prep HPLC system (EM Separations Technology). The column used was a 5x50 cm Chiralpak AD column at 30 °C. The mobile phase is 5% isopropanol/95% heptane at a flow rate of 75 ml/min. Peak collection was monitored by UV detection at 285 nm. Following step three, the title compound was obtained (mp 259-262 °C); [α]²⁵_{D} = -21° (*c* 0.43, DMSO).

### EXAMPLE 12 Preparation of (+)-4-methyl-5,6,9,10,11,12-hexahydro-4H,8H-azepino[4',5':4,5]pyrrolo[3,2,1-ij]quinoline hydrochloride

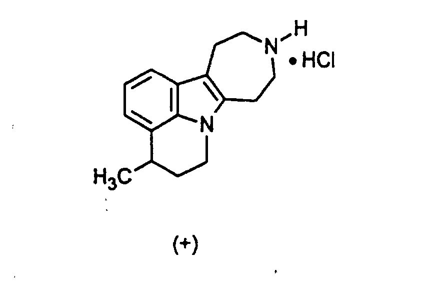

Following the general procedure of Example 7 making non-critical variations, preparative chiral HPLC was performed after step two on an EM ST 140R closed loop recycling prep HPLC system (EM Separations Technology). The column used was a 5x50 cm Chiralpak AD column at 30 °C. The mobile phase was 5% isopropanol/95% heptane at a flow rate of 75 ml/min. Peak collection was monitored by UV detection at 285 nm. Following step three, the title compound was obtained (mp 261-263 °C); [α]²⁵_{D} = +39 (*c* 0.41, chloroform).

### EXAMPLE 13 Preparation of (-)-4-methyl-5,6,9,10,11,12-hexahydro-4H,8H-azepino[4',5':4,5]pyrrolo[3,2,1-ij]quinoline hydrochloride

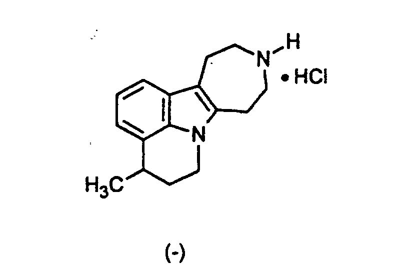

Following the general procedure of Example 7 making non-critical variations, preparative chiral HPLC was performed after step two on an EM ST 140R closed loop recycling prep HPLC system (EM Separations Technology). The column used was a 5x50 cm Chiralpak AD column at 30 °C. The mobile phase was 5% isopropanol/95% heptane at a flow rate of 75 ml/min. Peak collection was monitored by UV detection at 285 nm. Following step three, the title compound is obtained (mp 261-263 °C); [α]²⁵_{D} = -39 (c 0.51, chloroform).

### EXAMPLE 14 Preparation of 4,5,8,9,10,11-hexahydro-7H-azepino[4,5-b]pyrrolo[3,2,1-hi]indole hydrochloride

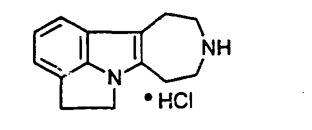

Following the general procedure outlined in EXAMPLE 1, starting with indoline, and utilizing 1-benzoylhexahydoazepine as the ketone in step 4, 5,7,8,10,11-hexahydro-9H-azepino[4,5-b]pyrrolo[3,2,1-hi]indol-9-yl(phenyl)methanone was obtained (mp 130-133 °C). ¹H NMR (CDCl₃) δ 7.42, 7.08, 6.94, 6.85, 4.28, 4.00, 3.76, 3.63, 3.15, 2.88; MS (ESI+) for C₂₁H₂₀N₂O H *m*/*z* 240.1 (M+H)⁺.

Step 4 A mixture of 4,5,7,8,10,11-hexahydro-9H-azepino[4,5-b]pyrrolo[3,2,1-hi]indol-9-yl(phenyl)methanone (1.0 g, 3.2 mmol) and potassium hydroxide (0.89 g, 15.8 mmol) in ethylene glycol (10 ml) was heated under N₂ at 170 °C for 3 h. The reaction was cooled to rt, poured into water (50 ml) and extracted with methylene chloride (4 x 50 ml). The combine organics were washed with brine, dried over anhydrous potassium carbonate, and concentrated *in vacuo*. The residue was trapped as its HCl salt by treatment of a solution of the product in methanol with ethereal HCl. The resulting precipitate is recrystallized from methanol and ethyl acetate to give the desired product (0.4 g) (mp 249-250 °C). ¹H NMR (CDCl₃) δ 7.16, 6.93, 6.82, 4.38, 3.74, 3.08, 2.94; IR (drift) 2925,2916,2885, 2847, 2805, 1510, 1462, 1409, 1350, 1336, 1306, 1279, 767, 758, 746 cm⁻¹.

### EXAMPLE 15 Preparation of 2-fluoro-4,5,8,9,10,11-hexahydro-7H-azepino[4,5-b]pyrrolo[3,2,1-hi]indole hydrochloride

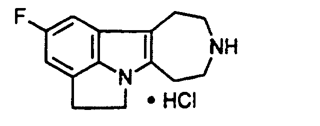

Following the general procedure outlined in EXAMPLE 14, and making non-critical variations but starting with 5-fluoro indoline, the title compound was obtained (mp 250-252 °C). ¹H NMR (CD₃OD) δ 6.68, 6.43, 4.15, 3.47, 2.88, 2.76; IR (drift) 2930, 2911, 1661, 1507, 1412, 1354, 1261, 1171, 1112, 938, 857, 848, 839, 708, 688 cm⁻¹.

### EXAMPLE 16 Preparation of 2-methoxy-4,5,8,9,10,11-hexahydro-7H-azepino[4,5-b]pyrrolo[3,2,1-hi]indole hydrochloride

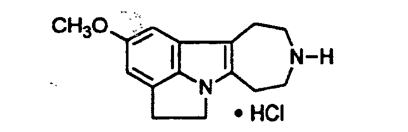

Following the general procedure outlined in EXAMPLE 14, and making non-critical variations but starting with 5-methoxy indoline, the title compound was obtained (mp 269-271 °C). ¹H NMR (CD₃OD) δ 6.70, 6.57, 4.44, 3.80, 3.72, 3.45, 3.23, 3.16; IR (drift) 2974, 2948, 2908, 2891, 2841, 2817, 2805, 2769, 2718, 1509, 1421, 1255, 1244, 1233, 1142 cm ⁻¹.

### EXAMPLE 17 Preparation of 5-methyl-4,5,8,9,10,11-hexahydio-7H-azepino[4,5-b]pyrrolo[3,2,1-hi]indole hydrochloride

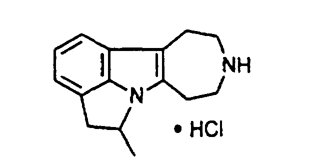

Following the general procedure outlined in EXAMPLE 1, and making non-critical variations but starting with 2-methyl indoline, the title compound was obtained (mp 243-246 °C). ¹H NMR (CDCl₃) δ 7.13, 6.91, 7.80, 4.99-4.87, 3.93, 3.51-3.23, 3.17, 1.47; IR (drift) 2973, 2960, 2941, 2927, 2901, 2883, 2848, 2826, 2794, 2737, 2650, 2549, 2438, 1292, 751 cm⁻¹.

### EXAMPLE 18 Preparation of 4,5-dimethyl-4,5,8,9,10,11-hexahydro-7H-azepino[4,5-b]pyrrolo[3,2,1-hi]indole hydrochloride

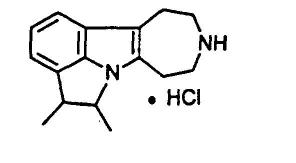

Following the general procedure outlined in EXAMPLE 1, and making non-critical variations but starting with 2, 3-dimethyl indole, the title compound was obtained (mp 195-197 °C). ¹H NMR (CD₃OD) δ 7.16, 6.92, 6.81, 4.40, 3.64, 3.53-3.35, 3.16, 1.54, 1.43; IR (drift) 2960, 2938, 2925, 2866, 2847, 2821, 2727, 2657, 2635, 2533, 2427, 1465, 1372, 1287, 746 cm⁻¹.

### EXAMPLE 19 Preparation of 2,3,4,5,8b,9,10,11,12,12a-decahydro-1H-azepino[4',5':4,5]pyrrolo[3,2,1-jk]carbazole hydrochloride

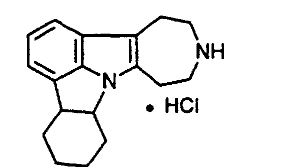

Following the general procedure outlined in EXAMPLE 1, and making non-critical variations but starting with 1,2,3,4 tetramethyl carbazole, the title compound was obtained (mp 210-212 °C). ¹H NMR (CD₃OD) δ 7.16, 6.94, 6.82, 4.75, 4.14, 3.42, 3.25, 3.16, 2.12-1.96, 1.54, 1.41, 1.26, 1.08; IR (drift) 3046, 2936, 2929, 2846, 2810, 2744, 2633, 2541, 2519, 2429, 1460, 1328, 1290, 754, 744 cm ⁻¹;

### EXAMPLE 20 Preparation of 6-methyl-1,2,8,9,10,11-hexahydro-7H-azepino[4,5-b][1,4]oxazino[2,3,4-hi]indole hydrochloride

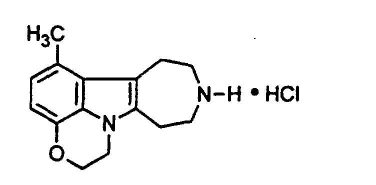

### Step 5 Preparation of 6-methyl-2H-1,4-benzaxazin-3(4H)-one

To a solution of 4-methyl-2-nitrophenol (9.95 g, 65.0 mmol) in acetone (170 ml) was added, in one portion, potassium carbonate (10.8 g, 78.0 mmol) and ethyl bromoacetate (7.9 ml, 71.5 mmol). The resulting mixture was heated at reflux for 3.5 hours. The acetone was removed *in vacuo*, and the resulting material was partitioned between ethyl acetate and water. The layers were separated, and the aqueous phase was extracted twice with ethyl acetate. The ethyl acetate layer was washed with saturated sodium bicarbonate, brine, dried over magnesium sulfate, filtered, and evaporated to afford an orange oil. The intermediate nitro compound was taken up in ethanol (150 ml) and hydrogenated with Pd/C (0.33 g) at 51 psi for 90 minutes. The mixture was filtered through celite, and the filtrate evaporated. The product was recrystallized from methanol to yield 7.29 g of the title compound as an off-white solid (mp 204-205 °C). ¹H NMR (DMSO-*d*_{*6*}) δ 6.81-6.78, 6.70-6.67, 4.48, 2.18; MS [MH⁻] 162.1.

### Step 6. Preparation of 6-methyl-3,4-dihydro-2H-1,4-benzoxazine

To a solution of 6-methyl-2H-1,4-benzoxazin-3(4H)-one (7.02 g, 43.0 mmol) in tetrahydrofuran (130 ml) was added borane dimethylsulfide complex (43 ml, 1 0M). This solution was stirred at rt for 2.5 hours, then quenched with 1 M hydrochloric acid. The solvent was evaporated, and the residue was partitioned between saturated sodium bicarbonate and dichloromethane. The layers were separated, and the aqueous phase was extracted twice with dichloromethane. The dichloromethane layer was washed with brine, dried over magnesium sulfate, and evaporated to yield 5.1 g of the title compound as a pale yellow oil. ¹H NMR (CDCl₃) δ 6.70-6.68, 6.49-6.46, 6.42, 4.24-4.21, 3.41-3.38, 2.22; MS [MH⁺] 150.2.

Following the remainder of the general procedure of Example 1 (steps 1-3) and making non-critical variations but starting with 6-methyl-3,4-dihydro-2H-1,4-benzoxazine, the title compound was obtained (mp 271-273 °C). ¹H NMR (CD₃OD) δ 6.59, 6.40, 4.44, 4.17, 3.52, 3.49, 3.27, 3.24, 2.57; IR (drift) 2979, 2957, 2934, 2872, 2855, 2828, 2792, 2760, 2746, 2683, 2652, 1511, 1265, 1243, 1223 cm ⁻¹.

### EXAMPLE 21 Preparation of 1,2,8,9,10,11-hexahydro-7H-azepino[4,5-b][1,4]oxazino[2,3,4-hi]indole hydrochloride

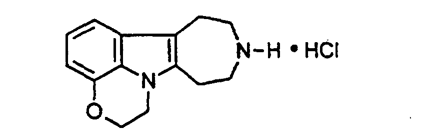

Following the general procedure of Example 20 but starting with 2 H-1,4-Benzoxazine-3(4 H)-one, utilizing 1-benzoylhexahydoazepine as the ketone in the hydrazone formation of step two, and thus the deprotection sequence described in step 4 of example 14, the title compound was obtained (mp 217-219 °C). ¹H NMR (DMSO-*d*₆) δ 9.6, 7.01,6.85, 6.50, 4.46, 4.18, 3.28-3.08, 2.50; IR (drift) 2949, 2925, 2878, 2843, 2812, 2758, 2689, 2673, 1499, 1328, 1247, 1036, 872, 771, 729 cm ⁻¹.

### EXAMPLE 22 Preparation of 6-chloro-1,2,8,9,10,11-hexahydro-7H-azepino[4,5-b][1,4]oxazino[2,3,4-hi]indole hydrochloride

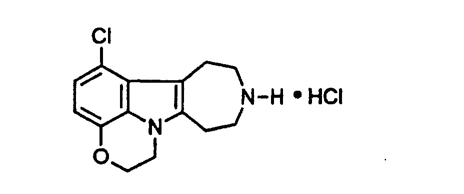

Following the general procedure of Example 20 and making non-critical variations but starting with 2-amino-4-chlorophenol, the title compound was obtained (mp decompose >275°C). ¹H NMR (CD₃OD) δ 6.84, 6.50, 4.49-4.48, 4.22-4.20, 3.65-3.63, 3.53-3.48, 3.30-3.27; IR (free amine) (drift) 2932, 2897, 2882, 2823,1495, 1466, 1379, 1355, 1323, 1277, 1269, 1236, 1217, 1199, 1014 cm⁻¹.

### EXAMPLE 23 Preparation of 5-fluoro-1,2,8,9,10,11-hexahydro-7H-azepino[4,5-b][1,4]oxazino[2,3,4-hi]indole hydrochloride

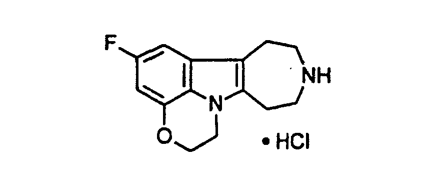

Following the general procedure of Example 20 and making non-critical variations but starting with 5-fluoro-2-nitrophenol, the title compound was obtained (mp decompose >250°C). ¹H NMR (CD₃OD) δ 6.76-6.72, 6.38-6.33, 4.52-4.49, 4.20-4.17, 3.51-3.43, 3.28-3.25, 3.18-3.14; IR (drift) 2950, 2855, 2805, 2767, 2757, 2710, 2675, 2651, 2565, 2449, 1645, 1591, 1500, 1333, 1109 cm ⁻¹.

### EXAMPLE 24 Preparation of 5-methyl-1,2,8,9,10,11-hexahydro-7H-azepino[4,5-b][1,4]oxazino[2,3,4-hi]indole hydrochloride

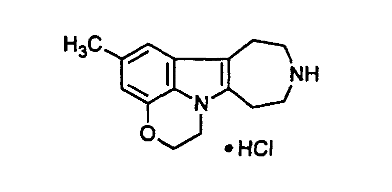

Following the general procedure of Example 20 but starting from 5-methyl-2-nitrophenol and using glacial acetic acid for the cyclization in step 3, the title compound was obtained (mp 274-275 °C). ¹H NMR (CD₃OD) δ 6.81, 6.37, 4.44, 4.12, 3.48-3.41, 3.22, 3.15, 2.36; IR (drift) 2983, 2965, 2940, 2880, 2856, 2822,2809, 2758, 2731, 2667, 1590, 1503, 1331, 1031, 847 cm⁻¹.

### EXAMPLE 25 Preparation of 6-fluoro-1,2,8,9,10,11-hexahydro-7H-azepino[4,5-b][1,4]oxazino[2,3,4-hi]indole hydrochloride

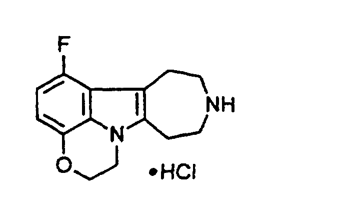

Following the general procedure of Example 20 but starting from 4-fluoro-2-nitrophenol and using *p*-toluene sulfonic acid for the cyclization in step 3, the title compound was obtained (mp 257-259°C). ¹H NMR (CD₃OD) δ 6.55-6.49, 6.43-6.40, 4.44, 4.17, 3.49-3.44, 3.38-3.33, 3.26-3.23; IR (drift) 2949, 2876, 2845, 2815, 2768, 2685, 2676, 2626, 1511, 1362, 1277, 1224, 1023, 881, 791 cm ⁻¹.

### EXAMPLE 26 Preparation of 2-methyl-1,2,8,9,10,11-hexahydro-7H-azepino[4,5-b][1,4]oxazino[2,3,4-hi]indole hydrochloride

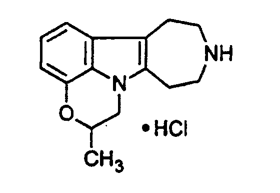

Following the general procedure of Example 20 and making non-critical variations but starting with 2-nitrophenol and ethyl-2-bromopropionate, the title compound was obtained (mp 255-257 °C). ¹H NMR (CD₃OD) δ 7.01, 6.88, 6.54, 4.40-4.35, 4.30, 3.68-3.63, 3.49-3.42, 3.25-3.17, 1.51; IR (drift) 2967, 2931, 2809, 2790, 2736, 2711, 2655, 2647, 2557, 2439, 1502, 1377, 1322, 1243, 794 cm ⁻¹.

### EXAMPLE 27 Preparation of 1,2,8,9,10,11-hexahydro-7H-azepino [4,5-b][1,4]thiazino[2,3,4-hi]indole hydrochloride

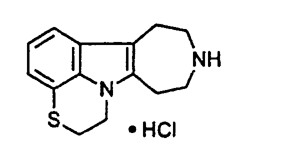

Following the general procedure of Example 21 but starting from 2H-1,4-benzothiazin-3(4H)-one and using 10% sulfuric acid for the cyclization in step 3, the title compound was obtained (mp 263 °C). ¹H NMR (CD₃OD) δ 7.24, 6.94, 6.87, 3.36-3.24, 3.12-3.10; IR (drift) 2937, 2929, 2862, 2814, 2718, 2663, 2626, 2594, 2544, 2428, 1463, 1412, 1335, 782, 739 cm⁻¹.

### EXAMPLE 28 Preparation of 2,2-dimethyl-1,2,8,9,10,11-hexahydro-7H-azepino [4,5-b] [1,4]oxazino[2,3,4-hi]indole hydrochloride

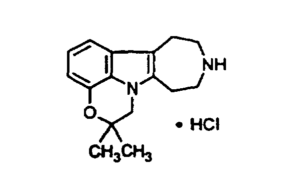

Following the general procedure of Example 20 but starting with 2-nitrophenol and ethyl-2-bromoisobutyrate, and using p-toluene sulfonic acid for the cyclization in step 3, the title compound was obtained (mp decompose >260°C). ¹H NMR (CD₃OD) δ 7.03, 6.92, 6.52, 3.96, 3.53-3.46, 3.26-3.20, 1.41; IR (drift) 2972, 2948, 2858, 2824, 2766, 2751, 1583, 1498, 1385, 1332, 1245, 1202 cm ⁻¹.

### EXAMPLE 29 Preparation of 4-fluoro-1,2,8,9,10,11-hexahydro-7H-azepino[4,5-b][1,4] oxazino[2,3,4-hi]indole maleic acid

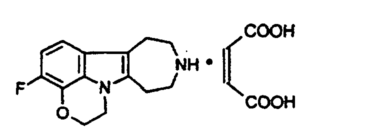

Following the general procedure of Example 20 but starting with 2-fluoro-6-nitrophenol, using p-toluene sulfonic acid for the cyclization in step 3, and preparing the Maleic acid salt, the title compound was obtained (mp 176-177°C ). ¹H NMR (CD₃OD) δ 6.95, 6.78, 6.24, 4.51, 4.19, 3.42-3.50, 3.23, 3.16; IR (drift) 3009, 2925, 2899, 2879, 2835, 2786, 2714, 1705, 1626, 1550, 1531, 1526, 1486, 1358 cm⁻¹.

### EXAMPLE 30 Preparation of 4-chloro-1,2,8,9,10,11-hexahydro-7H-azepino[4,5-b][1,4]oxazino[2,3,4-hi]indole maleic acid

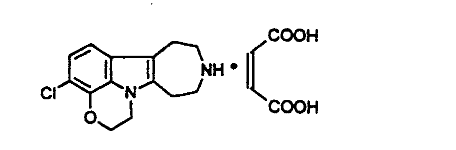

Following the general procedure of Example 20 but starting with 6-chloro-2-nitrophenol, using p-toluene sulfonic acid for the cyclization in step 3, and preparing the Maleic acid salt, the title compound was obtained (mp 183-184 °C ). ¹H NMR (CD₃OD) δ 6.99, 6.91, 6.24, 4.56, 4.20, 3.43-3.51, 3.23, 3.16; IR (drift) 1645, 1639, 1627, 1608, 1568, 1536, 1517, 1497, 1476, 1385, 1377, 1372, 1355, 1196 cm⁻¹.

### EXAMPLE 31 Preparation of 5-chloro-1,2,8,9,10,11-hexahydro-7H-azepino[4,5-b][1,4] oxazino[2,3,4-hi]indole maleic acid

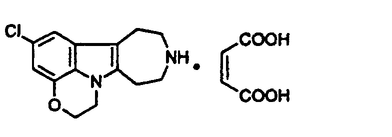

Following the general procedure of Example 20 but starting with 5-chloro-2-nitrophenol, using TFA in EtOH for the cyclization in step 3, and preparing the Maleic acid salt, the title compound was obtained (mp 177-179 °C ). ¹H NMR (CD₃OD) δ 7.05, 6.55, 6.24, 4.50, 4.18, 3.42-3.50, 3.23-3.25, 3.14-3.16; IR (drift) 2892, 2867, 2779, 2745, 1627, 1569, 1536, 1491, 1466, 1451, 1369, 1329, 1024 cm⁻¹.

### EXAMPLE 32 Preparation of 6-(trifluoromethyl)-1,2,8,9,10,11-hexahydro-7H-azepino[4,5-b][1,4]oxazino[2,3,4-hi]indole hydrochloride

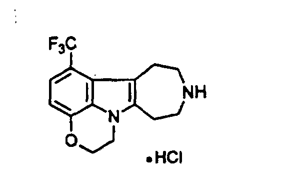

Following the general procedure of Example 20 but starting with 2-nitro-4-(trifluoro methyl)-phenol, using p-toluene sulfonic acid for the cyclization in step 3, the title compound was obtained (mp 248-250 °C ). ¹H NMR (CD₃OD) δ 7.30, 6.61, 4.55, 4.26, 3.46-3.53; IR (drift) 2815, 2797, 2741, 2670, 2560,1583, 1333, 1244, 1198, 1158, 1107, 1094, 1060, 1021 cm⁻¹.

### EXAMPLE 33 Preparation of 5,6-difluoro-1,2,8,9,10,11-hexahydro-7H-azepino[4,5-b][1,4]oxazino[2,3,4-hi]indole maleic acid

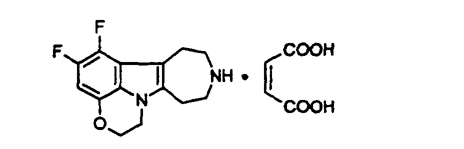

Following the general procedure of Example 20 but starting with 4,5-difluoro-2-nitrophenol, using 10% sulfuric acid in step 3, and preparing the Maleic acid salt, the title compound was obtained (mp 184-185 °C). ¹H NMR (CD₃OD) δ 6.45, 6.23, 4.46, 4.17, 3.45-3.50, 3.34-3.37, 3.220-3.24; IR (drift) 3070, 2879, 2780, 2728, 1601, 1575, 1518, 1482, 1457, 1373, 1353, 1171, 1042, 1000 cm⁻¹.

### EXAMPLE 34 Preparation of 5-chloro-6-fluoro-1,2,8,9,10,11 -hexahydro-7H-azepino[4,5-b][1,4]oxazino[2,3,4-hi]indole maleic acid

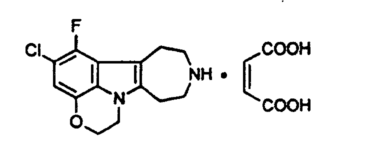

Following the general procedure of Example 20 but starting with 5-chloro-4-fluoro-2-nitrophenol, using 10% sulfuric acid in step 3, and preparing the Maleic acid salt, the title compound was obtained (mp 194-195 °C). ¹H NMR (CD₃OD) δ 6.52, 6.24, 4.46, 4.18, 3.51-3.45, 3.29-3.37, 3.21-3.25; IR (drift) 1615, 1583, 1552, 1501, 1480, 1456, 1368, 1355, 1280, 1222, 1169 cm⁻¹.

### EXAMPLE 35 Preparation of 5-fluoro-6-chloro-1,2,8,9,10,11-hexahydro-7H-azepino[4,5-b][1,4]oxazino[2,3,4-hi]indole maleic acid

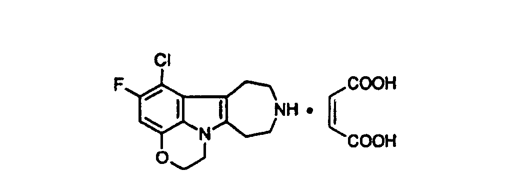

Following the general procedure of Example 20 but starting with 4-chloro-5-fluoro-2-nitrophenol, using 10% sulfuric acid in step 3, and preparing the Maleic acid salt, the title compound was obtained (mp 173-175 °C). ¹H NMR (CD₃OD) δ 6.50, 3.25, 4.50, 4.19, 3.56-3.59, 3.44-3.50, 3.22-3.25; IR (drift) 3062, 3028, 2971, 2897, 1626, 1608, 1585, 1498, 1463, 1372, 1365, 1352, 1153, 1036 cm⁻ ¹.

### EXAMPLE 36 Preparation of 5,6-dichloro-1,2,8,9,10,11-hexahydro-7H-azepino[4,5-b][1,4]oxazino[2,3,4-hi]indole maleic acid

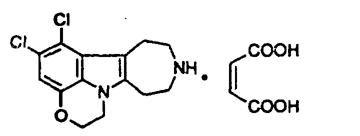

Following the general procedure of Example 20 but starting with 4,5-dichloro-2-nitrophenol, using 10% sulfuric acid in step 3, and preparing the Maleic acid salt, the title compound was obtained (mp 180-181 °C). ¹H NMR (CD₃OD) δ 6.68 (s, 1 H), 6.23 (s, 2 H), 4.49 (t, *J =* 4.4 Hz, 2 H), 4.20 (t, *J =* 4.8 Hz, 2 H), 3.59-3.62 (t, *J*= 2 Hz, H), 3.45-3.51 (m, 4 H), 3.22-3.26 (m, 2 H); IR (drift) 2887, 2840, 1625, 1608, 1578, 1551, 1486, 1458, 1372, 1351, 1273, 1023 cm⁻¹.

### EXAMPLE 37 Preparation of 4,6-dichloro-1,2,8,9,10,11-hexahydro-7H-azepino[4,5-b][1,4]oxazino[2,3,4-hi]indole hydrochloride

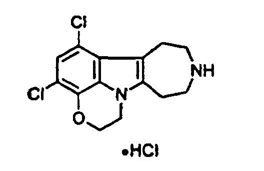

Following the general procedure of Example 20 but starting with 3,5-dichloro-2-nitrophenol, and using 10% sulfuric acid in step 3, the title compound was obtained (decompose >255°C). ¹H NMR (CD₃OD) δ 6.89, 4.55, 4.21, 3.56-3.59, 3.44-3.51, 3.23-3.27; IR (drift) 2955, 2895, 2743, 2652, 2635, 2556, 2428, 1494, 1468, 1420, 1345, 1294, 1229 cm⁻¹.

### EXAMPLE 38 Preparation of 1-chloro-2-fluoro-4,5,8,9,10,11-hexahydro-7H-azepino[4,5-b]pyrrolo[3,2,1-hi]indole maleic acid

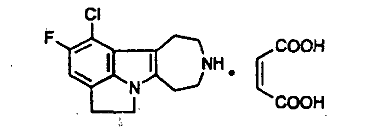

Following the general procedure outlined in EXAMPLE 14, and making non-critical variations but starting with 6-chloro-5-fluoro indoline, the title compound was obtained (amorphous solid). ¹H NMR (CD₃OD) 6.70, 6.23, 4.52, 3.69, 3.34-3.22, 3.08; IR (drift) 2420 (b), 1628, 1561, 1506 (s), 1449, 1421, 1378 (s), 1350, 1337, 1323, 1300, 1291, 1278, 1254, 1147 cm⁻¹; MS (ESI+) for C₁₄H₁₄ClFN₂ *m*/*z* 265.2 (M+H)⁺.

### EXAMPLE 39 Preparation of 2-chloro-4,5,8,9,10,11-hexahydro-7H-azepino[4,5-b]pyrrolo[3,2,1-hi]indole maleic acid

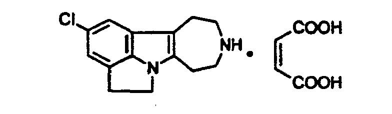

Following the general procedure outlined in EXAMPLE 14, and making non-critical variations but starting with 5-chloro-indoline, the title compound was obtained (amorphous solid). ¹H NMR (CD₃OD) 7.16, 6.91, 6.81, 4.45, 3.74, 3.40, 3.24, 3.15;.MS (ESI+) for C₁₄H₁₅ClN₂ *m*/*z* 247.2 (M+H)⁺.

### EXAMPLE 40 Preparation of 1-chloro-4,5,8,9,10,11-hexahydro-7H-azepino[4,5-b]pyrrolo[3,2,1-hi]indole maleic acid

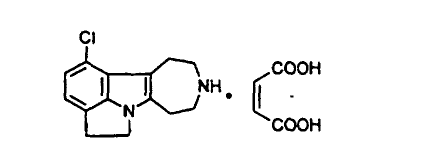

Following the general procedure outlined in EXAMPLE 14, and making non-critical variations but starting with 6-chloro-indoline, the title compound was obtained (amorphous solid). ¹H NMR (CD₃OD) 6.91, 6.81, 6.77, 4.90, 3.69, 3.34, 3.21;.MS (ESI+) for C₁₄H₁₅ClN₂ *m*/*z* 247.2 (M+H)⁺.

### EXAMPLE 41 Preparation of 5,6,9,10,11,12-hexahydro-4H,8H-azepino[4',5':4,5]pyrrolo[3,2,1-ij]quinolin-4-one oxalate.

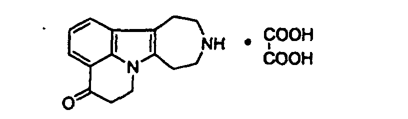

### Step 7. Preparation of ethyl 3-(3-benzoyl-2,3,4,5-tetrahydroazepino[4,5-b]indol-6(1H)-yl)propanoate.

A mixture of 3-benzoyl-1,2,3,4,5,6-hexahydroazepino[4,5-b]indole (5.00 g, 17.2 mmol), cesium carbonate (5.61 g, 17.2 mmol), and ethyl acrylate (1.90 mL, 17.5 mmol) in acetonitrile (250 mL) was heated under N₂ at 50 °C for 5 h (reaction can also be run in DMF at room temperature). Cooled to room temperature, diluted with H₂O, and extracted with EtOAc. The combined organic extracts were washed with saturated aqueous NaCl, dried over Na₂SO₄, decanted, and concentrated under reduced pressure. The crude product was purified by silica gel chromatography with EtOAc/hexanes (2:1) to give 4.82 g (72%) of the title compound as a yellow oil. ¹H NMR (CDCl₃) δ 7.57- 7.38, 7.32, 7.23-7.07, 4.49-4.33, 4.20-4.02, 3.77-3.66, 3.28-3.13, 2.94, 2.72, 1.24; IR (liq.) 2981, 1731, 1631, 1467, 1445, 1422, 1381, 1368, 1349, 1319, 1295,1272, 1242, 1187, 742, 707 cm⁻¹; MS (ESI) 391.0 (M⁺+H).

### Step 8. Preparation of 10-benzoyl-5,6,9,10,11,12-hexahydro-4H,8H-azepino[4',5':4,5]pyrrolo[3,2,1-ij]quinolin-4- one.

A solution of potassium hydroxide (0.80 g, 14.3 mmol) in H₂O (20 mL) was added to a solution of ethyl 3-(3-benzoyl-2,3,4,5-tetrahydroazepino[4,5-b]indol-6(1H)-yl)propanoate (4.25 g, 10.9 mmol) in THF (30 mL). The reaction mixture was heated at 60 °C for 1 h, then cooled to room temperature, acidified with 10% aqueous HCl, and extracted with EtOAc. The combined organic extracts were washed with saturated aqueous NaCl, dried over Na₂SO₄, decanted, and concentrated under reduced pressure to give 3.73 g of crude acid. The crude acid (2.00 g, 5.52 mmol) was then added to neat PPA (18.6 g) stirring at 100 °C under N₂. After 1.5 h, the reaction was cooled to room temperature, quenched with ice and 10% aqueous NaOH, and then extracted with EtOAc. The combined organic extracts were washed with saturated aqueous NaCl, dried over Na₂SO₄, decanted, and concentrated under reduced pressure. The crude product was purified by silica gel chromatography with EtOAc/hexanes (gradient 1:1 to 2:1) to give 0.81 g (43%) of the title compound as a yellow solid (mp 177.5-181 °C). ¹H NMR (*d*-DMSO) δ 7.75, 7.46, 7.09, 4.45-4.24, 3.93, 3.61, 3.34, 3.17, 3.11-2.80; IR (drift) 1676, 1628, 1587, 1493, 1483, 1466, 1428, 1357, 1324, 1295, 1276, 1266, 1191, 754, 706 cm⁻¹.

### Step 9. Preparation of 5,6,9,10,11,12-hexahydro-4H,8H-azepino[4',5':4,5]pyrrolo[3,2,1-ij]quinolin-4-one oxalate.

A solution of 10-benzoyl-5,6,9,10,11,12-hexahydro-4H,8H-azepino[4',5':4,5] pyrrolo[3,2,1-ij]quinolin-4- one (0.20 g, 0.58 mmol) in THF/ MeOH/50% aqueous NaOH (3:2:1, 9 mL) was heated under N₂ at reflux for 4 d. Cooled to room temperature, diluted with saturated aqueous NaCl, and extracted with CH₂Cl₂. The combined organic extracts were washed with saturated aqueous NaCl, dried over Na₂SO₄, decanted, and concentrated under reduced pressure. The crude product was purified by silica gel chromatography with CH₂Cl₂/MeOH/Et₂NH (gradient 95:5:0 to 95:4:1) to give 0.10 g (74%) of the free base of title compound as a dark yellow foam. The oxalate salt was prepared by treating a solution of the free base in methanol with a solution of oxalic acid in ether to give a precipitate that crystallized from methanol as small yellow needles of the title compound (mp 199-202 °C [dec.]). ¹H NMR (*d*-DMSO) δ 7.78, 7.46, 7.15, 4.39, 4.19, 3.39, 3.34, 3.24, 3.14, 3.02; IR (drift) 2965, 2890, 2841, 2799, 2732, 2516, 2484, 1720, 1673, 1626, 1607, 1591, 1492, 1460, 1206 cm⁻¹.

### EXAMPLE 42 Preparation of 2-fluoro-5,6,9,10,11,12-hexahydro-4H,8H-azepino[4',5':4,5]pyrrolo[3,2,1-ij]quinolin-4-one maleate.

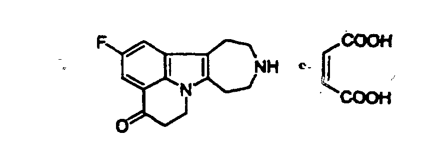

Following the general procedure of Example 41, making non-critical variations but starting with 3-benzoyl-9-fluoro-1,2,3,4,5,6-hexahydroazepino[4,5-b]indole and using maleic acid for the salt formation, the title compound was obtained (mp 242-244 °C [dec.]). ¹H NMR (*d*-DMSO) δ 9.18, 7.67, 7.21, 4.40, 3.33, 3.25, 3.12, 3.04; IR (drift) 3358, 2970, 2959, 2830, 2792, 2785, 2752, 1681, 1593, 1490, 1464, 1383, 1330, 1216, 907 cm⁻¹.

### EXAMPLE 43 Preparation of 2-chloro-5,6,9,10,11,12-hexahydro-4H,8H-azepino[4',5':4,5]pyrrolo[3,2,1-ij]quinolin-4-one oxalate.

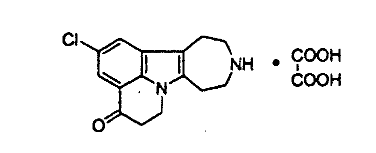

Following the general procedure of Example 41, making non-critical variations but starting with 3-benzoyl-9-chloro-1,2,3,4,5,6-hexahydroazepino[4,5-b]indole, the title compound was obtained (mp 236.5-238 °C [dec.]). ¹H NMR (*d*-DMSO) δ 7.89, 7.38, 4.41, 3.69, 3.38, 3.33, 3.23, 3.13, 3.04; IR (mull) 3440, 2644, 2514, 1915, 1686, 1491, 1420, 1327, 1318, 1251, 1138, 1094, 1030, 914, 720 cm⁻¹.

### EXAMPLE 44 Preparation of 6-methyl-5,6,9,10,11,12-hexahydro-4H,8H-azepino[4',5':4,5]pyrrolo[3,2,1-ij]quinolin-4-one oxalate.

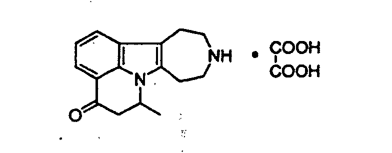

Following the general procedure of Example 41, making non-critical variations but using ethyl crotonate in step 7, the title compound was obtained (mp 215-215.5 °C). ¹H NMR (*d*-DMSO) δ 7.79, 7.47, 4.97, 3.50-3.20, 3.15, 2.73, 1.16; IR (drift) 3020, 2984, 2969, 1727, 1684, 1609, 1589, 1471, 1327, 1217, 1194, 1178, 1100, 752, 706 cm⁻¹.

### EXAMPLE 45 Preparation of 2-fluoro-6-methyl-5,6,9,10,11,12-hexahydro-4H,8H-azepino[4',5':4,5]pyrrolo[3,2,1-ij]quinolin-4-one oxalate.

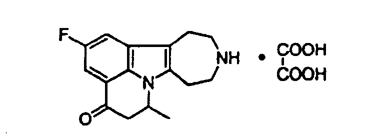

Following the general procedure of Example 41, making non-critical variations but starting with 3-benzoyl-9-fluoro-1,2,3,4,5,6-hexahydroazepino[4,5-b]indole and using ethyl crotonate in step 7, the title compound was obtained (mp 209.5-211.5 °C). ¹H NMR (*d*-DMSO) δ 7.69, 7.22, 4.99, 3.44-3.22, 3.12, 2.78, 1.16; IR (drift) 2969, 1743, 1727, 1716, 1688, 1646, 1616, 1480, 1418, 1377, 1214, 1145, 1102, 858, 602 cm⁻¹.

### EXAMPLE 46 Preparation of 2,3-dichloro-6-methyl-5,6,9,10,11,12-hexahydro-4H,8H-azepino[4',5':4,5]pyrrolo[3,2,1-ij]quinolin-4-one mesylate.

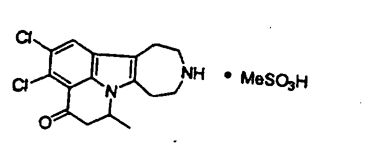

Following the general procedure of Example 41, making non-critical variations but starting with 3-benzoyl-8,9-dichloro-1,2,3,4,5,6-hexahydroazepino[4,5-b]indole, using ethyl crotonate in step 7, and using methanesulfonic acid for the salt formation, the title compound was obtained (mp 225-230 °C [dec.]). ¹H NMR (*d*-DMSO) δ 8.94, 8.12, 4.97, 3.48-3.20, 3.13, 2.77, 2.30, 1.20; IR (drift) 3029, 2979, 2849, 2792, 1683, 1481, 1410, 1312, 1214, 1196, 1184, 1161, 1145, 1040, 775 cm⁻¹.

### EXAMPLE 47 Preparation of 6-propyl-5,6,9,10,11,12-hexahydro-4H,8H-azepino[4',5':4,5]pyrrolo[3,2,1-ij]quinoun-4-one hydrochloride.

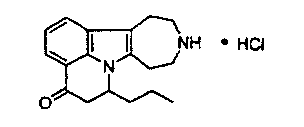

Following the general procedure of Example 41, making non-critical variations but using ethyl trans-hexenoate in step 7 and using hydrochloric acid for the salt formation, the title compound was obtained (mp 193-194.5 °C). ¹H NMR (*d*-DMSO) δ 9.38, 7.79, 7.45, 7.14, 4.87, 3.54, 3.45-3.19, 3.16, 2.83, 1.47, 1.13, 0.78; IR (drift) 2957, 2926, 2870, 2853, 2741, 1683, 1588, 1471, 1416, 1354, 1325, 1279, 1191, 795, 751 cm⁻¹.

### EXAMPLE 48 Preparation of 6-(trifluoromethyl)-5,6,9,10,11,12-hexahydro-4H,8H-azepino[4',5':4,5]pyrrolo[3,2,1-ij]quinolin-4-one oxalate.

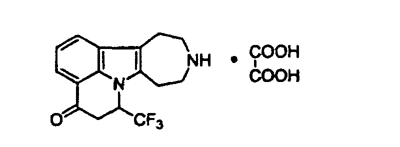

Following the general procedure of Example 41, making non-critical variations but using ethyl 4,4,4-tsifluorocrotonate in step 7 and using hydrochloric acid for the salt formation, the title compound was obtained (mp 201.5-202.5 °C). ¹H NMR (*d*-DMSO) δ 7.86, 7.54, 7.24, 5.94, 3.73, 3.67, 3.43-3.19, 3.16, 3.00; IR (drift) 1726, 1694, 1645, 1639, 1592, 1469, 1416, 1336, 1272, 1253, 1204, 1195, 1177, 1127, 945 cm⁻¹.

### EXAMPLE 49 Preparation of 5,6,8,9,10,11,12,12a-octahydro-4H,7aH-azepino[4',5':4,5]pyrrolo[3,2,1-ij]quinolin-4-ol

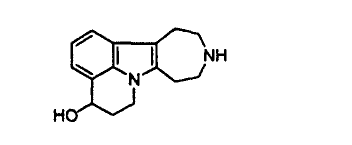

### Step 10 Preparation of 10-benzoyl-5,6,8,9,10,11,12,12a-octahydro-4H,7aH-azepino[4',5':4,5]pyrrolo[3,2,1-ij]quinolin-4-ol.

A solution of 10-benzoyl-5,6,9,10,11,12-hexahydro-4H,8H-azepino [4',5':4,5] pyrrolo[3,2,1-ij]quinolin-4- one (1.46 g, 4.24 mmol) in ethanol (40mL) was cooled to 0 °C under N₂. Added sodium borohydride (0.32 g, 8.46 mmol) and allowed to warm slowly to room temperature. Concentrated under reduced pressure to approximately 10 mL, diluted with H₂O, and extracted with CH₂Cl₂. The combined organic extracts were washed with saturated aqueous NaCl, dried over Na₂SO₄, decanted, and concentrated under reduced pressure. The crude product was purified by silica gel chromatography with EtOAc/heptanes (2:1) to give 1.24 g (84%) of the title compound as a yellow foam. ¹H NMR (CDCl₃) δ 7.42, 7.33, 7.12-7.02, 5.10, 4.13, 4.02, 3.68, 3.21, 2.92, 2.38, 2.23, 1.80; IR (drift) 1627, 1614, 1475, 1466, 1457, 1448, 1429, 1372, 1360, 1329, 1292, 1268, 785, 747, 706 cm⁻¹; MS (ESI) 369.1 (M⁺+Na).

The 10-benzoyl-5,6,8,9,10,11,12,12a-octahydro-4H,7aH-azepino[4',5':4,5] pyrrolo[3,2,1-ij]quinolin-4-ol was then hydrolyzed following the procedure outlined in step 9 to give 5,6,8,9,10,11,12,12a-octahydro-4H,7aH-azepino[4',5':4,5]pyrrolo[3,2,1-ij]quinolin-4-ol as a tan foam. ¹H NMR (CDCl₃) δ 7.43, 7.09, 5.11, 4.11, 3.16, 3.02, 2.38, 2.26; IR (drift) 3300, 3047, 2923, 2878, 2828, 2751, 1479, 1454, 1430, 1415, 1371, 1330, 1199, 1074, 746 cm⁻¹; HRMS (EI) calcd for C₁₅H₁₈N₂O 242.1419, found 242.1422.

### EXAMPLE 50 Preparation of 4-methoxy-5,6,8,9,10,11,12,12a-octahydro-4H,7aH-azepino[4',5':4,5]pyrrolo[3,2,1-ij]quinoline

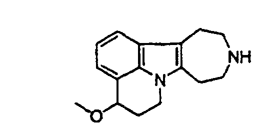

### Step 11. Preparation of 10-benzoyl-4-methoxy-5,6,8,9,10,11,12,12a-octahydro-4H,7aH-azepino[4',5':4,5]pyrrolo[3,2,1-ij]quinoline.

NaH (60% dispersion in mineral oil, 0.50g, 1.2 mmol) was added to a solution of 10-benzoyl-5,6,8,9,10,11,12,12a-octahydro-4H,7aH-azepino[4',5':4,5]pyrrolo[3,2,1-ij]quinolin-4-ol (0.20 g, 0.58 mmol) in DMF (5.0 mL) at 0 °C under N₂. After 30 min, iodomethane (0.040 mL, 0.64 mmol) was added. The reaction was quenched with saturated aqueous NH₄Cl after 1 h and then allowed to warm to room temperature prior to extracting with EtOAc. The combined organic extracts were washed with saturated aqueous NaCl, dried over Na₂SO₄, decanted, and concentrated under reduced pressure. The crude product was purified by silica gel chromatography with EtOAc/heptanes (2:1) to give 0.16 g (79%) of the title compound as a beige foam. ¹H NMR (CDCl₃) δ 7.46-7.30, 6.94, 4.51, 4.22-4.03, 4.01-3.79, 3.65-3.41, 3.29, 3.12, 3.04, 2.90, 2.82, 2.34, 2.08; IR (drift) 1631, 1493, 1477, 1459, 1422, 1370, 1359, 1324, 1292, 1267, 1098, 1085, 786, 748, 706 cm⁻¹; HRMS (FAB) calcd for C₂₃H₂₄N₂O₂+H 361.1916, found 361.1913.

Following the general procedure of step 4, making non-critical variations but starting with 10-benzoyl-4-methoxy-5,6,8,9,10,11,12,12a-octahydro-4H,7aH-azepino[4',5' :4,5]pyrrolo[3,2,1-ij]quinoline, the title compound was obtained as a brown oil. ¹H NMR (CDCl₃) δ 7.45, 7.07, 4.56, 4.26, 4.09, 3.42, 3.27-3.13, 3.11-2.94, 2.49, 2.18; IR (liq.) 2926, 2905, 2882, 2820, 1492, 1479, 1453, 1415, 1370, 1332, 1200, 1098, 1083, 1066, 748 cm⁻¹, HRMS (FAB) calcd for C₁₆H₂₀N₂O+H 257.1654, found 257.1665.

### EXAMPLE 51 Preparation of 4-phenoxy-5,6,9,10,11,12-hexahydro-4H,8H-azepino[4',5':4,5]pyrrolo[3,2,1-ij]quinoline

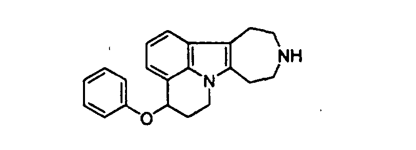

### Step 12. Preparation of 10-benzoyl-4-phenoxy-5,6,9,10,11,12-hexahydro-4H,8H-azepino[4',5':4,5]pyrrolo[3,2,1-ij]quinoline

1,1'-Azobis(N,N-dimethylformamide) (0.34 g, 2.0 mmol) was added to a solution of 10-benzoyl-5,6,8,9,10,11,12,12a-octahydro-4H,7aH-azepino[4',5':4,5]pyrrolo [3,2,1-ij]quinolin-4-ol (0.45 g, 1.3 mmol), phenol (0.18 g, 1.9 mmol), and tributylphosphine (0.49 mL, 2.0 mmol) in dry benzene (4.0 mL) at room temperature under N₂. The reaction mixture immediately congealed but resumed stirring upon heating to 60 °C. After 5 h, the reaction was cooled to room temperature and filtered to remove white precipitate. The filtrate was concentrated under reduced pressure and purified by silica gel chromatography with heptanes/EtOAc (gradient 3:1 to 3:2) to give 0.26 g (47%) of the title compound as a beige foam. ¹H NMR (CDCl₃) δ 7.52-7.38, 7.32, 7.11-6.95, 5.68, 4.21-3.90, 3.69, 3.21, 2.91, 2.63, 2.34; IR (drift) 1630, 1596, 1492, 1458, 1421, 1359, 1327, 1291, 1267, 1226, 1192, 786, 750, 706, 694 cm⁻¹; MS (ESI) 423.3 (M⁺+H).

Following the general procedure of step 4, making non-critical variations but starting with 10-benzoyl-4-phenoxy-5,6,9,10,11,12-hexahydro-4H,8H-azepino [4',5':4,5]pyrrolo[3,2,1-ij]quinoline, the title compound was obtained. MS (ESI) 319.2 (M⁺+H).

### EXAMPLE 52 Using synthetic procedures similar to those described herein, the following compounds of formula (I) wherein R₂ is hydrogen can also be prepared:

4-bromo-1,2,8,9,10,11-hexahydro-7H-azepino[4,5-b][1,4]oxazino[2,3,4-hi]indole (100);
5-bromo-1,2,8,9,10,11-hexahydro-7H-azepino[4,5-b][1,4]oxazino[2,3,4-hi]indole (101);
6-bromo-1,2,8,9,10,11-hexahydro-7H-azepino[4,5-b][1,4]oxazino[2,3,4-hi]indole (102);
5,6-dibromo-1,2,8,9,10,11-hexahydro-7H-azepino[4,5-b][1,4]oxazino[2,3,4-hi]indole (103);
4,6-dibromo-1,2,8,9,10,11-hexahydro-7H-azepino[4,5-b][1,4]oxazino[2,3,4-hi]indole (104);
4-methoxy-1,2,8,9,10,11-hexahydro-7H-azepino[4,5-b][1,4]oxazino[2,3,4-hi]indole (105);
5-methoxy-1,2,8,9,10,11-hexahydro-7H-azepino[4,5-b][1,4]oxazino[2,3,4-hi]indole (106);
6-methoxy-1,2,8,9,10,11-hexahydro-7H-azepino[4,5-b][1,4]oxazino[2,3,4-hi]indole (107);
4-(triflouromethyl)-1,2,8,9,10,11-hexahydro-7H-azepino[4,5-b][1,4] oxazino[2,3,4-hi]indole (108);
5-(triflouromethyl)-1,2,8,9,10,11-hexahydro-7H-azepino[4,5-b][1,4] oxazino[2,3,4-hi]indole (109);
4-benzyloxy-1,2,8,9,10,11-hexahydro-7H-azepino[4,5-b][1,4]oxazino[2,3,4-hi]indole (110);
5-benzyloxy-1,2,8,9,10,11-hexahydro-7H-azepino[4,5-b][1,4]oxazino[2,3,4-hi]indole (111);
6-benzyloxy-1,2,8,9,10,11-hexahydro-7H-azepino[4,5-b][1,4]oxazino[2,3,4-hi]indole (112);
4-fluoro-1,2,8,9,10,11-hexahydro-7H-azepino[4,5-b][1,4]thiazino[2,3,4-hi]indole (113);
5-fluoro-1,2,8,9,10,11-hexahydro-7H-azepino[4,5-b][1,4]thiazino[2,3,4-hi]indole (114);
6-fluoro-1,2,8,9,10,11-hexahydro-7H-azepino[4,5-b][1,4]thiazino[2,3,4-hi]indole (115);
4-chloro-1,2,8,9,10,11-hexahydro-7H-azepino[4,5-b][1,4]thiazino[2,3,4-hi]indole (116);
5-chloro-1,2,8,9,10,11-hexahydro-7H-azepino[4,5-b][1,4]thiazino[2,3,4-hi]indole (117);
6-chloro-1,2,8,9,10,11-hexahydro-7H-azepino[4,5-b][1,4]thiazino[2,3,4-hi]indole (118);
4,5-difluoro-1,2,8,9,10,11-hexahydro-7H-azepino[4,5-b][1,4]thiazino[2,3,4-hi]indole (119);
5,6-difluoro-1,2,8,9,10,11-hexahydro-7H-azepino[4,5-b][1,4]thiazino[2,3,4-hi]indole (120);
4,6-difluoro-1,2,8,9,10,11-hexahydro-7H-azepino[4,5-b][1,4]thiazino[2,3,4-hi]indole (121);
4-chloro-5-fluoro-1,2,8,9,10,11-hexahydro-7H-azepino[4,5-b][1,4] thiazino[2,3,4-hi]indole (122);
4-chloro-6-fluoro-1,2,8,9,10,11-hexahydro-7H-azepino[4,5-b][1,4] thiazino[2,3,4-hi]indole (123);
5-chloro-6-fluoro-1,2,8,9,10,11-hexahydro-7H-azepino[4,5-b][1,4] thiazino[2,3,4-hi]indole (124);
6-chloro-5-fluoro-1,2,8,9,10,11-hexahydro-7H-azepino[4,5-b][1,4] thiazino[2,3,4-hi]indole (125);
4-methyl-1,2,8,9,10,11-hexahydro-7H-azepino[4,5-b][1,4] thiazino[2,3,4-hi]indole (126);
5-methyl-1,2,8,9,10,11-hexahydro-7H-azepino[4,5-b][1,4] thiazino[2,3,4-hi]indole (127);
6-methyl-1,2,8,9,10,11-hexahydro-7H-azepino[4,5-b][1,4] thiazino[2,3,4-hi]indole (128);
4-methoxy-1,2,8,9,10,11-hexahydro-7H-azepino[4,5-b][1,4] thiazino[2,3,4-hi]indole (129);
5-methoxy-1,2,8,9,10,11-hexahydro-7H-azepino[4,5-b][1,4] thiazino[2,3,4-hi]indole (130);
6-methoxy-1,2,8,9,10,11-hexahydro-7H-azepino[4,5-b][1,4] thiazino[2,3,4-hi]indole; (131)
4-(triflouromethyl)-1,2,8,9,10,11-hexahydro-7H-azepino[4,5-b][1,4] thiazino[2,3,4-hi]indole (132);
5-(triflouromethyl)-1,2,8,9,10,11-hexahydro-7H-azepino[4,5-b][1,4] thiazino[2,3,4-hi]indole (133);
6-(triflouromethyl)-1,2,8,9,10,11-hexahydro-7H-azepino[4,5-b][1,4] thiazino[2,3,4-hi]indole (134);
4-benzyloxy-1,2,8,9,10,11-hexahydro-7H-azepino[4,5-b][1,4] thiazino[2,3,4-hi]indole (135);
5-benzyloxy-1,2,8,9,10,11-hexahydro-7H-azepino[4,5-b][1,4] thiazino[2,3,4-hi]indole (136);
6-benzyloxy-1,2,8,9,10,11-hexahydro-7H-azepino[4,5-b][1,4] thiazino[2,3,4-hi]indole (137);
1-fluoro-4,5,8,9,10,11-hexahydro-7H-azepino[4,5-b]pyrrolo[3,2,1-hi]indole (138);
3-fluoro-4,5,8,9,10,11-hexahydro-7H-azepino[4,5-b]pyrrolo[3,2,1-hi]indole (139);
1-bromo-4,5,8,9,10,11-hexahydro-7H-azepino[4,5-b]pyrrolo[3,2,1-hi]indole (140);
2-bromo-4,5,8,9,10,11-hexahydro-7H-azepino[4,5-b]pyrrolo[3,2,1-hi]indole (141);
3-bromo-4,5,8,9,10,11-hexahydro-7H-azepino[4,5-b]pyrrolo[3,2,1-hi]indole (142);
3-chloro-1-4,5,8,9,10,11-hexahydro-7H-azepino[4,5-b]pyrrolo[3,2,1-hi]indole (143);
2-chloro-1-fluoro-4,5,8,9,10,11-hexahydro-7H-azepmo[4,5-b]pyrrolo[3,2,1-hi]indole (144);
1-methoxy-4,5,8,9,10,11-hexahydro-7H-azepino[4,5-b]pyrrolo[3,2,1-hi]indole (145);
3-methoxy-4,5,8,9,10,11-hexahydro-7H-azepino[4,5-b]pyrrolo[3,2,1-hi]indole (146);
4-methyl-4,5,8,9,10,11-hexahydro-7H-azepino[4,5-b]pyrrolo[3,2;1-hi]indole (147);
1-(trifluoromethyl)-4,5,8,9,10,11-hexahydro-7H-azepino[4,5-b]pyrrolo[3,2,1-hi]indole (148);
2-(trifluoromethyl)-4,5,8,9,10,11-hexahydro-7H-azepino[4,5-b]pyrrolo[3,2,1-hi]indole (149);
3-(trifluoromethyl)-4,5,8,9,10,11-hexahydro-7H-azepino[4,5-b]pyrrolo[3,2,1-hi]indole (150);
4-benzyloxy-5,6,8,9,10,11,12,12a-octahydro-4H,7aH-azepino[4',5':4,5]pyrrolo[3,2,1-ij]quinoline (151);
4-(3-chlorophenoxy)-5,6,9,10,11,12-hexahydro-4H,8H-azepino[4',5':4,5]pyrrolo[3,2,1-ij]quinoline (152);
4-(2-chlorophenoxy)-5,6,9,10,11,12-hexahydro-4H,8H-azepino[4',5':4,5]pyrrolo[3,2,1-ij]quinoline (153);
4-(4-methoxyphenoxy)-5,6,9,10,11,12-hexahydro-4H,8H-azepino[4',5':4,5]pyrrolo[3,2,1-ij]quinoline (154);
4-(3-methoxyphenoxy)-5,6,9,10,11,12-hexahydro-4H,8H-azepino[4',5':4,5]pyrrolo[3,2,1-ij]quinoline (155);
4-(2-methoxyphenoxy)-5,6,9,10,11,12-hexahydro-4H,8H-azepino[4',5':4,5]pyrrolo[3,2,1-ij]quinoline (156);
4-(4-bromo-2-methoxypheaoxy)-5,6,9,10,11,12-hexahydro-4H,8H-azepino[4',5':4,5]pyrrolo[3,2,1-ij]quinoline (157);
5,6,9,10,11,12-hexahydro-4H,8H-azepino[4',5':4,5]pyrrolo[3,2,1-ij]quinolin-4-amine (158);
N-phenyl-5,6,9,10,11,12-hexahydro-4H,8H-azepino[4',5':4,5]pyrrolo[3,2,1-ij]quinolin-4-amine (159);
N-(4-chlorophenyl)-5,6,9,10,11,12-hexahydro-4H,8H-azepino[4',5':4,5]pyrrolo[3,2,1-ij]quinolin-4-amine (160);
N-(3-chlorophenyl)-5,6,9,10,11,12-hexahydro-4H,8H-azepino[4',5':4,5]pyrrolo[3,2,1-ij]quinolin-4-amine (161);
N-(2-chlorophenyl)-5,6,9,10,11,12-hexahydro-4H,8H-azepino[4',5':4,5]pyrrolo[3,2,1-ij]quinolin-4-amine (162);
N-(4-methoxyphenyl)-5,6,9,10,11,12-hexahydro-4H,8H-azepino[4',5':4,5]pyrolo[3,2,1-ij]quinolin-4-arnine (163);
N-(3-methoxyphenyl)-5,6,9,10,11,12-hexahydro-4H,8H-azepino[4',5':4,5]pyrrolo[3,2,1-ij]quinolin-4-amine (164);
N-(2-methoxyphenyl)-5,6,9,10,11,12-hexahydro-4H,8H-azepino[4',5':4,5]pyrrolo[3,2,1-ij]quinolin-4-amine (165);
N-(4-bromo-2-methoxyphenyl)-5,6,9,10,11,12-hexahydro-4H,8H-azepino[4',5':4,5]pyrrolo[3,2,1-ij]quinolin-4-amine (166);
5,6,9,10,11,12-hexahydro-4H,8H-azepino[4',5':4,5]pyrrolo[3,2,1-ij]quinoline-4-thione (167);
4-(phenylsulfonyl)-5,6,9,10,11,12-hexahydro-4H,8H-azepino[4',5':4,5]pyrrolo[3,2,1-ij]quinoline (168);
4-(4-chlorophenylsulfonyl)-5,6,9,10,11,12-hexahydro-4H,8H-azepino[4',5':4,5]pyrrolo[3,2,1-ij]quinoline (169);
4-(3-chlorophenylsulfonyl)-5,6,9,10,11,12-hexahydro-4H,8H-azepino[4',5':4,5]pyrrolo[3,2,1-ij]quinoline (170);
4-(2-chlorophenylsulfonyl)-5,6,9,10,11,12-hexahydro-4H,8H-azepino[4',5':4,5]pyrrolo[3,2,1-ij]quinoline (171);
4-(4-methoxyphenylsulfonyl)-5,6,9,10,11,12-hexahydro-4H,8H-azepino[4',5':4,5]pyrrolo[3,2,1-ij]quinpline (172);
4-(3-methoxyphenylsulfonyl)-5,6,9,10,11,12-hexahydro-4H,8H-azepino[4',5':4,5]pyrrolo[3,2,1-ij]quinoline (173);
4-(2-methoxyphenylsulfonyl)-5,6,9,10,11,12-hexahydro-4H,8H-azepino[4',5':4,5]pyrrolo[3,2,1-ij]quinoline (174);
4-(4-bromo-2-methoxyphenylsulfonyl)-5,6,9,10,11,12-hexahydro-4H,8H-azepino[4',5':4,5]pyrrolo[3,2,1-ij]quinoline (175);
5,6,8,9,10,11,12,12a-octahydro-4H,7aH-azepino[4',5':4,5]pyrrolo[3,2,1-ij]quinolin-5-ol (176);
5-methoxy-5,6,8,9,10,11,12,12a-octahydro-4H,7aH-azepino[4',5':4,5]pyrrolo[3,2,1-ij]quinoline (177);
5-benzyloxy-5,6,8,9,10,11,12,12a-octahydro-4H,7aH-azepino[4',5':4,5]pyrrolo[3,2,1-ij]quinoline (178);
5-phenoxy-5,6,9,10,11,12-hexahydro-4H,8H-azepino[4',5':4,5]pyrrolo[3,2,1-ij]quinoline (179);
5-(4-chlorophenoxy)-5,6,9,10,11,12-hexahydro-4H,8H-azepino[4',5':4,5]pyrrolo[3,2,1-ij]quinoline (180);
5-(3-chlorophenoxy)-5,6,9,10,11,12-hexahydro-4H,8H-azepino[4',5':4,5]pyrrolo[3,2,1-ij]quinoline (181);
5-(2-chlorophenoxy)-5,6,9,10,11,12-hexahydro-4H,8H-azepino[4',5':4,5]pyrrolo[3,2,1-ij]quinoline (182);
5-(4-methoxyphenoxy)-5,6,9,10,11,12-hexahydro-4H,8H-azepino[4',5':4,5]pyrrolo[3,2,1-ij]quinoline (183);
5-(3-methoxyphenoxy)-5,6,9,10,11,12-hexahydro-4H,8H-azepino[4',5':4,5]pyrrolo[3,2,1-ij]quinoline (184);
5-(2-methoxyphenoxy)-5,6,9,10,11,12-hexahydro-4H,8H-azepino[4',5':4,5]pyrrolo[3,2,1-ij]quinoline (185);
5-(4-bromo-2-methoxyphenoxy)-5,6,9,10,11,12-hexahydro-4H,8H-azepino[4',5':4,5]pyrrolo[3,2,1-ij]quinoline (186);
5,6,9,10,11,12-hexahydro-4H,8H-azepino[4',5':4,5]pyrrolo[3,2,1-ij]quinolin-5-amine (187);
N-phenyl-5,6,9,10,11,12-hexahydro-4H,8H-azepino[4',5':4,5]pyrrolo[3,2,1-ij]quinolin-5-amine (188);
N-(4-chlorophenyl)-5,6,9,10,11,12-hexahydro-4H,8H-azepino[4',5':4,5]pyrrolo[3,2,1-ij]quinolin-5-amine (189);
N-(3-chlorophenyl)-5,6,9,10,11,12-hexahydro-4H,8H-azepino[4',5':4,5]pyrrolo[3,2,1-ij]quinolin-5-amine (190);
N-(2-chlorophenyl)-5,6,9,10,11,12-hexahydro-4H,8H-azepino[4',5':4,5]pyrrolo[3,2,1-ij]quinolin-5-amine (191);
N-(4-methoxyphenyl)-5,6,9,10,11,12-hexahydro-4H,8H-azepino[4',5':4,5]pyrrolo[3,2,1-ij]quinolin-5-amine (192);
N-(3-methoxyphenyl)-5,6,9,10,11,12-hexahydro-4H,8H-azepino[4',5':4,5]pyrrolo[3,2,1-ij]quinolin-5-amine (193);
N-(2-methoxyphenyl)-5,6,9,10,11,12-hexahydro-4H,8H-azepino[4',5':4,5]pyrrolo[3,2,1-ij]quinolin-5-amine (194);
N-(4-bromo-2-methoxyphenyl)-5,6,9,10,11,12-hexahydro-4H,8H-azepino[4',5':4,5]pyrrolo[3,2,1-ij]quinolin-5-amine (195);
5,6,9,10,11,12-hexahydro-4H,8H-azepino[4',5':4,5]pyirolo[3,2,1-ij]quinoline-5-thione (196);
5-(phenylsulfonyl)-5,6,9,10,11,12-hexahydro-4H,8H-azepino[4',5':4,5]pyrrolo[3,2,1-ij]quinoline (197);
5-(4-chlorophenylsulfonyl)-5,6,9,10,11,12-hexahydro-4H,8H-azepino[4',5':4,5]pyrrolo[3,2,1-ij]quinoline (198);
5-(3-chlorophenylsulfonyl)-5,6,9,10,11,12-hexahydro-4H,8H-azepino[4',5':4,5]pyrrolo[3,2,1-ij]quinoline (199);
5-(2-chlorophenylsulfonyl)-5,6,9,10,11,12-hexahydro-4H,8H-azepino[4',5':4,5]pyrrolo[3,2,1-ij]quinoline (200);
5-(4-methoxyphenylsulfonyl)-5,6,9,10,11,12-hexahydro-4H,8H-azepino[4',5':4,5]pyrrolo[3,2,1-ij]quinoline (201);
5-(3-methoxyphenylsulfonyl)-5,6,9,10,11,12-hexahydro-4H,8H-azepino[4',5':4,5]pyrrolo[3,2,1-ij]quinoline (202);
5-(2-methoxyphenylsulfonyl)-5,6,9,10,11,12-hexahydro-4H,8H-azepino[4',5':4,5]pyrrolo[3,2,1-ij]quinoline (203);
5-(4-bromo-2-methoxyphenylsulfonyl)-5,6,9,10,11,12-hexahydro-4H,8H-azepino[4',5':4,5]pyrrolo[3,2,1-ij]quinoline (204);
4-(4-chlorophenoxy)-5,6,9,10,11,12-hexahydro-4H,8H-azepino[4',5':4,5]pyrrolo[3,2,1-ij]quinoline (205);
1-[2-(4-fluorophenoxy)ethoxy]-5,6,9,10,11,12-hexahydro-4H,8H-azepino[4',5':4,5]pyrrolo[3,2,1-ij]quinoline (206);
1-[2-(3-fluorophenoxy)ethoxy]-5,6,9,10,11,12-hexahydro-4H,8H-azepino[4',5':4,5)pyrrolo[3,2,1-ij]quinoline (207);
1-[2-(2-fluorophenoxy)ethoxy]-5,6,9,10,11,12-hexahydro-4H,8H-azepino[4',5':4,5]pyrrolo[3,2,1-ij]quinoline (208);
1-[2-(4-chlorophenoxy)ethoxy]-5,6,9,10,11,12-hexahydro-4H,8H-azepino[4',5':4,5]pyrrolo[3,2,1-ij]quinoline (209);
1-[2-(3-chlorophenoxy)ethoxy]-5,6,9,10,11,12-hexahydro-4H,8H-azepino[4',5':4,5]pyrrolo[3,2,1-ij]quinoline (210);
1-[2-(2-chlorophenoxy)ethoxy]-5,6,9,10,11,12-hexahydro-4H,8H-azepino[4',5':4,5]pyrrolo[3,2,1-ij]quinoline (211);
1-[2-(4-bromophenoxy)ethoxy]-5,6,9,10,11,12-hexahydro-4H,8H-azepino[4',5':4,5]pyrrolo[3,2,1-ij]quinoline (212);
1-[2-(3-bromophenoxy)ethoxy]-5,6,9,10,11,12-hexahydro-4H,8H-azepino[4',5':4,5]pyrrolo[3,2,1-ij]quinoline (213);
1-[2-(2-bromophenoxy)ethoxy]-5,6,9,10,11,12-hexahydro-4H,8H-azepino[4',5':4,5]pyrrolo[3,2,1-ij]quinoline (214);
1-[2-(4-methoxyphenoxy)ethoxy]-5,6,9,10,11,12-hexahydro-4H,8H-azepino[4',5':4,5]pyrrolo[3,2,1-ij]quinoline (215);
1-[2-(3-methoxyphenoxy)ethoxy]-5,6,9,10,11,12-hexahydro-4H,8H-azepino[4',5':4,5]pyrrolo[3,2,1-ij]quinoline (216);
1-[2-(2-methoxyphenoxy)ethoxy]-5,6,9,10,11,12-hexahydro-4H,8H-azepino[4',5':4,5]pyrrolo[3,2,1-ij]quinoline (217);
1-[2-(4-methylphenoxy)ethoxy]-5,6,9,10,11,12-hexahydro-4H,8H-azepino[4',5':4,5]pyrrolo[3,2,1-ij]quinoline (218);
1-[2-(3-methylphenoxy)ethoxy]-5,6,9,10,11,12-hexahydro-4H,8H-azepino[4',5':4,5]pyrrolo[3,2,1-ij]quinoline (219);
1-[2-(2-methylphenoxy)ethoxy]-5,6,9,10,11,12-hexahydro-4H,8H-azepino[4',5':4,5]pyrrolo[3,2,1-ij]quinoline (220);
1-[2-(1-naphthyloxy)ethoxy]-5,6,9,10,11,12-hexahydro-4H,8H-azepino[4',5':4,5]pyrrolo[3,2,1-ij]quinoline (221);
1-[2-(2-naphthyloxy)ethoxy]-5,6,9,10,11,12-hexahydro-4H,8H-azepino[4',5':4,5]pyrrolo[3,2,1-ij]quinoline (222);
1-[2-([1,1'-biphenyl]-4-yloxy)ethoxy]-5,6,9,10,11,12-hexahydro-4H,8H-azepino[4',5':4,5]pyrrolo[3,2,1-ij]quinoline (223);
1-[2-([1,1'-biphenyl]-3-yloxy)ethoxy]-5,6,9,10,11,12-hexahydro-4H,8H-azepino[4',5':4,5]pyrrolo[3,2,1-ij]quinoline (224);
1-[2-([1,1'-biphenyl]-2-yloxy)ethoxy]-5,6,9,10,11,12-hexahydro-4H,8H-azepino[4',5':4,5]pyrrolo[3,2,1-ij]quinoline (225);
1- {2-[4-(trifluoromethoxy)phenoxy]ethoxy}-5,6,9,10,11,12-hexahydro-4H,8H-azepino[4',5':4,5]pyrrolo[3,2,1-ij]quinoline (226);
1-{2-[3-(trifluoromethoxy)phenoxy]ethoxy}-5,6,9,10,11,12-hexahydro-4H,8H-azepino[4',5':4,5]pyrrolo[3,2,1-ij]quinoline (227);
1- {2-[2-(trifluoromethoxy)phenoxy]ethoxy}-5,6,9,10,11,12-hexahydro-4H,8H-azepino[4',5':4,5]pyrrolo[3,2,1-ij]quinoline (228);
1-{2-[4-(trifluoromethyl)phenoxy]ethoxy}-5,6,9,10,11,12-hexahydro-4H,8H-azepino[4',5':4,5]pyrrolo[3,2,1-ij]quinoline (229);
1-{2-[3-(trifluoromethyl)phenoxy]ethoxy}-5,6,9,10,11,12-hexahydro-4H,8H-azepino[4',5':4,5]pyrrolo[3,2,1-ij]quinoline (230); and
1- {2-[2-(trifluoromethyl)phenoxy]ethoxy}-5,6,9,10,11,12-hexahydro-4H,8H-azepino[4',5':4,5]pyrrolo[3,2,1-ij]quinoline (231).

### EXAMPLE 53 Using synthetic procedures similar to those described herein, the following compounds of formula (I) wherein R₂ is a protecting group can also be prepared:

benzyl 5,6,8,9,11,12-hexahydro-4H,10H-azepino[4',5':4,5]pyrrolo[3,2,1-ij]quinoline-10-carboxylate;
benzyl 2-methyl-5,6,8,9,11,12-hexahydro-4H,10H-azepino[4',5':4,5]pyrrolo[3,2,1-ij]quinoline-10-carboxylate;
10-benzoyl-1-methoxy-5,6,9,10,11,12-hexahydro-4H,8H-azepino[4',5':4,5]pyrrolo-[3,2,1-ij]quinoline;
benzyl 2-fluoro-5,6,8,9,11,12-hexahydro-4H,10H-azepino[4',5':4,5]pyrrolo[3,2,1-ij]quinoline-10-carboxylate;
benzyl 6-methyl-5,6,8,9,11,12-hexahydro-4H,10H-azepino[4',5':4,5]pyrrolo[3,2,1-ij]quinoline-10-carboxylate;
benzyl 5-methyl-5,6,8,9,11,12-hexahydro-4H,10H-azepino[4',5':4,5]pyrrolo[3,2,1-ij]quinoline-10-carboxylate;
benzyl 4-methyl-5,6,8,9,11,12-hexahydro-4H,10H-azepino[4',5':4,5]pyrrolo[3,2,1-ij]quinoline-10-carboxylate;
(+)-benzyl 6-methyl-5,6,8,9,11,12-hexahydro-4H,10H-azepino[4',5':4,5]pyrrolo[3,2,1-ij]quinoline-10-carboxylate;
(-)-benzyl 6-methyl-5,6,8,9,11,12-hexahydro-4H,10H-azepino[4',5':4,5]pyrrolo[3,2,1-ij]quinoline-10-carboxylate;
(+)-benzyl 5-methyl-5,6,8,9,11,12-hexahydro-4H,10H-azepino[4',5':4,5]pyrrolo[3,2,1-ij]quinoline-10-carboxylate;
(-)-benzyl 5-methyl-5,6,8,9,11,12-hexahydro-4H,10H-azepino[4',5':4,5]pyrrolo[3,2,1-ij]quinoline-10-carboxylate;
(+)-benzyl 4-methyl-5,6,8,9,11,12-hexahydro-4H,10H-azepino[4',5':4,5]pyrrolo[3,2,1-ij]quinoline-10-carboxylate;
(-)-benzyl 4-methyl-5,6,8,9,11,12-hexahydro-4H,10H-azepino[4',5':4,5]pyrrolo[3,2,1-ij]quinoline-10-carboxylate;
benzyl 2-methyl-1,2,7,8,10,11-hexahydro-9H-azepino[4,5-b][1,4]oxazino[2,3,4-hi]indole-9-carboxylate;
benzyl 2,2-dimethyl-1,2,7,8,10,11-hexahydro-9H-azepino[4,5-b][1,4]oxazino[2,3,4-hi]indole-9-carboxylate;
benzyl 4-fluoro-1,2,7,8,10,11-hexahydro-9H-azepino[4,5-b][1,4]oxazino[2,3,4-hi]indole-9-carboxylate;
benzyl 4-chloro-1,2,7,8,10,11-hexahydro-9H-azepino[4,5-b][1,4]oxazino[2,3,4-hi]indole-9-carboxylate;
benzyl 5-fluoro-1,2,7,8,10,11-hexahydro-9H-azepino[4,5-b][1,4]oxazino[2,3,4-hi]indole-9-carboxylate;
benzyl 5-chloro-1,2,7,8,10,11-hexahydro-9H-azepino[4,5-b][1,4]oxazino[2,3,4-hi]indole-9-carboxylate;
benzyl 5-methyl-1,2,7,8,10,11-hexahydro-9H-azepino[4,5-b][1,4]oxazino[2,3,4-hi]indole-9-carboxylate;
benzyl 6-fluoro-1,2,7,8,10,11-hexahydro-9H-azepino[4,5-b][1,4]oxazino[2,3,4-hi]indole-9-carboxylate;
benzyl 6-chloro-1,2,7,8,10,11-hexahydro-9H-azepino[4,5-b][1,4]oxazino[2,3,4-hi]indole-9-carboxylate;
benzyl 6-methyl-1,2,6b,7,8,10,11,11a-octahydro-9H-azepino[4,5-b][1,4]oxazino[2,3,4-hi]indole-9-carboxylate;
benzyl 6-(trifluoromethyl)-1,2,7,8,10,11-hexahydro-9H-azepino[4,5-b][1,4]oxazino[2,3,4-hi]indole-9-carboxylate;
benzyl 5,6-difluoro-1,2,7,8,10,11-hexahydro-9H-azepino[4,5-b] [1,4]oxazino[2,3,4-hi]indole-9-carboxylatebenzyl;
benzyl 5-chloro-6-fluoro-1,2,7,8,10,11-hexahydro-9H-azepino[4,5-b][1,4]oxazino[2,3,4-hi]indole-9-carboxylate;
benzyl 5-fluoro-6-chloro-1,2,7,8,10,11-hexahydro-9H-azepino[4,5-b][ 1,4]oxazino[2,3,4-hi]indole-9-carboxylate;
benzyl 5,6-dichloro-1,2,7,8,10,11-hexahydro-9H-azepino[4,5-b] [1,4]oxazino[2,3,4-hi]indole-9-carboxylate;
benzyl 4,6-dichloro-1,2,7,8,10,11-hexahydro-9H-azepino[4,5-b][1,4]oxazino[2,3,4-hi]indole-9-carboxylate;
7-benzoyl 4,5,8,9,10,11-hexahydro-7H-azepino[4,5-b]pyrrolo[3,2,1-hi]indole;
7-benzoyl 2-fluoro-4,5,8,9,10,11-hexahydro-7H-azepino[4,5-b]pyrrolo[3,2,1-hi]indole;
7-benzoyl 2-methoxy-4,5,8,9,10,11-hexahydro-7H-azepino[4,5-b]pyrrolo[3,2,1-hi]indole;
benzyl-5-methyl-4,5,8,9,10,11-hexahydro-7H-azepino[4,5-b]pyrrolo[3,2,1-hi]-7-carboxylate;
benzyl-4,5-dimethyl-4,5,8,9,10,11-hexahydro-7H-azepino[4,5-b]pyrrolo[3,2,1-hi]indole-7-carboxylate;
benzyl-2,3,4,5,8b,9,10,11,12,12a-decahydro-1H-azepino[4',5':4,5]pyrrolo[3,2,1-jk]carbazole-7-carboxylate;
benzyl-1-chloro-2-fluoro-4,5,8,9,10,11-hexahydro-7H-azepino[4,5-b]pyrrolo[3,2,1-hi]indole-7-carboxylate;
benzyl-2-chloro-4,5,8,9,10,11-hexahydro-7H-azepino[4,5-b]pyrrolo[3,2,1-hi]indole-7-carboxylate;
benzyl-1-chloro-4,5,8,9,10,11-hexahydro-7H-azepino[4,5-b]pyrrolo[3,2,1-hi]indole-7-carboxylate;
10-benzoyl-5,6,9,10,11,12-hexahydro-4H,8H-azepino[4',5':4,5]pyrrolo[3,2,1-ij]quinolin-4- one;
10-benzoyl-2-fluoro-5,6,9,10,11,12-hexahydro-4H,8H-azepino[4',5':4,5]pyrrolo[3,2,1-ij]quinolin-4-one;
10-benzoyl-2-chloro-5,6,9,10,11,12-hexahydro-4H,8H-azepino[4',5':4,5]pyrrolo[3,2,1-ij]quinolin-4-one;
10-benzoyl-6-methyl-5,6,9,10,11,12-hexahydro-4H,8H-azepino[4',5':4,5]pyrrolo-[3,2,1-ij]quinolin-4-one;
10-benzoyl-2-fluoro-6-methyl-5,6,9,10,11,12-hexahydro-4H,8H-azepino[4',5':4,5]-pyrrolo[3,2,1-ij]quinolin-4-one;
10-benzoyl-2,3-dichloro-6-methyl-5,6,9,10,11,12-hexahydro-4H,8H-azepino[4',5':4,5]pyrrolo[3,2,1-ij]quinolin-4-one;
10-benzoyl-6-propyl-5,6,9,10,11,12-hexahydro-4H,8H-azepino[4',5':4,5]pyrrolo[3,2,1-ij]quinolin-4-one;
10-benzoyl-6-(trifluoromethyl)-5,6,9,10,11,12-hexahydro-4H,8H-azepino[4',5':4,5]pyrrolo[3,2,1-ij]quinolin-4-one;
10-benzoyl-5,6,8,9,10,11,12,12a-octahydro-4H,7aH-azepino[4',5':4,5]pyrrolo[3,2,1-ij]quinolin-4-ol; and
10-benzoyl-4-methoxy-5,6,8,9,10,11,12,12a-octahydro-4H,7aH-azepino[4',5':4,5]pyrrolo[3,2,1-ij]quinoline.

All cited publications, patents, and patent documents are incorporated by reference herein, as though individually incorporated by reference. The invention has been described with reference to various specific and preferred embodiments and techniques. However, it should be understood that many variations and modifications may be made while remaining within the spirit and scope of the invention.

## Claims

1. A compound of formula I: 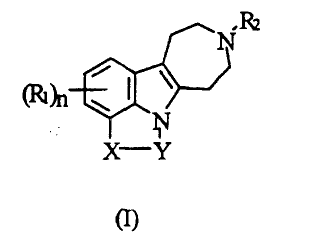 wherein,
each R₁ is independently hydroxy, nitro, halo, cyano, trifluoromethyl, trifluoromethoxy, C₁₋₇alkyl, C₁₋₇alkoxy, C₁₋₇alkanoyl, C₁₋ ₇alkoxycarbonyl, C₁₋₇alkanoyloxy, aryl, heteroaryl, -S(O)ₘNRₐR_{b}, NR_{c}R_{d}, -S(O)ₘRₑ, or -C(=O)NRₐR_{b}, wherein any C₁₋₇alkyl, C₁₋₇alkoxy, C₁₋₇alkanoyl, C₁₋₇alkoxycarbonyl, or C₁₋₇alkanoyloxy of R₁ is optionally partially unsaturated and is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋ ₇alkanoyloxy, -S(O)ₘRₑ, -S(O)ₘNRₐR_{b}, NR_{c}R_{d}, or -C(=O)NRₐR_{b};
R₂ is hydrogen, C₁₋₇alkyl, C₁₋₇alkanoyl, arylcarbonyl, aryl, (aryl)C₁₋₇alkyl, C₁₋₇alkoxycarbonyl, aryloxycarbonyl, arylsulfonyl, or (aryl)C₁₋ ₇alkoxycarbonyl;
X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo (=O), thioxo (=S), -NR_{q}Rᵣ, -S(O)ₚRₛ, or -ORₜ, or with one or two substituents independently selected from the group consisting of C₁₋₇alkyl, (C₁₋₇alkoxy)C₁₋ ₇alkyl, aryl, (aryl)C₁₋₇alkyl, heteroaryl, (heteroaryl)C₁₋₇alkyl, and (aryl)oxyC₁₋₇alkyl; or wherein the chain is optionally substituted on a carbon with a 4, 5, or 6 membered spirocyclic carbon ring; or wherein the chain is optionally substituted on two adjacent atoms with a 2, 3, or 4 membered alkylene chain (e.g. -CH₂CH₂-, -CH₂CH₂CH₂-, or -CH₂CH₂CH₂CH₂-) forming a ring that is fused to the ring comprising X and Y;
each m is independently 0, 1, or 2;
n is 0, 1, 2, or 3;
p is 0, 1, or 2;
each Rₐ and R_{b} is independently hydrogen, C₁₋₇alkyl, aryl, (aryl)C₁₋₇alkyl, heteroaryl, or (heteroaryl)C₁₋₇alkyl; or Rₐ and R_{b} together with the nitrogen to which they are attached form a pyrrolidino, piperidino, morpholino, or thiomorpholino ring;
each R_{c} and R_{d} is independently hydrogen, C₁₋₇alkyl, C₁₋₇alkanoyl, C₁₋₇alkoxycarbonyl, aryl, (aryl)C₁₋₇alkyl, heteroaryl, (heteroaryl)C₁₋₇alkyl, arylcarbonyl, heteroarylcarbonyl, aryloxycarbonyl, or heteroaryloxycarbonyl; or R_{c} and R_{d} together with the nitrogen to which they are attached form a pyrrolidino, piperidino, morpholino, or thiomorpholino ring;
each Rₑ is independently hydrogen, C₁₋₇alkyl, aryl, (aryl)C₁₋₇alkyl, heteroaryl, or (heteroaryl)C₁₋₇alkyl;
R_{f} is hydrogen, C₁₋₇alkyl, aryl, (aryl)C₁₋₇alkyl, heteroaryl, (heteroaryl)C₁₋₇alkyl, or is a bond to a 2, 3, or 4 membered alkylene chain that forms a ring that is fused to the ring comprising X and Y;
each R_{q} and Rᵣ is independently hydrogen, C₁₋₇alkyl, aryl, (aryl)C₁₋₇alkyl, heteroaryl, or (heteroaryl)C₁₋₇alkyl; or R_{q} and Rᵣ together with the nitrogen to which they are attached form a pyrrolidino, piperidino, morpholino, or thiomorpholino ring;
Rₛ is C₁₋₇alkyl, aryl, (aryl)C₁₋₇alkyl, heteroaryl, or (heteroaryl)C₁₋ ₇alkyl; and
Rₜ is hydrogen, C₁₋₇alkyl, aryl, (aryl)C₁₋₇alkyl, heteroaryl, or (heteroaryl)C₁₋₇alkyl;
wherein any aryl or heteroaryl ring of R₁, R₂, X, Y, Rₐ-R_{f}, or R_{q}-Rₜ is optionally substituted with one or more (e.g. 1, 2, 3, or 4) substituents independently selected from halo, hydroxy, cyano, nitro, trifluoromethyl, trifluoromethoxy, C₁₋₇alkyl, C₁₋₇alkoxy, phenyl, sulfonyl, NRⱼRₖ, or-C(=O)NRⱼR_{k,}; wherein each Rⱼ and Rₖ is independently hydrogen, C₁₋₇alkyl, C₁₋₇alkanoyl, C₁₋₇alkoxycarbonyl, aryl, (aryl)C₁₋₇alkyl, arylcarbonyl, or aryloxycarbonyl; or Rⱼ and Rₖ together with the nitrogen to which they are attached form a pyrrolidino, piperidino, morpholino, or thiomorpholino ring;
or a pharmaceutically acceptable salt thereof;
provided Y is not oxy, thio, sulfinyl, or NR_{f}; and
provided X and Y together are not a 2-membered unsaturated chain; and
provided no carbon of X and Y is bonded to more than one oxy, thio, sulfinyl, or NR_{f}.

2. The compound of claim 1 wherein each R₁ is independently hydroxy, nitro, halo, cyano, trifluoromethyl, trifluoromethoxy, C₁₋₇alkyl, C₁₋₇alkoxy, C₁₋₇alkanoyl, C₁₋₇alkoxycarbonyl, C₁₋₇alkanoyloxy, aryl, heteroaryl,-S(O)ₘNRₐR_{b}, NR_{c}R_{d}, -S(O)ₘRₑ, or -C(=O)NRₐR_{b}, wherein any C₁₋₇alkyl, C₁₋₇alkoxy, C₁₋₇alkanoyl, C₁₋₇alkoxycarbonyl, or C₁₋₇alkanoyloxy of R₁ is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋₇alkanoyloxy, -S(O)ₘRₑ, -S(O)ₘNRₐR_{b}, NR_{c}R_{d}, or -C(=O)NRₐR_{b}.

3. The compound of claim 1 wherein each R₁ is independently hydroxy, nitro, halo, cyano, trifluoromethyl, trifluoromethoxy, C₁₋₇alkyl, C₁₋₇alkoxy, C₁₋₇alkanoyl, C₁₋₇alkoxycarbonyl, C₁₋₇alkanoyloxy, aryl, heteroaryl,-S(O)ₘNRₐR_{b}, NR_{c}R_{d}, -S(O)ₘRₑ, or -C(=O)NRₐR_{b}, wherein any C₁₋₇alkyl or C₁₋₇alkoxy of R₁ is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋₇alkanoyloxy, -S(O)ₘRₑ, -S(O)ₘNRₐR_{b}, NR_{c}R_{d}, or-C(=O)NRₐR_{b}.

4. The compound of claim 1 wherein each R₁ is independently hydroxy, nitro, halo, cyano, trifluoromethyl, trifluoromethoxy, C₁₋₇alkyl, C₁₋₇alkoxy, C₁₋₇alkanoyl, C₁₋₇alkoxycarbonyl, C₁₋₇alkanoyloxy, aryl, heteroaryl,-S(O)ₘNRₐR_{b}, NR_{c}R_{d}, -S(O)ₘRₑ, or -C(=O)NRₐR_{b}, wherein any C₁₋₇alkyl is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋₇alkanoyloxy, -S(O)ₘRₑ, -S(O)ₘNRₐR_{b}, NR_{c}R_{d}, or -C(=O)NRₐR_{b}.

5. The compound of claim 1 wherein each R₁ is independently hydroxy, nitro, halo, cyano, trifluoromethyl, trifluoromethoxy, C₁₋₇alkyl, C₁₋₇alkoxy, C₁₋₇alkanoyl, C₁₋₇alkoxycarbonyl, C₁₋₇alkanoyloxy, aryl, heteroaryl,-S(O)ₘNRₐR_{b}, NR_{c}R_{d}, -S(O)ₘRₑ, or -C(=O)NRₐR_{b}.

6. The compound of claim 1 wherein each R₁ is independently C₁₋₇alkyl, C₁₋₇alkoxy, trifluoromethyl, or halo.

7. The compound of claim 1 wherein n is 1 and R₁ is C₁₋₇alkyl, C₁₋₇alkoxy, or halo.

8. The compound of claim 1 wherein n is 1 and R₁ is methyl, methoxy, chloro, or fluoro.

9. The compound of claim 1 wherein R₂ is hydrogen.

10. The compound of claim 1 wherein n is 1, 2, or 3.

11. The compound of claim 1 wherein n is 0.

12. The compound of claim 1 wherein X and Y together are a 2, 3, or 4 membered saturated or partially unsaturated chain comprising one or more carbon atoms and optionally comprising one oxy (-O-), thio (-S-), sulfinyl (-SO-), sulfonyl (-S(O)₂-), or NR_{f} in the chain; wherein the chain is optionally substituted on each carbon with oxo (=O), thioxo (=S), -NR_{q}Rᵣ, -S(O)ₚRₛ, or -ORₜ, or with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl.

13. The compound of claim 1 wherein X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo (=O), thioxo (=S), -NR_{q}Rᵣ, -S(O)ₚRₛ, or - ORₜ.

14. The compound of claim 1 wherein X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl.

15. The compound of claim 1 wherein X and Y together are a 2, 3, or 4 membered chain comprising one or more carbon atoms and optionally comprising one oxy, thio, sulfinyl, sulfonyl, or NR_{f} in the chain; wherein the chain is optionally substituted on each carbon with oxo (=O), hydroxy, (aryl)oxy, heteroaryl(oxy) or C₁₋₇alkoxy, or with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl; and wherein the chain is optionally substituted on two adjacent atoms with a 2, 3, or 4 membered alkylene chain forming a ring that is fused to the ring comprising X and Y.

16. The compound of claim 1 wherein X and Y together are a 2, 3, or 4 membered carbon chain wherein the chain is optionally substituted on each carbon with oxo, hydroxy, (aryl)oxy, heteroaryl(oxy) or C₁₋₇alkoxy, or with one or two substituents independently selected from the group consisting of C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl.

17. The compound of claim 1 wherein X and Y together are a 2 or 3 membered carbon chain optionally substituted on each carbon with oxo or hydroxy, or with one or two C₁₋₇alkyl.

18. The compound of claim 1 wherein X is -O-, -S-, or -C(R_{g})(Rₕ)-, wherein R_{g} and Rₕ are each independently hydrogen, C₁₋₇alkyl, (C₁₋₇alkoxy)C₁₋ ₇alkyl, aryl, (aryl)C₁₋₇alkyl, heteroaryl, (heteroaryl)C₁₋₇alkyl or (aryl)oxyC₁₋₇alkyl, or R_{g} and Rₕ together are oxo.

19. The compound of claim 1 wherein Y is -C(R_{g})(Rₕ)-, - C(R_{g})(Rₕ)C(R_{g})(Rₕ)-, -C(R_{g})(Rₕ)C(R_{g})(Rₕ)C(R_{g})(Rₕ)-, -C(R_{g})(Rₕ)C(=O)-, - C(R_{g})(Rₕ)C(R_{g})(Rₕ)C(=O)-, -C(=O)C(R_{g})(Rₕ)-, or -C(=O)C(R_{g})(Rₕ)C(R_{g})(Rₕ)-, and each R_{g} and Rₕ is independently hydrogen or C₁₋₇alkyl.

20. The compound of claim 1 wherein X is -O-, -S-, or -C(R_{g})(Rₕ)-; and Y is -C(R_{g})(Rₕ)C(=O)-, or -C(R_{g})(Rₕ)C(R_{g})(Rₕ)-, wherein each R_{g} and Rₕ is independently hydrogen or C₁₋₇alkyl.

21. The compound of claim 1 wherein X is -O- or -S-; and Y is -C(R_{g})(Rₕ)C(=O)-, -C(=O)C(R_{g})(Rₕ)-, or -C(R_{g})(Rₕ)C(R_{g})(Rₕ)-, wherein each R_{g} and Rₕ is independently hydrogen or C₁₋₇alkyl.

22. The compound of claim 1 wherein X and Y together are--CH(R_{g})CH₂-, -CH(R_{g})CH(R_{g})CH₂-, -CH(R_{g})CH(R_{g})CH(R_{g})CH₂-, -C(R_{g})=C(R_{g})CH₂-, -CH=CHCH(R_{g})CH₂-, -CH(R_{g})CH=CHCH₂-, -O-CH₂CH₂-, -O-CH₂CH(R_{g})CH₂-, -S-CH₂CH₂-, -S-CH₂CH(R_{g})CH₂-, -S(O)-CH₂CH₂-, -S(O)-CH₂CH(R_{g})CH₂-, -S(O)₂-CH₂CH₂-, -S(O)₂-CH₂CH(R_{g})CH₂-, -NR_{f}-CH₂CH₂-, -NR_{f}-CH₂CH(R_{g})CH₂-, -CH₂C(=O)-, -CH(R_{g})CH₂C(=O)-, -CH(R_{g})CH(R_{g})CH₂C(=O)-, -CH₂OC(=O)-,-CH(R_{g})CH₂OC(=O)-, -OCH₂C(=O)-, or -OCH₂CH₂C(=O)-; wherein each R_{g} is independently -NR_{q}Rᵣ, -S(O)ₚRₛ, or -ORₜ.

23. The compound of claim 1 wherein X and Y together are--CH(R_{g})CH(R_{g})-, -CH(R_{g})CH(R_{g})CH(R_{g})-, -CH(R_{g})CH(R_{g})CH(R_{g})CH(R_{g})-, -CH=CHCH(R_{g})-, -C(R_{g})=C(R_{g})CH(R_{g})CH(R_{g})-, -CH(R_{g})C(R_{g})=C(R_{g})CH(R_{g})-,--O-CH(R_{g})CH(R_{g})-, -O-CH(R_{g})CH(R_{g})CH(R_{g})-, -S-CH(R_{g})CH(R_{g})-, -S-CH(R_{g})CH(R_{g})CH(R_{g})-, -S(O)-CH(R_{g})CH(R_{g})-, -S(O)-CH(R_{g})CH(R_{g})CH(R_{g})-, -S(O)₂-CH(R_{g})CH(R_{g})-, -S(O)₂-CH(R_{g})CH(R_{g})CH(R_{g})-, -NR_{f}-CH(R_{g})CH(R_{g})-, -NR_{f}-CH(R_{g})CH(R_{g})CH(R_{g})-, -CH(R_{g})C(=O)-, -CH(R_{g})CH(R_{g})C(=O)-, -CH(R_{g})CH(R_{g})CH(R_{g})C(=O)-, -CH(R_{g})OC(=O)-, -CH(R_{g})CH(R_{g})OC(=O)-, -OCH(R_{g})C(=O)-, or -OCH(R_{g})CH(R_{g})C(=O)-; wherein each R_{g} is independently hydrogen, C₁₋₇alkyl, aryl, (aryl)C₁₋₇alkyl, (aryl)oxy, heteroaryl(oxy), or (aryl)oxyC₁₋₇alkyl.

24. The compound of claim 1 wherein X and Y together are--CH(R_{g})CH(R_{g})-, -CH(R_{g})CH(R_{g})CH(R_{g})-, -C(R_{g})=C(R_{g})CH(R_{g})-, - O-CH(R_{g})CH(R_{g})-, -S-CH(R_{g})CH(R_{g})-, -S(O)-CH(R_{g})CH(R_{g})-, - S(O)₂-CH(R_{g})CH(R_{g})-, -NR_{f}-CH(R_{g})CH(R_{g})-, -CH(R_{g})C(=O)-, -CH(R_{g})CH(R_{g})C(=O)-, -CH(R_{g})OC(=O)-, -OCH(R_{g})C(=O)-; wherein each R_{g} is independently hydrogen or C₁₋₇alkyl.

25. The compound of claim 1 wherein X and Y together are--CH(R_{g})CH₂-, -CH(R_{g})CH(R_{g})CH₂-, or -O-CH₂CH₂-, wherein each R_{g} is independently -NR_{q}Rᵣ, -S(O)ₚRₛ, -ORₜ, C₁₋₇alkyl, (C₁₋₇alkoxy)C₁₋₇alkyl, aryl, (aryl)C₁₋₇alkyl, heteroaryl, (heteroaryl)C₁₋₇alkyl, and (aryl)oxyC₁₋₇alkyl.

26. The compound of claim 1 wherein X and Y together are-- C(=O)CH₂-, -CH₂C(=O)-, -C(=S)CH₂-, -CH₂C(=S)-, -C(=O)CH₂CH₂-, -CH₂C(=O)CH₂-, -CH₂CH₂C(=O)-, -C(=S)CH₂CH₂-, -CH₂C(=S)CH₂-, or - CH₂CH₂C(=S)-.

27. The compound of claim 1 wherein X and Y together are--CH(R_{g})CH(R_{g})-, -CH(R_{g})CH(R_{g})CH(R_{g})-, -C(R_{g})=C(R_{g})CH(R_{g})-, - O-CH(R_{g})CH(R_{g})-, -S-CH(R_{g})CH(R_{g})-, -S(O)-CH(R_{g})CH(R_{g})-, - S(O)₂-CH(R_{g})CH(R_{g})-, -NR_{f}-CH(R_{g})CH(R_{g})-, or -CH(R_{g})CH(R_{g})C(=O)-; wherein each R_{g} is independently hydrogen, C₁₋₇alkyl, or together with an R_{g} on an adjacent carbon atom forms a fused 4, 5, or 6, membered carbocyclic ring.

28. The compound of claim 1 wherein X and Y together are--CH(R_{g})CH(R_{g})-, -CH(R_{g})CH(R_{g})CH(R_{g})-, -O-CH(R_{g})CH(R_{g})-, -S-CH(R_{g})CH(R_{g})-; wherein each R_{g} is independently hydrogen, C₁₋₇alkyl, aryl, or (aryl)C₁₋₇alkyl.

29. The compound of claim 1 wherein X and Y together are-- CH₂CH₂CH₂-, -CH₂CH₂C(CH₃)H-, -CH₂C(CH₃)HCH₂-, -C(CH₃)HCH₂CH₂-,-- CH₂CH₂-, -CH₂C(CH₃)H-, -C(CH₃)HC(CH₃)H-, -CH(R_{g})CH(R_{g})-, -O-CH₂CH₂-, - O-C(CH₃)HCH₂-,
or -S-CH₂CH₂-.

30. The compound of claim 1 wherein X and Y together are--CH₂CH₂-, -CH₂CH₂CH₂-, -CH=CHCH₂-, -O-CH₂CH₂-, -S-CH₂CH₂-, -S(O)-CH₂CH₂-, -S(O)₂-CH₂CH₂-, -NR_{f}-CH₂CH₂-, -CH₂C(=O)-, -CH₂CH₂C(=O)-, -CH₂OC(=O)-, -OCH₂C(=O)-.

31. The compound of any one of claims 1 to 30 wherein R₂ is hydrogen

32. The compound of claim 1 which is:
5,6,9,10,11,12-hexahydro-4H,8H-azepino[4',5':4,5]pyrrolo[3,2,1-ij]quinoline;
2-methyl-5,6,9,10,11,12-hexahydro-4H,8H-azepino[4',5':4,5]pyrrolo[3,2,1-ij]quinoline;
1-methoxy-5,6,9,10,11,12-hexahydro-4H,8H-azepino[4',5':4,5]pyrrolo[3,2,1-ij]quinoline;
2-fluoro-5,6,9,10,11,12-hexahydro-4H,8H-azepino[4',5':4,5]pyrrolo[3,2,1-ij]quinoline;
6-methyl-5,6,9,10,11,12-hexahydro-4H,8H-azepino[4',5':4,5]pyrrolo[3,2,1-ij]quinoline;
5-methyl-5,6,9,10,11,12-hexahydro-4H,8H-azepino[4',5':4,5]pyrrolo[3,2,1-ij]quinoline;
4-methyl-5,6,9,10,11,12-hexahydro-4H,8H-azepino[4',5':4,5]pyrrolo[3,2,1-ij]quinoline;
(+)-6-methyl-5,6,9,10,11,12-hexahydro-4H,8H-azepino[4',5':4,5]pyrrolo[3,2,1-ij]quinoline;
(-)-6-methyl-5,6,9,10,11,12-hexahydro-4H,8H-azepino[4',5':4,5]pyrrolo[3,2,1-ij]quinoline;
(+)-5-methyl-5,6,9,10,11,12-hexahydro-4H,8H-azepino[4',5':4,5]pyrrolo[3,2,1-ij]quinoline;
(-)-5-methyl-5,6,9,10,11,12-hexahydro-4H,8H-azepino[4',5':4,5]pyrrolo[3,2,1-ij]quinoline;
(+)-4-methyl-5,6,9,10,11,12-hexahydro-4H,8H-azepino[4',5':4,5]pyrrolo[3,2,1-ij]quinoline;
(-)-4-methyl-5,6,9,10,11,12-hexahydro-4H,8H-azepino[4',5':4,5]pyrrolo[3,2,1-ij]quinoline;
4,5,8,9,10,11-hexahydro-7H-azepino[4,5-b]pyrrolo[3,2,1-hi]indole;
2-fluoro-4,5,8,9,10,11-hexahydro-7H-azepino[4,5-b]pyrrolo[3,2,1-hi]indole;
2-methoxy-4,5,8,9,10,11-hexahydro-7H-azepino[4,5-b]pyrrolo[3,2,1-hi]indole;
5-methyl-4,5,8,9,10,11-hexahydro-7H-azepino[4,5-b]pyrrolo[3,2,1-hi]indole;
4,5-dimethyl-4,5,8,9,10,11-hexahydro-7H-azepino[4,5-b]pyrrolo[3,2,1-hi]indole;
2,3,4,5,8b,9,10,11,12,12a-decahydro-1H-azepino[4',5':4,5]pyrrolo[3,2,1-jk]carbazole;
6-methyl-1,2,8,9,10,11-hexahydro-7H-azepino[4,5-b][1,4]oxazino[2,3,4-hi]indole;
1,2,8,9,10,11-hexahydro-7H-azepino[4,5-b][1,4]oxazino[2,3,4-hi]indole;
6-chloro-1,2,8,9,10,11-hexahydro-7H-azepino[4,5-b][1,4]oxazino[2,3,4-hi]indole;
5-fluoro-1,2,8,9,10,11-hexahydro-7H-azepino[4,5-b][1,4]oxazino[2,3,4-hi]indole;
5-methyl-1,2,8,9,10,11-hexahydro-7H-azepino[4,5-b][1,4]oxazino[2,3,4-hi]indole;
6-fluoro-1,2,8,9,10,11-hexahydro-7H-azepino[4,5-b][1,4]oxazino[2,3,4-hi]indole;
2-methyl-1,2,8,9,10,11-hexahydro-7H-azepino[4,5-b][1,4]oxazino[2,3,4-hi]indole;
1,2,8,9,10,11-hexahydro-7H-azepino[4,5-b][1,4]thiazino[2,3,4-hi]indole;
2,2-dimethyl-1,2,8,9,10,11-hexahydro-7H-azepino[4,5-b][1,4]oxazino[2,3,4-hi]indole;
4-fluoro-1,2,8,9,10,11-hexahydro-7H-azepino[4,5-b][1,4]oxazino[2,3,4-hi]indole;
4-chloro-1,2,8,9,10,11-hexahydro-7H-azepino[4,5-b][1,4]oxazino[2,3,4-hi]indole;
5-chloro-1,2,8,9,10,11-hexahydro-7H-azepino[4,5-b][1,4]oxazino[2,3,4-hi]indole;
6-(trifluoromethyl)-1,2,8,9,10,11-hexahydro-7H-azepino[4,5-b][1,4]oxazino[2,3,4-hi]indole;
5,6-difluoro-1,2,8,9,10,11-hexahydro-7H-azepino[4,5-b][1,4]oxazino[2,3,4-hi]indole;
5-chloro-6-fluoro-1,2,8,9,10,11-hexahydro-7H-azepino[4,5-b][1,4]oxazino[2,3,4-hi]indole;
6-chloro-5-fluoro-1,2,8,9,10,11-hexahydro-7H-azepino[4,5-b][1,4]oxazino[2,3,4-hi]indole;
5,6-dichloro-1,2,8,9,10,11-hexahydro-7H-azepino[4,5-b][1,4]oxazino[2,3,4-hi]indole;
4,6-dichloro-1,2,8,9,10,11-hexahydro-7H-azepino[4,5-b][1,4]oxazino[2,3,4-hi]indole;
1-chloro-2-fluoro-4,5,8,9,10,11-hexahydro-7H-azepino[4,5-b]pyrrolo[3,2,1-hi]indole;
1-chloro-4,5,8,9,10,11-hexahydro-7H-azepino[4,5-b]pyrrolo[3,2,1-hi]indole;
2-chloro-4,5,8,9,10,11-hexahydro-7H-azepino[4,5-b]pyrrolo[3,2,1-hi]indole;
5,6,9,10,11,12-hexahydro-4H,8H-azepino[4',5':4,5]pyrrolo[3,2,1-ij]quinolin-4-one;
2-fluoro-5,6,9,10,11,12-hexahydro-4H,8H-azepino[4',5':4,5]pyrrolo[3,2,1-ij]quinolin-4-one;
2-chloro-5,6,9,10,11,12-hexahydro-4H,8H-azepino[4',5':4,5]pyrrolo[3,2,1-ij]quinolin-4-one;
6-methyl-5,6,9,10,11,12-hexahydro-4H,8H-azepino[4',5':4,5]pyrrolo[3,2,1-ij]quinolin-4-one;
2-fluoro-6-methyl-5,6,9,10,11,12-hexahydro-4H,8H-azepino[4',5':4,5]pyrrolo[3,2,1-ij]quinolin-4-one;
2,3-dichloro-6-methyl-5,6,9,10,11,12-hexahydro-4H,8H-azepino[4',5':4,5]pyrcolo[3,2,1-ij]quinolin-4-one;
6-propyl-5,6,9,10,11,12-hexahydro-4H,8H-azepino[4',5':4,5]pyrrolo[3,2,1-ij]quinolin-4-one;
6-(trifluoromethyl)-5,6,9,10,11,12-hexahydro-4H,8H-azepino[4',5':4,5]pyrrolo[3,2,1-ij]quinolin-4-one;
5,6,8,9,10,11,12,12a-octahydro-4H,7aH-azepino[4',5':4,5]pyrrolo[3,2,1-ij]quinolin-4-ol;
4-methoxy-5,6,8,9,10,11,12,12a-octahydro-4H,7aH-azepino[4',5':4,5]pyrrolo[3,2,1-ij]quinoline;
10-benzoyl-4-phenoxy-5,6,9,10,11,12-hexahydro-4H,8H-azepino[4',5':4,5]pyrrolo[3,2,1-ij]quinoline; or
4-phenoxy-5,6,9,10,11,12-hexahydro-4H,8H-azepino[4',5':4,5]pyrrolo[3,2,1-ij]quinoline;
or a pharmaceutically acceptable salt thereof.

33. The compound 1,2,8,9,10,11-hexahydro-7H-azepino[4,5-b][1,4]oxazino[2,3,4-hi]indole; or a pharmaceutically acceptable salt thereof.

34. The compound of claim 1 which is:
4-bromo-1,2,8,9,10,11-hexahydro-7H-azepino[4,5-b][1,4] oxazino[2,3,4-hi]indole;
5-bromo-1,2,8,9,10,11-hexahydro-7H-azepino[4,3-b][1,4] oxazino[2,3,4-hi]indole;
6-bromo-1,2,8,9,10,11-hexahydro-7H-azepmo[4,5-b][1,4] oxazino[2,3,4-hi]indole;
5,6-dibromo-1,2,8,9,10,11-hexahydro-7H-azepino[4,5-b][1,4] oxazino[2,3,4-hi]indole;
4,6-dibromo-1,2,8,9,10,11-hexahydro-7H-azepino[4,5-b][1,4] oxazino[2,3,4-hi]indole;
4-methoxy-1,2,8,9,10,11-hexahydro-7H-azepino[4,5-b][1,4] oxazino[2,3,4-hi]indole;
5-methoxy-1,2,8,9,10,11-hexahydro-7H-azepino[4,5-b][1,4] oxazino[2,3,4-hi]indole;
6-methoxy-1,2,8,9,10,11-hexahydro-7H-azepino[4,5-b][1,4] oxazino[2,3,4-hi]indole;
4-(triflouromethyl)-1,2,8,9,10,11-hexahydro-7H-azepino[4,5-b][1,4] oxazino[2,3,4-hi]indole;
5-(triflouromethyl)-1,2,8,9,10,11-hexahydro-7H-azepino[4,5-b][1,4] oxazino[2,3,4-hi]indole;
4-benzyloxy-1,2,8,9,10,11-hexahydro-7H-azepino[4,5-b][1,4] oxazino[2,3,4-hi]indole;
5-benzyloxy-1,2,8,9,10,11-hexahydro-7H-azepino[4,5-b][1,4] oxazino[2,3,4-hi]indole;
6-benzyloxy-1,2,8,9,10,11-hexahydro-7H-azepmo[4,5-b][1,4] oxazino[2,3,4-hi]indole;
4-fluoro-1,2,8,9,10,11-hexahydro-7H-azepino[4,5-b][1,4] thiazino[2,3,4-hi]indole;
5-fluoro-1,2,8,9,10,11-hexahydro-7H-azepino[4,5-b][1,4] thiazino[2,3,4-hi]indole;
6-fluoro-1,2,8,9,10,11-hexahydro-7H-azepino[4,5-b][1,4] thiazino[2,3,4-hi]indole;
4-chloro-1,2,8,9,10,11-hexahydro-7H-azepino[4,5-b][1,4] thiazino[2,3,4-hi]indole;
5-chloro-1,2,8,9,10,11-hexahydro-7H-azepino[4,5-b][1,4] thiazino[2,3,4-hi]indole;
6-chloro-1,2,8,9,10,11-hexahydro-7H-azepino[4,5-b][1,4] thiazino[2,3,4-hi]indole;
4,5-difluoro-1,2,8,9,10,11-hexahydro-7H-azepino[4,5-b][1,4] thiazino[2,3,4-hi]indole;
5,6-difluoro-1,2,8,9,10,11-hexahydro-7H-azepino[4,5-b][1,4] thiazino[2,3,4-hi]indole;
4,6-difluoro-1,2,8,9,10,11-hexahydro-7H-azepino[4,5-b][1,4] thiazino[2,3,4-hi]indole;
4-chloro-5-fluoro-1,2,8,9,10,11-hexahydro-7H-azepino[4,5-b][1,4] thiazino[2,3,4-hi]indole;
4-chloro-6-fluoro-1,2,8,9,10,11-hexahydro-7H-azepino[4,5-b][1,4] thiazino[2,3,4-hi]indole;
5-chloro-6-fluoro-1,2,8,9,10,11-hexahydro-7H-azepino[4,5-b][1,4] thiazino[2,3,4-hi]indole;
6-chloro-5-fluoro-1,2,8,9,10,11-hexahydro-7H-azepino[4,5-b][1,4] thiazino[2,3,4-hi]indole;
4-methyl-1,2,8,9,10,11-hexahydro-7H-azepino[4,5-b][1,4] thiazino[2,3,4-hi]indole;
5-methyl-1,2,8,9,10,11-hexahydro-7H-azepino[4,5-b][1,4] thiazino[2,3,4-hi]indole;
6-methyl-1,2,8,9,10,11-hexahydro-7H-azepino[4,5-b][1,4] thiazino[2,3,4-hi]indole;
4-methoxy-1,2,8,9,10,11-hexahydro-7H-azepino[4,5-b][1,4] thiazino[2,3,4-hi]indole;
5-methoxy-1,2,8,9,10,11-hexahydro-7H-azepino[4,5-b][1,4] thiazino[2,3,4-hi]indole;
6-methoxy-1,2,8,9,10,11-hexahydro-7H-azepino[4,5-b][1,4] thiazino[2,3,4-hi]indole;
4-(triflouromethyl)-1,2,8,9,10,11-hexahydro-7H-azepino[4,5-b][ 1,4] thiazino[2,3,4-hi]indole;
5-(triflouromethyl)-1,2,8,9,10,11-hexahydro-7H-azepino[4,5-b][1,4] thiazino[2,3,4-hi]indole;
6-(triflouromethyl)-1,2,8,9,10,11-hexahydro-7H-azepino[4,5-b][1,4] thiazino[2,3,4-hi]indole;
4-benzyloxy-1,2,8,9,10,11-hexahydro-7H-azepino[4,5-b][1,4] thiazino[2,3,4-hi]indole;
5-benzyloxy-1,2,8,9,10,11-hexahydro-7H-azepmo[4,5-b][1,4] thiazino[2,3,4-hi]indole;
6-benzyloxy-1,2,8,9,10,11-hexahydro-7H-azepino[4,5-b][1,4] thiazino[2,3,4-hi]indole;
1-fluoro-4,5,8,9,10,11-hexahydro-7H-azepino[4,5-b]pyrrolo[3,2,1-hi]indole;
3-fluoro-4,5,8,9,10,11-hexahydro-7H-azepino[4,5-b]pyrrolo[3,2,1-hi]indole;
1-bromo-4,5,8,9,10,11-hexahydro-7H-azepino[4,5-b]pyrrolo[3,2,1-hi]indole;
2-bromo-4,5,8,9,10,11-hexahydro-7H-azepino[4,5-b]pyrrolo[3,2,1-hi]indole;
3-bromo-4,5,8,9,10,11-hexahydro-7H-azepino[4,5-b]pyrrolo[3,2,1-hi]indole;
3-chloro-1-4,5,8,9,10,11-hexahydro-7H-azepino[4,5-b]pyrrolo[3,2,1-hi]indole;
2-chloro-1-fluoro-4,5,8,9,10,11-hexahydro-7H-azepino[4,5-b]pyrrolo[3,2,1-hi]indole;
1-methoxy-4,5,8,9,10,11-hexahydro-7H-azepino[4,5-b]pyrrolo[3,2,1-hi]indole;
3-methoxy-4,5,8,9,10,11-hexahydro-7H-azepino[4,5-b]pyrrolo[3,2,1-hi]indole;
4-methyl-4,5,8,9,10,11-hexahydro-7H-azepino[4,5-b]pyrrolo[3,2,1-hi]indole;
1-(trifluoromethyl)-4,5,8,9,10,11-hexahydro-7H-azepino[4,5-b]pyrrolo[3,2,1-hi]indole;
2-(trifluoromethyl)-4,5,8,9,10,11-hexahydro-7H-azepino[4,5-b]pyrrolo[3,2,1-hi]indole;
3-(trifluoromethyl)-4,5,8,9,10,11-hexahydro-7H-azepino[4,5-b]pyrrolo[3,2,1-hi]indole;
4-benzyloxy-5,6,8,9,10,11,12,12a-octahydro-4H,7aH-azepino[4',5':4,5]pyrrolo[3,2,1-ij]quinoline;
4-(3-chlorophenoxy)-5,6,9,10,11,12-hexahydro-4H,8H-azepino[4',5':4,5]pyrrolo[3,2,1-ij]quinoline;
4-(2-chlorophenoxy)-5,6,9,10,11,12-hexahydro-4H,8H-azepino[4',5':4,5]pyrrolo[3,2,1-ij]quinoline;
4-(4-methoxyphenoxy)-5,6,9,10,11,12-hexahydro-4H,8H-azepino[4',5':4,5]pyrrolo[3,2,1-ij]quinoline;
4-(3-methoxyphenoxy)-5,6,9,10,11,12-hexahydro-4H,8H-azepino[4',5':4,5]pyrrolo[3,2,1-ij]quinoline;
4-(2-methoxyphenoxy)-5,6,9,10,11,12-hexahydro-4H,8H-azepino[4',5':4,5]pyrrolo[3,2,1-ij]quinoline;
4-(4-bromo-2-methoxyphenoxy)-5,6,9,10,11,12-hexahydro-4H,8H-azepino[4',5':4,5]pyrrolo[3,2,1-ij]quinoline;
5,6,9,10,11,12-hexahydro-4H,8H-azepino[4',5':4,5]pyrrolo[3,2,1-ij]quinolin-4-amine;
N-phenyl-5,6,9,10,11,12-hexahydro-4H,8H-azepino[4',5':4,5]pyrrolo[3,2,1-ij]quinolin-4-amine;
N-(4-chlorophenyl)-5,6,9,10,11,12-hexahydro-4H,8H-azepino[4',5':4,5]pyrrolo[3,2,1-ij]quinolin-4-amine;
N-(3-chlorophenyl)-5,6,9,10,11,12-hexahydro-4H,8H-azepino[4',5':4,5]pyrrolo[3,2,1-ij]quinolin-4-amine;
N-(2-chlorophenyl)-5,6,9,10,11,12-hexahydro-4H,8H-azepino[4',5':4,5]pyrrolo[3,2,1-ij]quinolin-4-amine;
N-(4-methoxyphenyl)-5,6,9,10,11,12-hexahydro-4H,8H-azepino[4',5':4,5]pyrrolo[3,2,1-ij]quinolin-4-amine;
N-(3-methoxyphenyl)-5,6,9,10,11,12-hexahydro-4H,8H-azepino[4',5':4,5]pyrrolo[3,2,1-ij]quinolin-4-amine;
N-(2-methoxyphenyl)-5,6,9,10,11,12-hexahydro-4H,8H-azepino[4',5':4,5]pyrrolo[3,2,1-ij]quinolin-4-amine;
N-(4-bromo-2-methoxyphenyl)-5,6,9,10,11,12-hexahydro-4H,8H-azepino[4',5':4,5]pyrrolo[3,2,1-ij]quinolin-4-amine;
5,6,9,10,11,12-hexahydro-4H,8H-azepino[4',5':4,5]pyrrolo[3,2,1-ij]quinoline-4-thione;
4-(phenylsulfonyl)-5,6,9,10,11,12-hexahydro-4H,8H-azepino[4',5':4,5]pyrrolo[3,2,1-ij]quinoline;
4-(4-chlorophenylsulfonyl)-5,6,9,10,11,12-hexahydro-4H,8H-azepino[4',5':4,5]pyrrolo[3,2,1-ij]quinoline;
4-(3-chlorophenylsulfonyl)-5,6,9,10,11,12-hexahydro-4H,8H-azepino[4',5':4,5]pyrrolo[3,2,1-ij]quiaoline;
4-(2-chlorophenylsulfonyl)-5,6,9,10,11,12-hexahydro-4H,8H-azepino[4',5':4,5]pyrrolo[3,2,1-ij]quinoline;
4-(4-methoxyphenylsulfonyl)-5,6,9,10,11,12-hexahydro-4H,8H-azepino[4',5':4,5]pyrrolo[3,2,1-ij]quinoline;
4-(3-methoxyphenylsulfonyl)-5,6,9,10,111,12-hexahydro-4H,8H-azepino[4',5':4,5]pyrrolo[3,2,1-ij]quinoline;
4-(2-methoxyphenylsulfonyl)-5,6,9,10,11,12-hexahydro-4H,8H-azepino[4',5':4,5]pyrrolo[3,2,1-ij]quinoline;
4-(4-bromo-2-methoxyphenylsulfonyl)-5,6,9,10,11,12-hexahydro-4H,8H-azepino[4',5':4,5]pyrrolo[3,2,1-ij]quinoline;
5,6,8,9,10,11,12,12a-octahydro-4H,7aH-azepino[4',5':4,5]pyrrolo[3,2,1-ij]quinolin-5-ol;
5-methoxy-5,6,8,9,10,11,12,12a-octahydro-4H,7aH-azepino[4',5':4,5]pyrrolo[3,2,1-ij]quinoline;
5-benzyloxy-5,6,8,9,10,11,12,12a-octahydro-4H,7aH-azepino[4',5':4,5]pyrrolo[3,2,1-ij]quinoline;
5-phenoxy-5,6,9,10,11,12-hexahydro-4H,8H-azepino[4',5':4,5]pyrrolo[3,2,1-ij]quinoline;
5-(4-chlorophenoxy)-5,6,9,10,11,12-hexahydro-4H,8H-azepino[4',5':4,5]pyrrolo[3,2,1-ij]quinoline;
5-(3-chlorophenoxy)-5,6,9,10,11,12-hexahydro-4H,8H-azepino[4',5':4,5]pyrrolo[3,2,1-ij]quinoline;
5-(2-chlorophenoxy)-5,6,9,10,11,12-hexahydro-4H,8H-azepino[4',5':4,5]pyrrolo[3,2,1-ij]quinoline;
5-(4-methoxyphenoxy)-5,6,9,10,11,12-hexahydro-4H,8H-azepino[4',5':4,5]pyrrolo[3,2,1-ij]quinoline;
5-(3-methoxyphenoxy)-5,6,9,10,11,12-hexahydro-4H,8H-azepino[4',5':4,5]pyrrolo[3,2,1-ij]quinoline;
5-(2-methoxyphenoxy)-5,6,9,10,11,12-hexahydro-4H,8H-azepino[4',5':4,5]pyrrolo[3,2,1-ij]quinoline;
5-(4-bromo-2-methoxyphenoxy)-5,6,9,10,11,12-hexahydro-4H,8H-azepino[4',5':4,5]pyrrolo[3,2,1-ij]quinoline;
5,6,9,10,11,12-hexahydro-4H,8H-azepino[4',5':4,5]pyrrolo[3,2,1-ij]quinolin-5-amine;
N-phenyl-5,6,9,10,11,12-hexahydro-4H,8H-azepino[4',5':4,5]pyrrolo[3,2,1-ij]quinolin-5-amine;
N-(4-chlorophenyl)-5,6,9,10,11,12-hexahydro-4H,8H-azepino[4',5':4,5]pyrrolo[3,2,1-ij]quinolin-5-amine;
N-(3-chlorophenyl)-5,6,9,10,11,12-hexahydro-4H,8H-azepino[4',5':4,5]pyrrolo[3,2,1-ij]quinolin-5-amine;
N-(2-chlorophenyl)-5,6,9,10,11,12-hexahydro-4H,8H-azepino[4',5':4,5]pyrrolo[3,2,1-ij]quinolin-5-amine;
N-(4-methoxyphenyl)-5,6,9,10,11,12-hexahydro-4H,8H-azepino[4',5':4,5]pyrrolo[3,2,1-ij]quinolin-5-amine;
N-(3-methoxyphenyl)-5,6,9,10,11,12-hexahydro-4H,8H-azepino[4',5':4,5]pyrrolo[3,2,1-ij]quinolin-5-amine;
N-(2-methoxyphenyl)-5,6,9,10,11,12-hexahydro-4H,8H-azepino[4',5':4,5]pyrrolo[3,2,1-ij]quinolin-5-amine;
N-(4-bromo-2-methoxyphenyl)-5,6,9,10,11,12-hexahydro-4H,8H-azepino[4',5':4,5]pyrrolo[3,2,1-ij]quinolin-5-amine;
5,6,9,10,11,12-hexahydro-4H,8H-azepino[4',5':4,5]pyrrolo[3,2,1-ij]quinoline-5-thione;
5-(phenylsulfonyl)-5,6,9,10,11,12-hexahydro-4H,8H-azepino[4',5':4,5]pyrrolo[3,2,1-ij]quinoline;
5-(4-chlorophenylsulfonyl)-5,6,9,10,11,12-hexahydro-4H,8H-azepino[4',5':4,5]pyrrolo[3,2,1-ij]quinoline;
5-(3-chlorophenylsulfonyl)-5,6,9,10,11,12-hexahydro-4H,8H-azepino[4',5':4,5]pyrrolo[3,2,1-ij]quinoline;
5-(2-chlorophenylsulfonyl)-5,6,9,10,11,12-hexahydro-4H,8H-azepino[4',5':4,5]pyrrolo[3,2,1-ij]quinoline;
5-(4-methoxyphenylsulfonyl)-5,6,9,10,11,12-hexahydro-4H,8H-azepino[4',5':4,5]pyrrolo[3,2,1-ij]quinoline;
5-(3-methoxyphenylsulfonyl)-5,6,9,10,11,12-hexahydro-4H,8H-azepino[4',5':4,5]pyrrolo[3,2,1-ij]quinoline;
5-(2-methoxyphenylsulfonyl)-5,6,9,10,11,12-hexahydro-4H,8H-azepino[4',5':4,5]pyrrolo[3,2,1-ij]quinoline;
3-(4-bromo-2-methoxyphenylsulfonyl)-5,6,9,10,11,12-hexahydro-4H,8H-azepino[4',5':4,5]pyrrolo[3,2,1-ij]quinoline;
4-(4-chlorophenoxy)-5,6,9,10,11,12-hexahydro-4H,8H-azepino[4',5':4,5]pyrrolo[3,2,1-ij]quinoline;
1-[2-(4-fluorophenoxy)ethoxy]-5,6,9,10,11,12-hexahydro-4H,8H-azepino[4',5':4,5]pyrrolo[3,2,1-ij]quinoline;
1-[2-(3-fluorophenoxy)ethoxy]-5,6,9,10,11,12-hexahydro-4H,8H-azepino[4',5':4,5]pyrrolo[3,2,1-ij]quinoline;
1-[2-(2-fluorophenoxy)ethoxy]-5,6,9,10,11,12-hexahydro-4H,8H-azepino[4',5':4,5]pyrrolo[3,2,1-ij]quinoline;
1-[2-(4-chlorophenoxy)ethoxy]-5,6,9,10,11,12-hexahydro-4H,8H-azepino[4',5':4,5]pyrrolo[3,2,1-ij]quinoline;
1-[2-(3-chlorophenoxy)ethoxy]-5,6,9,10,11,12-hexahydro-4H,8H-azepino[4',5':4,5]pyrrolo[3,2,1-ij]quinoline;
1-[2-(2-chlorophenoxy)ethoxy]-5,6,9,10,11,12-hexahydro-4H,8H-azepino[4',5':4,5]pyrrolo[3,2,1-ij]quinoline;
1-[2-(4-bromophenoxy)ethoxy]-5,6,9,10,11,12-hexahydro-4H,8H-azepino[4',5':4,5]pyrrolo[3,2,1-ij]quinoline;
1-[2-(3-bromophenoxy)ethoxy]-5,6,9,10,11,12-hexahydro-4H,8H-azepino[4',5':4,5]pyrrolo[3,2,1-ij]quinoline;
1-[2-(2-bromophenoxy)ethoxy]-5,6,9,10,11,12-hexahydro-4H,8H-azepino[4',5':4,5]pyrrolo[3,2,1-ij]quinoline;
1-[2-(4-methoxyphenoxy)ethoxy]-5,6,9,10,11,12-hexahydro-4H,8H-azepino[4',5':4,5]pyrrolo[3,2,1-ij]quinoline;
1-[2-(3-methoxyphenoxy)ethoxy]-5,6,9,10,11,12-hexahydro-4H,8H-azepino[4',5':4,5]pyrrolo[3,2,1-ij]quinoline;
1-[2-(2-methoxyphenoxy)ethoxy]-5,6,9,10,11,12-hexahydro-4H,8H-azepino[4',5':4,5]pyrrolo[3,2,1-ij]quinoline;
1-[2-(4-methylphenoxy)ethoxy]-5,6,9,10,11,12-hexahydro-4H,8H-azepino[4',5':4,5]pyrrolo[3,2,1-ij]quinoline;
1-[2-(3-methylphenoxy)ethoxy]-5,6,9,10,11,12-hexahydro-4H,8H-azepino[4',5':4,5]pyrrolo[3,2,1-ij]quinoline;
1-[2-(2-methylphenoxy)ethoxy]-5,6,9,10,11,12-hexahydro-4H,8H-azepino[4',5':4,5]pyrrolo[3,2,1-ij]quinoline;
1-[2-(1-naphthyloxy)ethoxy]-5,6,9,10,11,12-hexahydro-4H,8H-azepino[4',5':4,5]pyrrolo[3,2,1-ij]quinoline;
1-[2-(2-naphthyloxy)ethoxy]-5,6,9,10,11,12-hexahydro-4H,8H-azepino[4',5':4,5]pyrrolo[3,2,1-ij]quinoline;
1-[2-([1,1'-biphenyl]-4-yloxy)ethoxy]-5,6,9,10,11,12-hexahydro-4H,8H-azepino[4',5':4,5]pyrrolo[3,2,1-ij]quinoline;
1-[2-([1,1'-biphenyl]-3-yloxy)ethoxy]-5,6,9,10,11,12-hexahydro-4H,8H-azepino[4',5':4,5]pyrrolo[3,2,1-ij]quinoline;
1-[2-([1,1'-biphenyl]-2-yloxy)ethoxy]-5,6,9,10,11,12-hexahydro-4H,8H-azepino[4',5':4,5]pyrrolo[3,2,1-ij]quinoline;
1-{2-[4-(trifluoromethoxy)phenoxy]ethoxy}-5,6,9,10,11,12-hexahydro-4H,8H-azepino[4',5':4,5]pyrrolo[3,2,1-ij]quinoline;
1-{2-[3-(trifluoromethoxy)phenoxy]ethoxy}-5,6,9,10,11,12-hexahydro-4H,8H-azepino[4',5':4,5]pyrrolo[3,2,1-ij]quinoline;
1-{2-[2-(trifluoromethoxy)phenoxy]ethoxy}-5,6,9,10,11,12-hexahydro-4H,8H-azepino[4',5':4,5]pyrrolo[3,2,1-ij]quinoline;
1-{2-[4-(trifluoromethyl)phenoxy]ethoxy}-5,6,9,10,11,12-hexahydro-4H,8H-azepino[4',5':4,5]pyrrolo[3,2,1-ij]quinoline;
1-{2-[3-(trifluoromethyl)phenoxy]ethoxy}-5,6,9,10,11,12-hexahydro-4H,8H-azepino[4',5':4,5]pyrrolo[3,2,1-ij]quinoline; or
1-{2-[2-(trifluoromethyl)phenoxy]ethoxy}-5,6,9,10,11,12-hexahydro-4H,8H-azepino[4',5':4,5]pyrrolo[3,2,1-ij]quinoline; or a pharmaceutically acceptable salt thereof.

35. A pharmaceutical composition comprising a compound of any one of claims 31 to 34 and a pharmaceutically acceptable excipient.

36. A compound of any one of claims 31 to 34 for use in medical diagnosis or therapy.

37. The compound of claim 36 wherein the therapy is the treatment of a disease or disorder of the central nervous system.

38. The compound of claim 37 wherein the disease or disorder of the central nervous system is obesity, depression, schizophrenia, schizophreniform disorder, schizoaffective disorder, delusional disorder, a stress related disease, panic disorder, a phobia, obsessive compulsive disorder, post-traumatic-stress syndrome, immune system depression, a stress induced problem with the urinary, gastrointestinal or cardiovascular system, neurodegenerative disorders, autism, chemotherapy-induced vomiting, hypertension, migraine, headaches, cluster headaches, sexual dysfunction, addictive disorder and withdrawal syndrome, an adjustment disorder, an age-associated learning and mental disorder, anorexia nervosa, apathy, an attention-deficit disorder due to general medical conditions, attention-deficit hyperactivity disorder, behavioral disturbance, bipolar disorder, bulimia nervosa, chronic fatigue syndrome, conduct disorder, cyclothymic disorder, dysthymic disorder, fibromyalgia and other somatoform disorders, generalized anxiety disorder, an inhalation disorder, an intoxication disorder, movement disorder, oppositional defiant disorder, peripheral neuropathy, post-traumatic stress disorder, premenstrual dysphoric disorder, a psychotic disorder, mood disorder, seasonal affective disorder, a sleep disorder, a specific development disorder, agitation disorder, selective serotonin reuptake inhibition syndrome, or a Tic disorder.

39. The compound of claim 36 wherein the therapy is the treatment of anxiety, obesity, depression, or a stress related disease.

40. The use of a compound of any one of claims 31 to 34 to prepare a medicament for treating or preventing a disease or disorder of the central nervous system.

41. The use of claim 40 wherein the disease or disorder of the central nervous system is obesity, depression, schizophrenia, schizophreniform disorder, schizoaffective disorder, delusional disorder, a stress related disease, panic disorder, a phobia, obsessive compulsive disorder, post-traumatic-stress syndrome, immune system depression, a stress induced problem with the urinary, gastrointestinal or cardiovascular system, neurodegenerative disorders, autism, chemotherapy-induced vomiting, hypertension, migraine, headaches, cluster headaches, sexual dysfunction, addictive disorder and withdrawal syndrome, an adjustment disorder, an age-associated learning and mental disorder, anorexia nervosa, apathy, an attention-deficit disorder due to general medical conditions, attention-deficit hyperactivity disorder, behavioral disturbance, bipolar disorder, bulimia nervosa, chronic fatigue syndrome, conduct disorder, cyclothymic disorder, dysthymic disorder, fibromyalgia and other somatoform disorders, generalized anxiety disorder, an inhalation disorder, an intoxication disorder, movement disorder, oppositional defiant disorder, peripheral neuropathy, post-traumatic stress disorder, premenstrual dysphoric disorder, a psychotic disorder, mood disorder, seasonal affective disorder, a sleep disorder, a specific development disorder, agitation disorder, selective serotonin reuptake inhibition syndrome, or a Tic disorder.

42. The use of claim 40 wherein the disease or disorder of the central nervous system is anxiety, obesity, depression, or a stress related disease.

43. The use of claim 40 wherein the disease or condition is anxiety, obesity, depression, schizophrenia, a stress related disease, panic disorder, a phobia, obsessive compulsive disorder, post-traumatic-stress syndrome, immune system depression, a stress induced problem with the gastrointestinal or cardiovascular system or sexual dysfunction.

44. A compound of formula I: 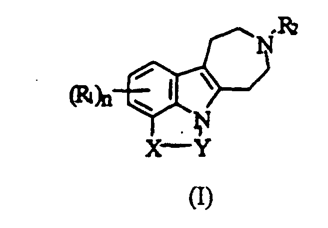 wherein,
each R₁ is independently hydroxy, nitro, halo, cyano, trifluoromethyl, trifluoromethoxy, C₁₋₇alkyl, C₁₋₇alkoxy, C₁₋₇alkanoyl, C₁₋ ₇alkoxycarbonyl, C₁₋₇alkanoyloxy, aryl, heteroaryl, -S(O)ₘNRₐR_{b}, NR_{c}R_{d}, - S(O)ₘRₑ, or -C(=O)NRₐR_{b}, wherein any C₁₋₇alkyl, C₁₋₇alkoxy, C₁₋₇alkanoyl, C₁₋₇alkoxycarbonyl, or C₁₋₇alkanoyloxy of R₁ is optionally partially unsaturated and is optionally substituted with aryl, aryloxy, heteroaryl, heteroaryloxy, hydroxy, nitro, halo, cyano, C₁₋₇alkoxy, C₁₋₇alkanoyl, C₁₋₇alkoxycarbonyl, C₁₋ ₇alkanoyloxy, -S(O)ₘRₑ, -S(O)ₘNRₐR_{b}, NR_{c}R_{d}, or -C(=O)NRₐR_{b};
R₂ is a suitable protecting group;
X and Y together are a 2, 3, or 4 membered saturated or partially unsaturated chain comprising one or more carbon atoms and optionally comprising one oxy (-O-), thio (-S-), sulfinyl (-SO-), sulfonyl (S(O)₂-), or NR_{f} in the chain; wherein the chain is optionally substituted on each carbon with oxo (=O), thioxo (=S), -NR_{q}Rᵣ, -S(O)ₚRₛ, or -ORₜ, or with one or two substituents independently selected from the group consisting of C₁₋₇alkyl, (C₁₋₇alkoxy)C₁₋ ₇alkyl, aryl, (aryl)C₁₋₇alkyl, heteroaryl, (heteroaryl)C₁₋₇alkyl, and (aryl)oxyC₁₋₇alkyl; or wherein the chain is optionally substituted on a carbon with a 4, 5, or 6 membered spirocyclic carbon ring; or wherein the chain is optionally substituted on two adjacent atoms with a 2, 3, or 4 membered alkylene chain (e.g. -CH₂CH₂-, -CH₂CH₂CH₂-, or -CH₂CH₂CH₂CH₂-) forming a ring that is fused to the ring comprising X and Y;
each m is independently 0, 1, or 2;
n is 0, 1, 2, or 3;
p is 0, 1, or 2;
each Rₐ and R_{b} is independently hydrogen, C₁₋₇alkyl, aryl, (aryl)C₁₋₇alkyl, heteroaryl, or (heteroaryl)C₁₋₇alkyl; or Rₐ and R_{b} together with the nitrogen to which they are attached form a pyrrolidino, piperidino, morpholino, or thiomorpholino ring;
each R_{c} and R_{d} is independently hydrogen, C₁₋₇alkyl, C₁₋₇alkanoyl, C₁₋₇alkoxycarbonyl, aryl, (aryl)C₁₋₇alkyl, heteroaryl, (heteroaryl)C₁₋₇alkyl, arylcarbonyl, heteroarylcarbonyl, aryloxycarbonyl, or heteroaryloxycarbonyl; or R_{c} and R_{d} together with the nitrogen to which they are attached form a pyrrolidino, piperidino, morpholino, or thiomorpholino ring;
each Rₑ is independently hydrogen, C₁₋₇alkyl, aryl, (aryl)C₁₋₇alkyl, heteroaryl, or (heteroaryl)C₁₋₇alkyl;
R_{f} is hydrogen, C₁₋₇alkyl, aryl, (aryl)C₁₋₇alkyl, heteroaryl, (heteroaryl)C₁₋₇alkyl, or is a bond to a 2, 3, or 4 membered alkylene chain that forms a ring that is fused to the ring comprising X and Y;
each R_{q} and Rᵣ is independently hydrogen, C₁₋₇alkyl, aryl, (aryl)C₁₋₇alkyl, heteroaryl, or (heteroaryl)C₁₋₇alkyl; or R_{q} and Rᵣ together with the nitrogen to which they are attached form a pyrrolidino, piperidino, morpholino, or thiomorpholino ring;
Rₛ is C₁₋₇alkyl, aryl, (aryl)C₁₋₇alkyl, heteroaryl, or (heteroaryl)C₁₋ ₇alkyl; and
Rₜ is hydrogen, C₁₋₇alkyl, aryl, (aryl)C₁₋₇alkyl, heteroaryl, or (heteroaryl)C₁₋₇alkyl;
wherein any aryl or heteroaryl ring of R₁, R₂, X, Y, Rₐ-R_{f}, or R_{q}-Rₜ is optionally substituted with one or more (e.g. 1, 2, 3, or 4) substituents independently selected from halo, hydroxy, cyano, nitro, trifluoromethyl, trifluoromethoxy, C₁₋₇alkyl, C₁₋₇alkoxy, phenyl, sulfonyl, NRⱼRₖ, or-C(=O)NRⱼR_{k,}; wherein each Rⱼ and Rₖ is independently hydrogen, C₁₋₇alkyl, C₁₋₇alkanoyl, C₁₋₇alkoxycarbonyl, aryl, (aryl)C₁₋₇alkyl, arylcarbonyl, or aryloxycarbonyl; or Rⱼ and Rₖ together with the nitrogen to which they are attached form a pyrrolidino, piperidino, morpholino, or thiomorpholino ring;
or a pharmaceutically acceptable salt thereof;
provided Y is not oxy, thio, sulfinyl, or NR_{f}; and
provided X and Y together are not a 2-membered unsaturated chain; and
provided no carbon of X and Y is bonded to more than one oxy, thio, sulfinyl, or NR_{f}.

45. The compound of claim 1 wherein R₂ is C₁₋₄alkyl, C₁₋₄alkanoyl, arylcarbonyl, (aryl)C₁₋₂alkyl, C₁₋₄alkoxycarbonyl, aryloxycarbonyl, arylsulfonyl, or (aryl)methoxycarbonyl, wherein any aryl is optionaly substituted with 1, 2, or 3 substituents independently selected from C₁₋₄alkyl and trifluoromethyl.

46. The compound of claim 1 wherein R₂ is methyl, ethyl, propyl, isopropyl, acetyl, *tert*-butoxycarbonyl, benzyloxycarbonyl benzyl, or *p*-toluenesulfonyl.

47. The compound of claim 1 which is: 10-benzoyl-5,6,9,10,11,12-hexahydro-4H,8H-azepino[4',5':4,5]pyrrolo[3,2,1-ij]quinolin-4- one;
10-benzoyl-2-fluoro-5,6,9,10,11,12-hexahydro-4H,8H-azepino[4',5':4,5]pyrrolo[3,2,1-ij]quinolin-4- one;
10-benzoyl-2-chloro-5,6,9,10,11,12-hexahydro-4H,8H-azepino[4',5':4,5]pyrrolo[3,2,1-ij]quinolin-4- one;
10-benzoyl-6-methyl-5,6,9,10,11,12-hexahydro-4H,8H-azepino[4',5':4,5]pyrrolo[3,2,1-ij]quinolin-4-one;
10-benzoyl-2-fluoro-6-methyl-5,6,9,10,11,12-hexahydro-4H,8H-azepino[4',5':4,5]pyrrolo[3,2,1-ij]quinolin-4-one;
10-benzoyl-2,3-dichloro-6-methyl-5,6,9,10,11,12-hexahydro-4H,8H-azepino[4',5':4,5]pyrrolo[3,2,1-ij]quinolin-4-one;
10-benzoyl-6-propyl-5,6,9,10,11,12-hexabydro-4H,8H-azepino[4',5':4,5]pyrrolo[3,2,1-ij]quinolin-4-one;
10-benzoyl-6-(trifluoromethyl)-5,6,9,10,11,12-hexahydro-4H,8H-azepino[4',5':4,5]pyrrolo[3,2,1-ij]quinolin-4-one;
10-benzoyl-5,6,8,9,10,11,12,12a-octahydro-4H,7aH-azepino[4',5':4,5]pyrrolo[3,2,1-ij]quinolin-4-ol; or
10-benzoyl-4-methoxy-5,6,8,9,10,11,12,12a-octahydro-4H,7aH-azepino[4',5':4,5]pyrrolo[3,2,1-ij]quinoline.

48. The compound of claim 1 which is: benzyl 5,6,8,9,11,12-hexahydro-4H,10H-azepino[4',5':4,5]pyrrolo[3,2,1-ij]quinoline-10-carboxylate;
benzyl 2-methyl-5,6,8,9,11,12-hexahydro-4H,10H-azepino[4',5':4,5]pyrrolo[3,2,1-ij]quinoline-10-carboxylate;
10-benzoyl-1-methoxy-5,6,9,10,11,12-hexahydro-4H,8H-azepino[4',5':4,5]pyrrolo-[3,2,1-ij]quinoline;
benzyl 2-fluoro-5,6,8,9,11,12-hexahydro-4H,10H-azepino[4',5':4,5]pyrrolo[3,2,1-ij]quinoline-10-carboxylate;
benzyl 6-methyl-5,6,8,9,11,12-hexahydro-4H,10H-azepino[4',5':4,5]pyrrolo[3,2,1-ij]quinoline-10-carboxylate;
benzyl 5-methyl-5,6,8,9,11,12-hexahydro-4H,10H-azepino[4',5':4,5]pyrrolo[3,2,1-ij]quinolino-10-carboxylate;
benzyl 4-methyl-5,6,8,9,11,12-hexahydro-4H,10H-azepino[4',5':4,5]pyrrolo[3,2,1-ij]quinoline-10-carboxylate;
(+)-benzyl 6-methyl-5,6,8,9,11,12-hexahydro-4H,10H-azepino[4',5':4,5]pyrrolo[3,2,1-ij]quinoline-10-carboxylate;
(-)-benzyl 6-methyl-5,6,8,9,11,12-hexahydro-4H,10H-azepino[4',5':4,5]pyrrolo[3,2,1-ij]quinoline-10-carboxylate;
(+)-benzyl 5-methyl-5,6,8,9,11,12-hexahydro-4H,10H-azepino[4',5':4,5]pyrrolo[3,2,1-ij]quinoline-10-carboxylate;
(-)-benzyl 5-methyl-5,6,8,9,11,12-hexahydro-4H,10H-azepino[4',5':4,5]pyrrolo[3,2,1-ij]quinoline-10-carboxylate;
(+)-benzyl 4-methyl-5,6,8,9,11,12-hexahydro-4H,10H-azepino[4',5':4,5]pyrrolo[3,2,1-ij]quinoline-10-carboxylate;
(-)-benzyl 4-methyl-5,6,8,9,11,12-hexahydro-4H,10H-azepino[4',5':4,5]pyrrolo[3,2,1-ij]quinoline-10-carboxylate;
benzyl 2-methyl-1,2,7,8,10,11-hexahydro-9H-azepino[4,5-b][1,4]oxazino[2,3,4-hi]indole-9-carboxylate;
benzyl 2,2-dimethyl-1,2,7,8,10,11-hexahydro-9H-azepino[4,5-b][1,4]oxazino[2,3,4-hi]indole-9-carboxylate;
benzyl 4-fluoro-1,2,7,8,10,11-hexahydro-9H-azepino[4,5-b][1,4]oxazino[2,3,4-hi]indole-9-carboxylate;
benzyl 4-chloro-1,2,7,8,10,11-hexahydro-9H-azepino[4,5-b][1,4]oxazino[2,3,4-hi]indole-9-carboxylate;
benzyl 5-fluoro-1,2,7,8,10,11-hexahydro-9H-azepino[4,5-b][1,4]oxazino[2,3,4-hi]indole-9-carboxylate;
benzyl 5-chloro-1,2,7,8,10,11-hexahydro-9H-azepino[4,5-b][1,4]oxazino[2,3,4-hi]indole-9-carboxylate;
benzyl 5-methyl-1,2,7,8,10,11-hexahydro-9H-azepino[4,5-b][1,4]oxazino[2,3,4-hi]indole-9-carboxylate;
benzyl 6-fluoro-1,2,7,8,10,11-hexahydro-9H-azepino[4,5-b][1,4]oxazino[2,3,4-hi]indole-9-carboxylate;
benzyl 6-chloro-1,2,7,8,10,11-hexahydro-9H-azepino[4,5-b][1,4]oxazino[2,3,4-hi]indole-9-carboxylate;
benzyl 6-methyl-1,2,6b,7,8,10,11,11a-octahydro-9H-azepino[4,5-b][1,4]oxazino[2,3,4-hi]indole-9-carboxylate;
benzyl 6-(trifluoromethyl)-1,2,7,8,10,11-hexahydro-9H-azepino[4,5-b][1,4]oxazino [2,3,4-hi]indole-9-carboxylate;
benzyl 5,6-difluoro-1,2,7,8,10,11-hexahydro-9H-azepino[4,5-b][1,4]oxazino[2,3,4-hi]indole-9-carboxylatebenzyl;
benzyl 5-chloro-6-fluoro-1,2,7,8,10,11-hexahydro-9H-azepino[4,5-b][1,4]oxazino[2,3,4-hi]indole-9-carboxylate;
benzyl 5-fluoro-6-chloro-1,2,7,8,10,11-hexahydro-9H-azepino[4,5-b][1,4]oxazino[2,3,4-hi]indole-9-carboxylate;
benzyl 5,6-dichloro-1,2,7,8,10,11-hexahydro-9H-azepino[4,5-b][1,4]oxazino[2,3,4-hi]indole-9-carboxylate;
benzyl 4,6-dichloro-1,2,7,8,10,11-hexahydro-9H-azepino[4,5-b][1,4]oxazino[2,3,4-hi]indole-9-carboxylate;
7-benzoyl 4,5,8,9,10,11-hexahydro-7H-azepino[4,5-b]pyrrolo[3,2,1-hi]indole;
7-benzoyl 2-fluoro-4,5,8,9,10,11-hexahydro-7H-azepino[4,5-b]pyrrolo[3,2,1-hi]indole;
7-benzoyl 2-methoxy-4,5,8,9,10,11-hexahydro-7H-azepino[4,5-b]pyrrolo[3,2,1-hi]indole;
benzyl-5-methyl-4,5,8,9,10,11-hexahydro-7H-azepino[4,5-b]pyrrolo[3,2,1-hi]-7-carboxylate;
benzyl-4,5-dimethyl-4,5,8,9,10,11-hexahydro-7H-azepino[4,5-b]pyrrolo[3,2,1-hi]indole-7-carboxylate;
benzyl-2,3,4,5,8b,9,10,11,12,12a-decahydro-1H-azepino[4',5':4,5]pyrrolo[3,2,1-jk]carbazole-7-carboxylate;
benzyl-1-chloro-2-fluoro-4,5,8,9,10,11-hexahydro-7H-azepino[4,5-b]pyrrolo[3,2,1-hi]indole-7-carboxylate;
benzyl-2-chloro-4,5,8,9,10,11-hexahydro-7H-azepino[4,5-b]pyrrolo[3,2,1-hi]indole-7-carboxylate;
benzyl-1-chloro-4,5,8,9,10,11-hexahydro-7H-azepino[4,5-b]pyrrolo[3,2,1-hi]indole-7-carboxylate;
10-benzoyl-5,6,9,10,11,12-hexahydro-4H,8H-azepino[4',5':4,5]pyrrolo[3,2,1-ij]quinolin-4-one;
10-benzoyl-2-fluoro-5,6,9,10,11,12-hexahydro-4H,8H-azepino[4',5':4,5]pyrrolo[3,2,1-ij]quinolin-4-one;
10-benzoyl-2-chloro-5,6,9,10,11,12-hexahydro-4H,8H-azepino[4',5':4,5]pyrrolo[3,2,1-ij]quinolin-4-one;
10-benzoyl-6-methyl-5,6,9,10,11,12-hexahydro-4H,8H-azepino[4',5':4,5]pyrrolo-[3,2,1-ij]quinolin-4-one;
10-benzoyl-2-fluoro-6-methyl-5,6,9,10,11,12-hexahydro-4H,8H-azepino[4',5':4,5]-pyrrolo[3,2,1-ij]quinolin-4-one;
10-benzoyl-2,3-dichloro-6-methyl-5,6,9,10,11,12-hexahydro-4H,8H-azepino[4',5':4,5]pyrrolo[3,2,1-ij]quinolin-4-one;
10-benzoyl-6-propyl-5,6,9,10,11,12-hexahydro-4H,8H-azepino[4',5':4,5]pyrrolo[3,2,1-ij]quinolin-4-one;
10-benzoyl-6-(trifluoromethyl)-5,6,9,10,11,12-hexahydro-4H,8H-azepino[4',5':4,5]pyrrolo[3,2,1-ij]quinolin-4-one;
10-benzoyl-5,6,8,9,10,11,12,12a-octahydro-4H,7aH-azepino[4',5':4,5]pyrrolo[3,2,1-ij]quinolin-4-ol; and
10-benzoyl-4-methoxy-5,6,8,9,10,11,12,12a-octahydro-4H,7aH-azepino[4',5':4,5]pyrrolo[3,2,1-ij]quinoline.

49. A method for preparing a compound of formula (I) wherein R₂ is hydrogen comprising deprotecting a corresponding compound of formula (I) wherein R₂ is a protecting group.

50. The method of claim 49 wherein the protecting group is benzyloxycarbonyl or benzoyl.

## Patentansprüche

1. Verbindung der Formel I: 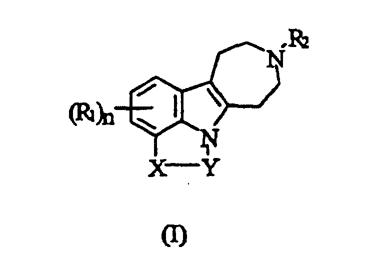 worin
jedes R₁ unabhängig voneinander Hydroxy, Nitro, Halogen, Cyano, Trifluormethyl, Trifluormethoxy, C₁₋₇-Alkyl, C₁₋₇-Alkoxy, C₁₋₇-Alkanoyl, C₁₋₇-Alkoxycarbonyl, C₁₋₇-Alkanoyloxy, Aryl, Heteroaryl, -S(O)ₘNRₐR_{b}, NR_{c}R_{d}, -S(O)ₘRₑ oder -C(=O)RₐR_{b} bedeutet, wobei jedes C₁₋₇-Alkyl, C₁₋₇-Alkoxy, C₁₋₇-Alkanoyl, C₁₋₇-Alkoxycarbonyl oder C₁₋₇-Alkanoyloxy von R₁ optional partiell ungesättigt ist und optional mit Aryl, Aryloxy, Heteroaryl, Heteroaryloxy, Hydroxy, Nitro, Halogen, Cyano, C₁₋₇-Alkoxy, C₁₋₇-Alkanoyl, C₁₋₇-Alkoxycarbonyl, C₁₋₇-Alkanoyloxy, -S(O)ₘRₑ, -S(O)ₘNRₐR_{b}, NR_{c}R_{d} oder -C(=O)NRₐR_{b} substituiert ist;
R₂ Wasserstoff, C₁₋₇-Alkyl, C₁₋₇-Alkanoyl, Arylcarbonyl, Aryl, (Aryl)C₁₋₇-alkyl, C₁₋₇-Alkoxycarbonyl, Aryloxycarbonyl, Arylsulfonyl oder (Aryl)C₁₋₇-alkoxycarbonyl bedeutet;
X und Y zusammen eine 2-, 3- oder 4-gliedrige gesättigte oder partiell ungesättigte Kette bilden, die ein oder mehrere Kohlenstoffatome umfasst und optional ein Oxy (-O-), Thio (-S-), Sulfinyl (-SO-), Sulfonyl (S(O)₂-) oder NR_{f} in der Kette umfasst; wobei die Kette an jedem Kohlenstoff optional mit Oxo (=O), Thioxo (=S), -NR_{q}Rᵣ, -S(O)ₚRₛ oder -ORₜ oder mit einem oder zwei Substituenten, die unabhängig voneinander aus der aus C₁₋₇-Alkyl, (C₁₋₇-Alkoxy)C₁₋₇-alkyl, Aryl, (Aryl)C₁₋₇-alkyl, Heteroaryl, (Heteroaryl)C₁₋₇-alkyl und (Aryl)oxy-C₁₋₇-alkyl bestehenden Gruppe ausgewählt sind, substituiert ist; oder wobei die Kette optional an einem Kohlenstoff mit einem 4-, 5- oder 6-gliedrigen spirocyclischen Kohlenstoffring substituiert ist; oder wobei die Kette optional an zwei benachbarten Atomen mit einer 2-, 3-oder 4-gliedrigen Alkylenkette (beispielsweise -CH₂CH₂-, -CH₂CH₂CH₂- oder -CH₂CH₂CH₂CH₂-) unter Bildung eines Rings, der an den X und Y umfassenden Ring kondensiert ist, substituiert ist;
jedes m unabhängig voneinander 0, 1 oder 2 bedeutet;
n 0, 1, 2 oder 3 bedeutet;
p 0, 1 oder 2 bedeutet;
jedes Rₐ und R_{b} unabhängig voneinander Wasserstoff, C₁₋₇-Alkyl, Aryl, (Aryl)C₁₋₇-alkyl, Heteroaryl oder (Heteroaryl)-C₁₋₇-alkyl bedeuten; oder Rₐ und R_{b} zusammen mit dem Stickstoff, an den sie gebunden sind, einen Pyrrolidino-, Piperidino-, Morpholino- oder Thiomorpholinoring bilden; jedes R_{c} und R_{d} unabhängig voneinander Wasserstoff, C₁₋₇-Alkyl, C₁₋₇-Alkanoyl, C₁₋₇-Alkoxycarbonyl, Aryl, (Aryl)C₁₋₇alkyl, Heteroaryl, (Heteroaryl)C₁₋₇-alkyl, Arylcarbonyl, Heteroarylcarbonyl, Aryloxycarbonyl oder Heteroaryloxycarbonyl bedeuten; oder R_{c} und R_{d} zusammen mit dem Stickstoff, an dem sie gebunden sind, einen Pyrrolidino-, Piperidino-, Morpholino- oder Thiomorpholinoring bilden;
jedes Rₑ unabhängig voneinander Wasserstoff, C₁₋₇-Alkyl, Aryl, (Aryl)C₁₋₇-alkyl, Heteroaryl oder (Heteroaryl)C₁₋₇alkyl bedeutet;
R_{f} Wasserstoff, C₁₋₇-Alkyl, Aryl, (Aryl)C₁₋₇-alkyl, Heteroaryl, (Heteroaryl)C₁₋₇-alkyl oder eine Bindung zu einer 2-, 3- oder 4-gliedrigen Alkylenkette, die einen Ring, der an den X- und Y umfassenden Ring kondensiert ist, bildet, bedeutet;
jedes R_{q} und Rᵣ unabhängig voneinander Wasserstoff, C₁₋₇-Alkyl, Aryl, (Aryl)C₁₋₇-alkyl, Heteroaryl oder (Heteroaryl)-C₁₋₇-alkyl bedeuten; oder R_{q} und Rₜ zusammen mit dem Stickstoff, an den sie gebunden sind, einen Pyrrolidino-, Piperidino-, Morpholino- oder Thiomorpholinoring bilden;
Rₛ C₁₋₇-Alkyl, Aryl, (Aryl)C₁₋₇-alkyl, Heteroaryl oder (Heteroaryl)C₁₋₇-alkyl bedeutet; und
Rₜ Wasserstoff, C₁₋₇-Alkyl, Aryl, (Aryl)C₁₋₇-alkyl, Heteroaryl oder (Heteroaryl)C₁₋₇-alkyl bedeutet;
wobei jeder Aryl- oder Heteroarylring von R₁, R₂, X, Y, Rₐ-R_{f} oder R_{q}-Rₜ optional mit einem oder mehreren (beispielsweise 1, 2, 3 oder 4) Substituenten substituiert ist, die unabhängig voneinander aus Halogen, Hydroxy, Cyano, Nitro, Trifluormethyl, Trifluormethoxy, C₁₋₇-Alkyl, C₁₋₇-Alkoxy, Phenyl, Sulfonyl, NRⱼRₖ oder -C(=O)NRⱼRₖ ausgewählt sind; wobei jedes Rⱼ und Rₖ unabhängig voneinander Wasserstoff, C₁₋₇-Alkyl, C₁₋₇-Alkanoyl, C₁₋₇-Alkoxycarbonyl, Aryl, (Aryl)C₁₋₇-alkyl, Arylcarbonyl oder Aryloxycarbonyl bedeuten; oder Rⱼ und Rₖ zusammen mit dem Stickstoff, an den sie gebunden sind, einen Pyrrolidino-, Piperidino-, Morpholinooder Thiomorpholinoring bilden;
oder ein pharmazeutisch akzeptables Salz derselben; mit dem Vorbehalt, dass Y nicht Oxy, Thio, Sulfinyl oder NR_{f} bedeutet; und
mit dem Vorbehalt, dass X und Y zusammen keine zweigliedrige ungesättigte Kette sind; und
mit dem Vorbehalt, dass kein Kohlenstoff von X und Y an mehr als ein Oxy, Thio, Sulfinyl oder NR_{f} gebunden ist.

2. Verbindung nach Anspruch 1, worin jedes R₁ unabhängig voneinander Hydroxy, Nitro, Halogen, Cyano, Trifluormethyl, Trifluormethoxy, C₁₋₇-Alkyl, C₁₋₇-Alkoxy, C₁₋₇-Alkanoyl, C₁₋₇-Alkoxycarbonyl, C₁₋₇-Alkanoyloxy, Aryl, Heteroaryl, - S(O)ₘNRₐR_{b}, NR_{c}R_{d}, -S(O)ₘRₑ oder -C(=O)NRₐR_{b} bedeutet, wobei jedes C₁₋₇-Alkyl, C₁₋₇-Alkoxy, C₁₋₇-Alkanoyl, C₁₋₇-Alkoxycarbonyl oder C₁₋₇-Alkanoyloxy von R₁ optional mit Aryl, Aryloxy, Heteroaryl, Heteroaryloxy, Hydroxy, Nitro, Halogen, Cyano, C₁₋₇-Alkoxy, C₁₋₇-Alkanoyl, C₁₋₇-Alkoxycarbonyl, C₁₋₇-Alkanoyloxy, -S(O)ₘRₑ, -S(O)ₘNRₐR_{b}, NR_{c}R_{d} oder -C(=O)NRₐR_{b} substituiert ist.

3. Verbindung nach Anspruch 1, worin jedes R₁ unabhängig voneinander Hydroxy, Nitro, Halogen, Cyano, Trifluormethyl, Trifluormethoxy, C₁₋₇-Alkyl, C₁₋₇-Alkoxy, C₁₋₇-Alkanoyl, C₁₋₇-Alkoxycarbonyl, C₁₋₇-Alkanoyloxy, Aryl, Heteroaryl, -S(O)ₘNRₐR_{b}, NR_{c}R_{d}, -S(O)ₘRₑ oder -C(=O)NRₐR_{b} bedeutet, wobei jedes C₁₋₇-Alkyl oder C₁₋₇-Alkoxy von R₁ optional mit Aryl, Aryloxy, Heteroaryl, Heteroaryloxy, Hydroxy, Nitro, Halogen, Cyano, C₁₋₇-Alkoxy, C₁₋₇-Alkanoyl, C₁₋₇-Alkoxycarbonyl, C₁₋₇-Alkanoyloxy, -S(O)ₘRₑ, -S(O)ₘNRₐR_{b}, NR_{c}R_{d} oder -C(=O)NRₐR_{b} substituiert ist.

4. Verbindung nach Anspruch 1, worin jedes R₁ unabhängig voneinander Hydroxy, Nitro, Halogen, Cyano, Trifluormethyl, Trifluormethoxy, C₁₋₇-Alkyl, C₁₋₇-Alkoxy, C₁₋₇-Alkanoyl, C₁₋₇-Alkoxycarbonyl, C₁₋₇-Alkanoyloxy, Aryl, Heteroaryl, -S(O)ₘNRₐR_{b}, NR_{c}R_{d}, -S(O)ₘRₑ oder -C(=O)NRₐR_{b} bedeutet, wobei jedes C₁₋₇-Alkyl optional mit Aryl, Aryloxy, Heteroaryl, Heteroaryloxy, Hydroxy, Nitro, Halogen, Cyano, C₁₋₇-Alkoxy, C₁₋₇-Alkanoyl, C₁₋₇-Alkoxycarbonyl, C₁₋₇-Alkanoyloxy, -S(O)ₘRₑ, -S(O)ₘNRₐR_{b}, NR_{c}R_{d} oder -C(=O)NRₐR_{b} substituiert ist.

5. Verbindung nach Anspruch 1, worin jedes R₁ unabhängig voneinander Hydroxy, Nitro, Halogen, Cyano, Trifluormethyl, Trifluormethoxy, C₁₋₇-Alkyl, C₁₋₇-Alkoxy, C₁₋₇-Alkanoyl, C₁₋₇-Alkoxycarbonyl, C₁₋₇-Alkanoyloxy, Aryl, Heteroaryl, -S(O)ₘNRₐR_{b}, NR_{c}R_{d}, -S(O)ₘRₑ oder -C(=O)RₐR_{b} bedeutet.

6. Verbindung nach Anspruch 1, worin jedes R₁ unabhängig voneinander C₁₋₇-Alkyl, C₁₋₇-Alkoxy, Trifluormethyl oder Halogen bedeutet.

7. Verbindung nach Anspruch 1, worin n 1 ist und R₁ C₁₋₇-Alkyl, C₁₋₇-Alkoxy oder Halogen bedeutet.

8. Verbindung nach Anspruch 1, worin n 1 ist und R₁ Methyl, Methoxy, Chlor oder Fluor bedeutet.

9. Verbindung nach Anspruch 1, worin R₂ Wasserstoff bedeutet.

10. Verbindung nach Anspruch 1, worin n 1, 2 oder 3 ist.

11. Verbindung nach Anspruch 1, worin n 0 ist.

12. Verbindung nach Anspruch 1, worin X und Y zusammen eine 2-, 3- oder 4-gliedrige gesättigte oder partiell ungesättigte Kette bilden, die ein oder mehrere Kohlenstoffatome umfasst und optional ein Oxy (-O-), Thio (-S-), Sulfinyl (-SO-), Sulfonyl -(S(O)₂-) oder NR_{f} in der Kette umfasst; wobei die Kette an jedem Kohlenstoff optional mit Oxo (=O), Thioxo (=S), -NR_{q}Rᵣ, -S(O)ₚRₛ oder ORₜ oder mit einem oder zwei Substituenten, die unabhängig voneinander aus der aus C₁₋₇-Alkyl, (C₁₋₇-Alkoxy)C₁₋₇-alkyl, Aryl, (Aryl)C₁₋₇-alkyl, Heteroaryl, (Heteroaryl)C₁₋₇-alkyl und (Aryl)oxy-C₁₋₇-alkyl bestehenden Gruppe ausgewählt sind, substituiert ist.

13. Verbindung nach Anspruch 1, worin X und Y zusammen eine 2-, 3- oder 4-gliedrige gesättigte oder partiell ungesättigte Kette bilden, die ein oder mehrere Kohlenstoffatome umfasst und optional ein Oxy (-O-), Thio (-S-), Sulfinyl (-SO-), Sulfonyl (S(O)₂- oder NR_{f} in der Kette umfasst; wobei die Kette an jedem Kohlenstoff optional mit Oxo (=O), Thioxo (=S), -NR_{q}Rᵣ, -S(O)ₚRₛ oder ORₜ substituiert ist.

14. Verbindung nach Anspruch 1, worin X und Y zusammen eine 2-, 3- oder 4-gliedrige gesättigte oder partiell ungesättigte Kette bilden, die ein oder mehrere Kohlenstoffatome umfasst und optional ein Oxy (-O-), Thio (-S-), Sulfinyl (-SO-), Sulfonyl (S(O)₂-) oder NR_{f} in der Kette umfasst;
wobei die Kette optional an jedem Kohlenstoff mit einem oder zwei Substituenten, die unabhängig voneinander aus der aus C₁₋₇-Alkyl, (C₁₋₇-Alkoxy)C₁₋₇-alkyl, Aryl, (Aryl)C₁₋₇alkyl, Heteroaryl, (Heteroaryl)C₁₋₇-alkyl und (Aryl)oxy-C₁₋₇alkyl bestehenden Gruppe ausgewählt sind, substituiert ist.

15. Verbindung nach Anspruch 1, worin X und Y zusammen eine 2-, 3- oder 4-gliedrige Kette bilden, die ein oder mehrere Kohlenstoffatome umfasst und optional ein Oxy, Thio, Sulfinyl, Sulfonyl oder NR_{f} in der Kette umfasst; wobei die Kette an jedem Kohlenstoff optional mit Oxo (=O), Hydroxy, (Aryl)oxy, Heteroaryl(oxy) oder C₁₋₇-Alkoxy oder mit einem oder zwei Substituenten, die unabhängig voneinander aus der aus C₁₋₇-Alkyl, (C₁₋₇-Alkoxy)C₁₋₇-alkyl, Aryl, (Aryl)C₁₋₇alkyl, Heteroaryl, (Heteroaryl)C₁₋₇-alkyl und (Aryl)oxy-C₁₋₇alkyl bestehenden Gruppe ausgewählt sind, substituiert ist und wobei die Kette optional an zwei benachbarten Atomen mit einer 2-, 3- oder 4-gliedrigen Alkylenkette unter Bildung eines Rings, der an den X und Y umfassenden Ring kondensiert ist, substituiert ist.

16. Verbindung nach Anspruch 1, worin X und Y zusammen eine 2-, 3- oder 4-gliedrige Kohlenstoffkette bedeuten, wobei die Kette optional an jedem Kohlenstoff mit Oxo, Hydroxy, (Aryl)oxy, Heteroaryl(oxy) oder C₁₋₇-Alkoxy oder mit einem oder zwei Substituenten, die unabhängig voneinander aus der aus C₁₋₇-Alkyl, (C₁₋₇-Alkoxy)C₁₋₇-alkyl, Aryl, (Aryl)C₁₋₇alkyl, Heteroaryl, (Heteroaryl)C₁₋₇-alkyl und (Aryl)oxy-C₁₋₇alkyl bestehenden Gruppe ausgewählt sind, substituiert ist.

17. Verbindung nach Anspruch 1, worin X und Y zusammen eine 2- oder 3-gliedrige Kohlenstoffkette, die optional an jedem Kohlenstoff mit Oxo oder Hydroxy oder mit einem oder zwei Resten von C₁₋₇-Alkyl substituiert ist, bedeuten.

18. Verbindung nach Anspruch 1, worin X -O-, -S- oder -C(R_{g})(Rₕ)- bedeutet, worin R_{g} und Rₕ jeweils unabhängig voneinander Wasserstoff, C₁₋₇-Alkyl, (C₁₋₇-Alkoxy)C₁₋₇-alkyl, Aryl, (Aryl)C₁₋₇-alkyl, Heteroaryl, (Heteroaryl)C₁₋₇-alkyl oder (Aryl)oxy-C₁₋₇-alkyl bedeuten oder R_{g} und Rₕ zusammen Oxo sind.

19. Verbindung nach Anspruch 1, worin Y -C(R_{g})(Rₕ)-, -C(R_{g})(Rₕ)C(R_{g})(Rₕ)-, -C(R_{g})(Rₕ)C(R_{g})(Rₕ)C(R_{g})(Rₕ)-, -C(R_{g})(Rₕ)C(=O)-, -C(R_{g})(Rₕ)C(R_{g})(Rₕ)C(=O)-, -C(=O)C(R_{g})(Rₕ)-oder -C(=O)C(R_{g})(Rₕ)C(R_{g})(Rₕ)- bedeutet und jedes R_{g} und Rₕ unabhängig voneinander Wasserstoff oder C₁₋₇-Alkyl bedeutet.

20. Verbindung nach Anspruch 1, worin X -O-, -S- oder -C(R_{g})(Rₕ)- bedeutet; und Y -C(R_{g})(Rₕ)C(=O)- oder -C(R_{g})(Rₕ)C(R_{g})(Rₕ)-, worin jedes R_{g} und Rₕ unabhängig voneinander Wasserstoff oder C₁₋₇-Alkyl ist, bedeutet.

21. Verbindung nach Anspruch 1, worin X -O- oder -Sbedeutet und Y -C(R_{g})(Rₕ)C(=O)-, -C(=O)C(R_{g})(Rₕ)- oder -C(R_{g})(Rₕ)C(R_{g})(Rₕ)-, worin jedes R_{g} und Rₕ unabhängig voneinander Wasserstoff oder C₁₋₇-Alkyl ist, bedeutet.

22. Verbindung nach Anspruch 1, worin X und Y zusammen -CH(R_{g})CH₂-, -CH(R_{g})CH(R_{g})CH₂-, -CH(R_{g})CH(R_{g})CH(R_{g})CH₂-, -C(R_{g})=C(R_{g})CH₂-, -CH=CHCH(R_{g})CH₂-, -CH(R_{g})CH=CHCH₂-, - O-CH₂CH₂-, -O-CH₂CH(R_{g})CH₂-, -S-CH₂CH₂-, -S-CH₂CH(R₉)CH₂-, -S(O)-CH₂CH₂-, -S(O)-CH₂CH(R_{g})CH₂-, -S(O)₂CH₂CH₂-, - S(O)₂CH₂CH(R_{g})CH₂-, -NR_{f}-CH₂CH₂-, -NR_{f}-CH₂CH(R_{g})CH₂-, -CH₂C(=O)-, -CH(R_{g})CH₂C(=O)-, -CH(R_{g})CH(R_{g})CH₂C(=O)-, -CH₂OC(=O)-, -CH(R_{g})CH₂OC(=O)-, -OCH₂C(=O)- oder -OCH₂CH₂C(=O)- bedeuten; wobei jedes R_{g} unabhängig voneinander -NR_{q}Rᵣ, -S(O)ₚRₛ oder -ORₜ bedeutet.

23. Verbindung nach Anspruch 1, worin X und Y zusammen -CH(R_{g})CH(R_{g})-, -CH(R_{g})CH(R_{g})CH(R_{g})-, -CH(R_{g})CH(R_{g})CH(R_{g})CH(R_{g})-, -CH=CHCH(R_{g})-, -C(R_{g})=C(R_{g})CH(R_{g})CH(R_{g})-, -CH(R_{g})C(R_{g})=C(R_{g})CH(R_{g})-, -O-CH(R_{g})CH(R_{g})-, -O-CH(R_{g})CH(R_{g})CH(R_{g})-, -S-CH(R_{g})CH(R_{g})-, -S-CH(R_{g})CH(R_{g})CH(R_{g})-, -S(O)-CH(R_{g})CH(R_{g})-, -S(O)-CH(R_{g})CH(R_{g})CH(R_{g})-, -S(O)₂CH(R_{g})CH(R_{g})-, - S(O)₂CH(R_{g})CH(R_{g})CH(R_{g})-, -NR_{f}-CH(R_{g})CH(R_{g})-, -NR_{f}-CH(R_{g})CH(R_{g})CH(R_{g})-, -CH(R_{g})C(=O)-, -CH(R_{g})CH(R_{g})C(=O)-, -CH(R_{g})CH(R_{g})CH(R_{g})C(=O)-, -CH(R_{g})OC(=O)-, -CH(R_{g})CH(R_{g})OC(=O)-, -OCH(R_{g})C(=O)- oder - OCH(R_{g})CH(R_{g})C(=O)- bedeuten; wobei jedes R_{g} unabhängig voneinander Wasserstoff, C₁₋₇-Alkyl, Aryl, (Aryl)C₁₋₇-alkyl, (Aryl)oxy, Heteroaryl(oxy) oder (Aryl)oxy-C₁₋₇-alkyl bedeutet.

24. Verbindung nach Anspruch 1, worin X und Y zusammen - CH(R_{g})CH(R_{g})-, -CH(R_{g})CH(R_{g})CH(R_{g})-, -C(R_{g})=C(R_{g})CH(R_{g})-, -O-CH(R_{g})CH(R_{g})-, -S-CH(R_{g})CH(R_{g})-, -S(O)-CH(R_{g})CH(R_{g})-, - S(O)₂CH(R_{g})CH(R_{g})-, -NR_{f}-CH(R_{g})CH(R_{g})-, -CH(R_{g})C(=O)-, -CH(R_{g})CH(R_{g})C(=O)-, -CH(R_{g})OC(=O)-, -OCH(R_{g})C(=O)- bedeuten; wobei jedes R_{g} unabhängig voneinander Wasserstoff oder C₁₋₇-Alkyl bedeutet.

25. Verbindung nach Anspruch 1, worin X und Y zusammen -CH(R_{g})CH₂-, -CH(R_{g})CH(R_{g})CH₂- oder -O-CH₂CH₂- bedeuten, worin jedes R_{g} unabhängig voneinander -NR_{q}Rᵣ, -S(O)ₚRₛ, -ORₜ, C₁₋₇-Alkyl, (C₁₋₇-Alkoxy)C₁₋₇-alkyl, Aryl, (Aryl)C₁₋₇-alkyl, Heteroaryl, (Heteroaryl)C₁₋₇-alkyl und (Aryl)oxy-C₁₋₇-alkyl bedeutet.

26. Verbindung nach Anspruch 1, worin X und Y zusammen -C(=O)CH₂-, -CH₂C(=O)-, -C(=S)CH₂-, -CH₂C(=S)-, -C(=O)CH₂CH₂-, -CH₂C(=O)CH₂-, -CH₂CH₂C(=O)-, -C(=S) CH₂CH₂-, -CH₂C(=S)CH₂- oder -CH₂CH₂-C(=S)- bedeuten.

27. Verbindung nach Anspruch 1, worin X und Y zusammen -CH(R_{g})CH(R_{g})-, -CH(R_{g})CH(R_{g})CH(R_{g})-, -C(R_{g})=C(R_{g})CH(R_{g})-, - O-CH(R_{g})CH(R_{g})-, -S-CH(R_{g})CH(R_{g})-, -S(O)-CH(R_{g})CH(R_{g})-, - S(O)₂CH(R_{g})CH(R_{g})-, -NR_{f}-CH(R_{g})CH(R_{g})- oder - CH(R_{g})CH(R_{g})C(=O)- bedeuten; wobei jedes R_{g} unabhängig voneinander Wasserstoff, C₁₋₇-Alkyl bedeutet oder zusammen mit einem Rg an einem benachbarten Kohlenstoffatom einen kondensierten 4-, 5- oder 6-gliedrigen carbocyclischen Ring bildet.

28. Verbindung nach Anspruch 1, worin X und Y zusammen - CH(R_{g})CH(R_{g})-, -CH(R_{g})CH(R_{g})CH(R_{g})-, -O-CH(R_{g})CH(R_{g})-, - S-CH(R_{g})CH(R_{g})- bedeuten; wobei jedes R_{g} unabhängig voneinander Wasserstoff, C₁₋₇-Alkyl, Aryl oder (Aryl)C₁₋₇-alkyl bedeutet.

29. Verbindung nach Anspruch 1, worin X und Y zusammen -CH₂CH₂CH₂-, -CH₂CH₂C(CH₃)H-, -CH₂C(CH₃)HCH₂-, -C(CH₃)HCH₂CH₂-, -CH₂CH₂-, -CH₂C(CH₃)H-, -C(CH₃)HC(CH₃)H-, -CH(R_{g})CH(R_{g})-, - O-CH₂CH₂-, -O-C(CH₃)HCH₂- oder -S-CH₂CH₂- bedeuten.

30. Verbindung nach Anspruch 1, worin X und Y zusammen -CH₂CH₂-, -CH₂CH₂CH₂-, -CH=CHCH₂-, -O-CH₂CH₂-, -S-CH₂CH₂-, -S(O)-CH₂CH₂-, -S(O)₂-CH₂CH₂-, -NR_{f}-CH₂CH₂-, -CH₂C(=O)-, - CH₂CH₂C(=O)-, -CH₂OC(=O)-, -OCH₂C(=O)- bedeuten.

31. Verbindung nach einem der Ansprüche 1 bis 30, worin R₂ Wasserstoff bedeutet.

32. Verbindung nach Anspruch 1, nämlich
5,6,9,10,11,12-Hexahydro-4H,8H-azepino[4',5':4,5]pyrrolo[3,2,1-ij]chinolin;
2-Methyl-5,6,9,10,11,12-hexahydro-4H,8H-azepino[4',5':4,5]-pyrrolo[3,2,1-ij]chinolin;
1-Methoxy-5,6,9,10,11,12-hexahydro-4H,8H-azepino[4',5':4,5]pyrrolo[3,2,1-ij]chinolin;
2-Fluoro-5,6,9,10,11,12-hexahydro-4H,8H-azepino[4',5':4,5]-pyrrolo[3,2,1-ij]chinolin;
6-Methyl-5,6,9,20,11,12-hexahydro-4H,8H-azepino[4',5':4,5]-pyrrolo[3,2,1-ij]chinolin;
5-Methyl-5,6,9,10,11,12-hexahydro-4H,8H-azepino[4',5':4,5]-pyrrolo[3,2,1-ij]chinolin;
4-Methyl-5,6,9,10,11,12-hexahydro-4H,8H-azepino[4',5':4,5]-pyrrolo[3,2,1-ij]chinolin;
(+)-6-Methyl-5,6,9,10,11,12-hexahydro-4H,8H-azepino[4',5':4,5]pyrrolo[3,2,1-ij]chinolin;
(-)-6-Methyl-5,6,9,10,11,12-hexahydro-4H,8H-azepino[4',5':4,5]pyrrolo[3,2,1-ij]chinolin;
(+)-5-Methyl-5,6,9,10,11,12-hexahydro-4H,8H-azepino[4',5':4,5]pyrrolo[3,2,1-ij]chinolin;
(-)-5-Methyl-5,6,9,10,11,12-hexahydro-4H,8H-azepino[4',5':4,5]pyrrolo[3,2,1-ij]chinolin;
(+)-4-Methyl-5,6,9,10,11,12-hexahydro-4H,8H-azepino[4',5':4,5]pyrrolo[3,2,1-ij]chinolin;
(-)-4-Methyl-5,6,9,10,11,12-hexahydro-4H,8H-azepino[4',5':4,5]pyrrolo[3,2,1-ij]chinolin;
4,5,8,9,10,11-Hexahydro-7H-azepino[4,5-b]pyrrolo[3,2,1-hi]-indol;
2-Fluor-4,5,8,9,10,11-hexahydro-7H-azepino[9,5-b]pyrrolo[3,2,1-hi]indol;
2-Methoxy-4,5,8,9,10,11-hexahydro-7H-azepino[4,5-b]pyrrolo[3,2,1-hi]indol;
5-Methyl-4,5,8,9,10,11-hexahydro-7H-azepino[4,5-b]pyrrolo[3,2,1-hi]indol;
4,5-Dimethyl-4,5,8,9,10,11-hexahydro-7H-azepino[4,5-b]pyrrolo[3,2,1-hi]indol;
2,3,4,5,8b,9,10,11,12,12a-Decahydro-1H-azepino[4',5':4,5]-pyrrolo[3,2,1-jk]carbazol;
6-Methyl-1,2,8,9,10,11-hexahydro-7H-azepino[4,5-b] [1,4]-oxazino[2,3,4-hi]indol;
1,2,8,9,10,11-Hexahydro-7H-azepino[4,5-b] [1,4]oxazino[2,3,4-hi]indol;
6-Chlor-1,2,8,9,10,11-hexahydro-7H-azepino[4,5-b] [1,4]-oxazino[2,3,4-hi]indol;
5-Fluor-1,2,8,9,10,11-hexahydro-7H-azepino[4,5-b] [1,4]-oxazino[2,3,4-hi]indol;
5-Methyl-1,2,8,9,10,11-hexahydro-7H-azepino[4,5-b] [1,4]-oxazino[2,3,4-hi]indol;
6-Fluor-1,2,8,9,10,11-hexahydro-7H-azepino[4,5-b] [1,4]-oxazino[2,3,4-hi]indol;
2-Methyl-1,2,8,9,10,11-hexahydro-7H-azepino[4,5-b] [1,4]-oxazino[2,3,4-hi]indol;
1,2,8,9,10,11-Hexahydro-7H-azepino[4,5-b] [1,4]thiazino[2,3,4-hi]indol;
2,2-Dimethyl-1,2,8,9,10,11-hexahydro-7H-azepino[4,5-b] [1,4]oxazino[2,3,4-hi]indol;
4-Fluor-1,2,8,9,10,11-hexahydro-7H-azepino[4,5-b] [1,4]-oxazino[2,3,4-hi]indol;
4-Chlor-1,2,8,9,10,11-hexahydro-7H-azepino[4,5-b] [1,4]-oxazino[2,3,4-hi]indol;
5-Chlor-1,2,8,9,10,11-hexahydro-7H-azepino[4,5-b] [1,4]-oxazino[2,3,4-hi]indol;
6-(Trifluormethyl)-1,2,8,9,10,11-hexahydro-7H-azepino[4,5-b] [1,4]oxazino[2,3,4-hi]indol;
5,6-Difluor-1,2,8,9,10,11-hexahydro-7H-azepino[4,5-b] [1,4]-oxazino[2,3,4-hi]indol;
5-Chlor-6-fluor-1,2,8,9,10,11-hexahydro-7H-azepino[4,5-b] [1,4]oxazino[2,3,4-hi]indol;
6-Chlor-5-fluor-1,2,8,9,10,11-hexahydro-7H-azepino[4,5-b] [1,4]oxazino[2,3,4-hi]indol;
5,6-Dichlor-1,2,8,9,10,11-hexahydro-7H-azepino[4,5-b] [1,4]-oxazino[2,3,4-hi]indol;
4,6-Dichlor-1,2,8,9,10,11-hexahydro-7H-azepino[4,5-b] [1,4]-oxazino[2,3,4-hi]indol;
1-Chlor-2-fluor-4,5,8,9,10,11-hexahydro-7H-azepino[4,5-b]pyrrolo[3,2,1-hi]indol;
1-Chlor-4,5,8,9,10,11-hexahydro-7H-azepino[4,5-b]pyrrolo[3,2,1-hi]indol;
2-Chlor-4,5,8,9,10,11-hexahydro-7H-azepino[4,5-b]pyrrolo[3,2,1-hi]indol;
5,6,9,10,11,12-Hexahydro-4H,8H-azepino[4',5':4,5]pyrrolo[3,2,1-ij]chinolin-4-on;
2-Fluor-5,6,9,10,11,12-hexahydro-4H,8H-azepino[4',5':4,5]pyrrolo[3,2,1-ij]chinolin-4-on;
2-Chlor-5,6,9,10,11,12-hexahydro-4H,8H-azepino[4',5':4,5]pyrrolo[3,2,1-ij]chinolin-4-on;
6-Methyl-5,6,9,10,11,12-hexahydro-4H,8H-azepino[4',5':4,5]pyrrolo[3,2,1-ij]chinolin-4-on;
2-Fluor-6-methyl-5,6,9,10,11,12-hexahydro-4H,8H-azepino[4',5':4,5]pyrrolo[3,2,1-ij]chinolin-4-on;
2,3-Dichlor-6-methyl-5,6,9,10,11,12-hexahydro-4H,8Hazepino[4',5':4,5]pyrrolo[3,2,1-ij]chinolin-4-on;
6-Propyl-5,6,9,10,11,12-hexahydro-4H,8H-azepino[4',5':4,5]pyrrolo[3,2,1-ij]chinolin-4-on;
6-(Trifluormethyl)-5,6,9,10,11,12-hexahydro-4H,8H-azepino[4',5':4,5]pyrrolo[3,2,1-ij]chinolin-4-on;
5,6,8,9,10,11,12,12a-Octahydro-4H,7aH-azepino[4',5':4,5]pyrrolo[3,2,1-ij]chinolin-4-ol;
4-Methoxy-5,6,8,9,10,11,12,12a-octahydro-4H,7aH-azepino[4',5':4,5]pyrrolo[3,2,1-ij]chinolin;
10-Benzoyl-4-phenoxy-5,6,9,10,11,12-hexahydro-4H,8Hazepino[4',5':4,5]pyrrolo[3,2,1-ij]chinolin oder
4-Phenoxy-5,6,9,10,11,12-hexahydro-4H,8H-azepino[4',5':4,5]pyrrolo[3,2,1-ij]chinolin,
oder ein pharmazeutisch akzeptables Salz derselben.

33. Die Verbindung 1,2,8,9,10,11-Hexahydro-7H-azepino[4,5-b][1,4]oxazino[2,3,4-hi]indol oder ein pharmazeutisch akzeptables Salz derselben.

34. Verbindung nach Anspruch 1, nämlich:
4-Brom-1,2,8,9,10,11-hexahydro-7H-azepino[4,5-b] [1,4]-oxazino[2,3,4-hi]indol;
5-Brom-1,2,8,9,10,11-hexahydro-7H-azepino[4,5-b] [1,4]-oxazino[2,3,4-hi]indol;
6-Brom-1,2,8,9,10,11-hexahydro-7H-azepino[4,5-b] [1,4]-oxazino[2,3,4-hi]indol;
5,6-Dibrom-1,2,8,9,10,11-hexahydro-7H-azepino[4,5-b] [1,4]-oxazino[2,3,4-hi]indol;
4,6-Dibrom-1,2,8,9,10,11-hexahydro-7H-azepino[4,5-b] [1,4]-oxazino[2,3,4-hi]indol;
4-Methoxy-1,2,8,9,10,11-hexahydro-7H-azepino[4,5-b] [1,4]-oxazino[2,3,4-hi]indol;
5-Methoxy-1,2,8,9,10,11-hexahydro-7H-azepino[4,5-b] [1,4]-oxazino[2,3,4-hi]indol;
6-Methoxy-1,2,8,9,10,11-hexahydro-7H-azepino[4,5-b] [1,4]-oxazino[2,3,4-hi]indol;
4-(Trifluormethyl)-1,2,8,9,10,11-hexahydro-7H-azepino[4,5-b] [1,4]oxazino[2,3,4-hi]indol;
5-(Trifluormethyl)-1,2,8,9,10,11-hexahydro-7H-azepino[4,5-b] [1,4]oxazino[2,3,4-hi]indol;
4-Benzyloxy-1,2,8,9,10,11-hexahydro-7H-azepino[4,5-b] [1,4]-oxazino[2,3,4-hi]indol;
5-Benzyloxy-1,2,8,9,10,11-hexahydro-7H-azepino[4,5-b] [1,4]-oxazino[2,3,4-hi]indol;
6-Benzyloxy-1,2,8,9,10,11-hexahydro-7H-azepino[4,5-b] [1,4]-oxazino[2,3,4-hi] indol;
4-Fluor-1, 2, 8, 9, 10,11-hexahydro-7H-azepino[4,5-b] [1,4]-thiazino[2,3,4-hi]indol;
5-Fluor-1,2,8,9,10,11-hexahydro-7H-azepino[4,5-b] [1,4]-thiazino[2,3,4-hi]indol;
6-Fluor-1,2,8,9,10,11-hexahydro-7H-azepino[4,5-b] [1,4]-thiazino[2,3,4-hi]indol;
4-Chlor-1,2,8,9,10,11-hexahydro-7H-azepino[4,5-b] [1,4]-thiazino[2,3,4-hi]indol;
5-Chlor-1,2,8,9,10,11-hexahydro-7H-azepino[4,5-b] [1,4]-thiazino[2,3,4-hi]indol;
6-Chlor-1,2,8,9,10,11-hexahydro-7H-azepino[4,5-b] [1,4]-thiazino[2,3,4-hi]indol;
4,5-Difluor-1,2,8,9,10,11-hexahydro-7H-azepino[4,5-b] [1,4]-thiazino[2,3,4-hi]indol;
5,6-Difluor-1,2,8,9,10,11-hexahydro-7H-azepino[4,5-b] [1,4]-thiazino[2,3,4-hi]indol;
4,6-Difluor-1,2,8,9,10,11-hexahydro-7H-azepino[4,5-b] [1,4]-thiazino[2,3,4-hi]indol;
4-Chlor-5-fluor-1,2,8,9,10,11-hexahydro-7H-azepino[4,5-b] [1,4]thiazino[2,3,4-hi]indol;
4-Chlor-6-fluor-1,2,8,9,10,11-hexahydro-7H-azepino[4,5-b] [1,4]thiazino[2,3,4-hi]indol;
5-Chlor-6-fluor-1,2,8,9,10,11-hexahydro-7H-azepino[4,5-b] [1,4]thiazino[2,3,4-hi]indol;
6-Chlor-5-fluor-1,2,8,9,10,11-hexahydro-7H-azepino[4,5-b] [1,4]thiazino[2,3,4-hi]indol;
4-Methyl-1,2,8,9,10,11-hexahydro-7H-azepino[4,5-b] [1,4]-thiazino[2,3,4-hi]indol;
5-Methyl-1,2,8,9,10,11-hexahydro-7H-azepino[4,5-b] [1,4]-thiazino[2,3,4-hi]indol;
6-Methyl-1,2,8,9,10,11-hexahydro-7H-azepino[4,5-b] [1,4]-thiazino[2,3,4-hi]indol;
4-Methoxy-1,2,8,9,10,11-hexahydro-7H-azepino[4,5-b] [1,4]-thiazino[2,3,4-hi]indol;
5-Methoxy-1,2,8,9,10,11-hexahydro-7H-azepino[4,5-b] [1,4]-thiazino[2,3,4-hi]indol;
6-Methoxy-1,2,8,9,10,11-hexahydro-7H-azepino[4,5-b] [1,4]-thiazino[2,3,4-hi]indol;
4-(Trifluormethyl)-1,2,8,9,10,11-hexahydro-7H-azepino[4,5-b] [1,4]thiazino[2,3,4-hi]indol;
5-(Trifluormethyl)-1,2,8,9,10,11-hexahydro-7H-azepino[4,5-b] [1,4]thiazino[2,3,4-hi]indol;
6-(Trifluormethyl)-1,2,8,9,10,11-hexahydro-7H-azepino[4,5-b] [1,4]thiazino[2,3,4-hi]indol;
4-Benzyloxy-1,2,8,9,10,11-hexahydro-7H-azepino[4,5-b] [1,4]-thiazino[2,3,4-hi]indol;
5-Benzyloxy-1,2,8,9,10,11-hexahydro-7H-azepino[4,5-b] [1,4]-thiazino[2,3,4-hi]indol;
6-Benzyloxy-1,2,8,9,10,11-hexahydro-7H-azepino[4,5-b] [1,4]-thiazino[2,3,4-hi]indol;
1-Fluor-4,5,8,9,10,11-hexahydro-7H-azepino[4,5-b]pyrrolo[3,2,1-hi]indol;
3-Fluor-4,5,8,9,10,11-hexahydro-7H-azepino[4,5-b]pyrrolo[3,2,1-hi]indol;
1-Brom-4,5,8,9,10,11-hexahydro-7H-azepino[4,5-b]pyrrolo[3,2,1-hi]indol;
2-Brom-4,5,8,9,10,11-hexahydro-7H-azepino[4,5-b]pyrrolo[3,2,1-hi]indol;
3-Brom-4,5,8,9,10,11-hexahydro-7H-azepino[4,5-b]pyrrolo[3,2,1-hi]indol;
3-Chlor-1-4,5,8,9,10,11-hexahydro-7H-azepino[4,5-b]pyrrolo[3,2,1-hi]indol;
2-Chlor-2-fluor-4,5,8,9,10,11-hexahydro-7H-azepino[4,5-b]-pyrrolo[3,2,1-hi]indol;
1-Methoxy-4,5,8,9,10,11-hexahydro-7H-azepino[4,5-b]pyrrolo[3,2,1-hi]indol;
3-Methoxy-4,5,8,9,10,11-hexahydro-7H-azepino[4,5-b]pyrrolo[3,2,1-hi]indol;
4-Methyl-4,5,8,9,10,11-hexahydro-7H-azepino[4,5-b]pyrrolo[3,2,1-hi]indol;
1-(Trifluormethyl)-4,5,8,9,10,11-hexahydro-7H-azepino[4,5-b]pyrrolo[3,2,1-hi]indol;
2-(Trifluormethyl)-4,5,8,9,10,11-hexahydro-7H-azepino[4,5-b]pyrrolo[3,2,1-hi]indol;
3-(Trifluormethyl)-4,5,8,9,10,11-hexahydro-7H-azepino[4,5-b]pyrrolo[3,2,1-hi]indol;
4-Benzyloxy-5,6,8,9,10,11,12,12a-octahydro-4H,7aHazepino[4',5':4,5]pyrrolo[3,2,1-ij]chinolin;
4-(3-Chlorphenoxy)-5,6,9,10,11,12-hexahydro-4H,8Hazepino[4',5':4,5]pyrrolo[3,2,1-ij]chinolin;
4-(2-Chlorphenoxy)-5,6,9,10,11,12-hexahydro-4H,8Hazepino[4',5':4,5]pyrrolo[3,2,1-ij]chinolin;
4-(4-Methoxyphenoxy)-5,6,9,10,11,12-hexahydro-4H,8Hazepino[4',5':4,5]pyrrolo[3,2,1-ij]chinolin;
4-(3-Methoxyphenoxy)-5,6,9,10,11,12-hexahydro-4H,8Hazepino[4',5':4,5]pyrrolo[3,2,1-ij]chinolin;
4-(2-Methoxyphenoxy)-5,6,9,10,11,12-hexahydro-4H,8Hazepino[4',5':4,5]pyrrolo[3,2,1-ij]chinolin;
4-(4-Brom-2-methoxyphenoxy)-5,6,9,10,11,12-hexahydro-4H,8Hazepino[4',5':4,5]pyrrolo[3,2,1-ij]chinolin;
5,6,9,10,11,12-Hexahydro-4H,8H-azepino[4',5':4,5]pyrrolo[3,2,1-ij]chinolin-4-amin;
N-Phenyl-5,6,9,10,11,12-hexahydro-4H,8H-azepino[4',5':4,5]-pyrrolo[3,2,1-ij]chinolin-4-amin;
N-(4-Chlorphenyl)-5,6,9,10,11,12-hexahydro-4H,8H-azepino[4',5':4,5]pyrrolo[3,2,1-ij]chinolin-4-amin;
N-(3-Chlorphenyl)-5,6,9,10,11,12-hexahydro-4H,8H-azepino[4',5':4,5]pyrrolo[3,2,1-ij]chinolin-4-amin;
N-(2-Chlorphenyl)-5,6,9,10,11,12-hexahydro-4H,8H-azepino[4',5':4,5]pyrrolo[3,2,1-ij]chinolin-4-amin;
N-(4-Methoxyphenyl)-5,6,9,10,11,12-hexahydro-4H,8Hazepino[4',5':4,5]pyrrolo[3,2,1-ij]chinolin-4-amin;
N-(3-Methoxyphenyl)-5,6,9,10,11,12-hexahydro-4H,8Hazepino[4',5':4,5]pyrrolo[3,2,1-ij]chinolin-4-amin;
N-(2-Methoxyphenyl)-5,6,9,10,11,12-hexahydro-4H,8Hazepino[4',5':4,5]pyrrolo[3,2,1-ij]chinolin-4-amin;
N-(4-Brom-2-methoxyphenyl)-5,6,9,10,11,12-hexahydro-4H,8Hazepino[4',5':4,5]pyrrolo[3,2,1-ij]chinolin-4-amin;
5,6,9,10,11,12-Hexahydro-4H,8H-azepino[4',5':4,5]pyrrolo[3,2,1-ij]chinolin-4-thion;
4-(Phenylsulfonyl)-5,6,9,10,11,12-hexahydro-4H,8Hazepino[4',5':4,5]pyrrolo[3,2,1-ij]chinolin;
4-(4-Chlorphenylsulfonyl)-5,6,9,10,11,12-hexahydro-4H,8Hazepino[4',5':4,5]pyrrolo[3,2,1-ij]chinolin;
4-(3-Chlorphenylsulfonyl)-5,6,9,10,11,12-hexahydro-4H,8Hazepino[4',5':4,5]pyrrolo[3,2,1-ij]chinolin;
4-(2-Chlorphenylsulfonyl)-5,6,9,10,11,12-hexahydro-4H,8Hazepino[4',5':'4,5]pyrrolo[3,2,1-ij]chinolin;
4-(4-Methoxyphenylsulfonyl)-5,6,9,10,11,12-hexahydro-4H,8Hazepino[4',5':4,5]pyrrolo[3,2,1-ij]chinolin;
4-(3-Methoxyphenylsulfonyl)-5,6,9,10,11,12-hexahydro-4H,8Hazepino[4',5':4,5]pyrrolo[3,2,1-ij]chinolin;
4-(2-Methoxyphenylsulfonyl)-5,6,9,10,11,12-hexahydro-4H,8Hazepino[4',5':4,5]pyrrolo[3,2,1-ij]chinolin;
4-(4-Brom-2-methoxyphenylsulfonyl)-5,6,9,10,11,12-hexahydro-4H,8H-azepino[4',5':4,5]pyrrolo[3,2,1-ij]-chinolin;
5,6,8,9,10,11,12,12a-Octahydro-4H,7aH-azepino[4',5':4,5]-pyrrolo[3,2,1-ij]chinolin-5-ol;
5-Methoxy-5,6,8,9,10,11,12,12a-octahydro-4H,7aH-azepino[4',5':4,5]pyrrolo[3,2,1-ij]chinolin;
5-Benzyloxy-5,6,8,9,10,11,12,12a-octahydro-4H,7aHazepino[4',5':4,5]pyrrolo[3,2,1-ij]chinolin;
5-Phenoxy-5,6,9,10,11,12-hexahydro-4H,8H-azepino[4',5':4,5]pyrrolo[3,2,1-ij]chinolin;
5-(4-Chlorphenoxy)-5,6,9,10,11,12-hexahydro-4H,8H-azepino[4',5':4,5]pyrrolo[3,2,1-ij]chinolin;
5-(3-Chlorphenoxy)-5,6,9,10,11,12-hexahydro-4H,8H-azepino[4',5':4,5]pyrrolo[3,2,1-ij]chinolin;
5-(2-Chlorphenoxy)-5,6,9,10,11,12-hexahydro-4H,8H-azepino[4',5':4,5]pyrrolo[3,2,1-ij]chinolin;
5-(4-Methoxyphenoxy)-5,6,9,10,11,12-hexahydro-4H,8Hazepino[4',5':4,5]pyrrolo[3,2,1-ij]chinolin;
5-(3-Methoxyphenoxy)-5,6,9,10,11,12-hexahydro-4H,8Hazepino[4',5':4,5]pyrrolo[3,2,1-ij]chinolin;
5-(2-Methoxyphenoxy)-5,6,9,10,11,12-hexahydro-4H,8Hazepino[4',5':4,5]pyrrolo[3,2,1-ij]chinolin;
5-(4-Brom-2-methoxyphenoxy)-5,6,9,10,11,12-hexahydro-4H,8Hazepino[4',5':4,5]pyrrolo[3,2,1-ij]chinolin;
5,6,9,10,11,12-Hexahydro-4H,8H-azepino[4',5':4,5]pyrrolo[3,2,1-ij]chinolin-5-amin;
N-Phenyl-5,6,9,20,11,12-hexahydro-4H,8H-azepino[4',5':4,5]-pyrrolo[3,2,1-ij]chinolin-5-amin;
N-(4-Chlorphenyl)-5,6,9,10,11,12-hexahydro-4H,8H-azepino[4',5':4,5]pyrrolo[3,2,1-ij]chinolin-5-amin;
N-(3-Chlorphenyl)-5,6,9,10,11,12-hexahydro-4H,8H-azepino[4',5':4,5]pyrrolo[3,2,1-ij]chinolin-5-amin;
N-(2-Chlorphenyl)-5,6,9,10,11,12-hexahydro-4H,8H-azepino[4',5':4,5]pyrrolo[3,2,1-ij]chinolin-5-amin;
N-(4-Methoxyphenyl)-5,6,9,10,11,12-hexahydro-4H,8H-azepino[4',5':4,5]pyrrolo[3,2,1-ij]chinolin-5-amin;
N-(3-Methoxyphenyl)-5,6,9,10,11,12-hexahydro-4H,8H-azepino[4',5':4,5]pyrrolo[3,2,1-ij]chinolin-5-amin;
N-(2-Methoxyphenyl)-5,6,9,10,11,12-hexahydro-4H,8H-azepino[4',5':4,5]pyrrolo[3,2,1-ij]chinolin-5-amin;
N-(4-Brom-2-methoxyphenyl)-5,6,9,10,11,12-hexahydro-4H,8Hazepino[4',5':4,5]pyrrolo[3,2,1-ij]chinolin-5-amin;
5,6,9,10,11,12-Hexahydro-4H,8H-azepino[4',5':4,5]pyrrolo[3,2,1-ij]chinolin-5-thion;
5-(Phenylsulfonyl)-5,6,9,10,11,12-hexahydro-4H,8H-azepino[4',5':4,5]pyrrolo[3,2,1-ij]chinolin;
5-(4-Chlorphenylsulfonyl)-5,6,9,10,11,12-hexahydro-4H,8Hazepino[4',5':4,5]pyrrolo[3,2,1-ij]chinolin;
5-(3-Chlorphenylsulfonyl)-5,6,9,10,11,12-hexahydro-4H,8Hazepino[4',5':4,5]pyrrolo[3,2,1-ij]chinolin;
5-(2-Chlorphenylsulfonyl)-5,6,9,10,11,12-hexahydro-4H,8Hazepino[4',5':4,5]pyrrolo[3,2,1-ij]chinolin;
5-(4-Methoxyphenylsulfonyl)-5,6,9,10,11,12-hexahydro-4H,8Hazepino[4',5':4,5]pyrrolo[3,2,1-ij]chinolin;
5-(3-Methoxyphenylsulfonyl)-5,6,9,10,11,12-hexahydro-4H,8Hazepino[4',5':4,5]pyrrolo[3,2,1-ij]chinolin;
5-(2-Methoxyphenylsulfonyl)-5,6,9,10,11,12-hexahydro-4H,8Hazepino[4',5':4,5]pyrrolo[3,2,1-ij]chinolin;
5-(4-Brom-2-methoxyphenylsulfonyl)-5,6,9,10,11,12-hexahydro-4H,8H-azepino[4',5':4,5]pyrrolo[3,2,1-ij]-chinolin;
4-(4-Chlorphenoxy)-5,6,9,10,11,12-hexahydro-4H,8H-azepino[4',5':4,5]pyrrolo[3,2,1-ij]chinolin;
1-[2-(4-Fluorphenoxy)ethoxy]-5,6,9,10,11,12-hexahydro-4H,8H-azepino[4',5':4,5]pyrrolo[3,2,1-ij]chinolin;
1-[2-(3-Fluorphenoxy)ethoxy]-5,6, 9,10,11,12-hexahydro-4H,8H-azepino[4',5':4,5]pyrrolo[3,2,1-ij]chinolin;
1-[2-(2-Fluorphenoxy)ethoxy]-5,6,9,10,11,12-hexahydro-4H,8H-azepino[4',5':4,5]pyrrolo[3,2,1-ij]chinolin;
1-[2-(4-Chlorphenoxy)ethoxy]-5,6,9,10,11,12-hexahydro-4H,8H-azepino[4',5':4,5]pyrrolo[3,2,1-ij]chinolin;
1-[2-(3-Chlorphenoxy)ethoxy]-5,6,9,10,11,12-hexahydro-4H,8H-azepino[4',5':4,5]pyrrolo[3,2,1-ij]chinolin;
1-[2-(2-Chlorphenoxy)ethoxy]-5,6,9,10,11,12-hexahydro-4H,8H-azepino[4',5':9,5]pyrrolo[3,2,1-ij]chinolin;
1-[2-(4-Bromphenoxy)ethoxy]-5,6,9,10,11,12-hexahydro-4H,8Hazepino[4',5':4,5]pyrrolo[3,2,1-ij]chinolin;
1-[2-(3-Bromphenoxy)ethoxy]-5,6,9,10,11,12-hexahydro-4H,8Hazepino[4',5':4,5]pyrrolo[3,2,1-ij]chinolin;
1-[2-(2-Bromphenoxy)ethoxy]-5,6,9,10, 11,12-hexahydro-4H,8Hazepino[4',5':4,5]pyrrolo[3,2,1-ij]chinolin;
1-[2-(4-Methoxyphenoxy)ethoxy]-5,6,9,10,11,12-hexahydro-4H,8H-azepino[4',5':4,5]pyrrolo[3,2,1-ij]chinolin;
1-[2-(3-Methoxyphenoxy)ethoxy]-5,6,9,10,11,12-hexahydro-4H,8H-azepino[4',5':4,5]pyrrolo[3,2,1-ij]chinolin;
1-[2-(2-Methoxyphenoxy)ethoxy]-5,6,9,10,11,12-hexahydro-4H,8H-azepino[4',5':4,5]pyrrolo[3,2,1-ij]chinolin;
1-[2-(4-Methylphenoxy)ethoxy]-5,6,9,10,11,12-hexahydro-4H,8H-azepino[4',5':4,5]pyrrolo[3,2,1-ij]chinolin;
1-[2-(3-Methylphenoxy)ethoxy]-5,6,9,10,11,12-hexahydro-4H,8H-azepino[4',5':4,5]pyrrolo[3,2,1-ij]chinolin;
1-[2-(2-Methylphenoxy)ethoxy]-5,6,9,10,11,12-hexahydro-4H,8H-azepino[4',5':4,5]pyrrolo[3,2,1-ij]chinolin;
1-[2-(1-Naphthyloxy)ethoxy]-5,6,9,10,11,12-hexahydro-4H,8Hazepino[4',5':4,5]pyrrolo[3,2,1-ij]chinolin;
1-[2-(2-Naphthyloxy)ethoxy]-5,6,9,10,11,12-hexahydro-4H,8Hazepino[4',5':4,5]pyrrolo[3,2,1-ij]chinolin;
1-[2-([1,1'-Biphenyl]-4-yloxy)ethoxy]-5,6,9,10,11,12-hexahydro-4H,8H-azepino[4',5':4,5]pyrrolo[3,2,1-ij]-chinolin;
1-[2-([1,1'-Biphenyl]-3-yloxy)ethoxy]-5,6,9,10,11,12-hexahydro-4H,8H-azepino[4',5':4,5]pyrrolo[3,2,1-ij]-chinolin;
1-[2-([1,1'-Biphenyl]-2-yloxy)ethoxy]-5,6,9,10,11,12-hexahydro-4H,8H-azepino[4',5':4,5]pyrrolo[3,2,1-ij]-chinolin;
1-{2-[4-(Trifluormethoxy)phenoxy]ethoxy}-5,6,9,10,11,12-hexahydro-4H,8H-azepino[4',5':4,5]pyrrolo[3,2,1-ij]-chinolin;
1-{2-[3-(Trifluormethoxy)phenoxy]ethoxy}-5,6,9,10,11,12-hexahydro-4H,8H-azepino[4',5':4,5]pyrrolo[3,2,1-ij]-chinolin;
1-{2-[2-(Trifluormethoxy)phenoxy]ethoxy}-5,6,9,10,11,12-hexahydro-4H,8H-azepino[4',5':4,5]pyrrolo[3,2,1-ij]-chinolin;
1-{2-[4-(Trifluormethyl)phenoxy]ethoxy}-5,6,9,10,11,12-hexahydro-4H,8H-azepino[4',5':4,5]pyrrolo[3,2,1-ij]-chinolin;
1-{2-[3-(Trifluormethyl)phenoxy]ethoxy}-5,6,19,10,11,12-hexahydro-4H,8H-azepino[4',5':4,5]pyrrolo[3,2,1-ij]-chinolin;
1-{2-[2-(Trifluormethyl)phenoxy]ethoxy}-5,6,9,10,11,12-hexahydro-4H,8H-azepino[4',5':4,5]pyrrolo[3,2,1-ij]-chinolin;
oder ein pharmazeutisch akzeptables Salz derselben.

35. Pharmazeutische Zusammensetzung, die eine Verbindung nach einem der Ansprüche 31 bis 34 und ein pharmazeutisch akzeptables Streckmittel umfasst.

36. Verbindung nach einem der Ansprüche 31 bis 34 zur Verwendung bei der medizinischen Diagnose oder Therapie.

37. Verbindung nach Anspruch 36, wobei die Therapie die Behandlung einer Erkrankung oder Störung des Zentralnervensystems ist.

38. Verbindung nach Anspruch 37, wobei die Erkrankung oder Störung des Zentralnervensystems Fettsucht, Depression, Schizophrenie, eine schizoide Störung, eine schizoaffektive Störung, ein paranoides Syndrom, eine stressbezogene Erkrankung, Panikstörung, Phobie, obsessive Zwangsstörung, post-traumatisches Belastungssyndrom, eine Immundepression, ein belastungsinduziertes Problem mit dem Harnwege-, Magen-Darm- oder kardiovaskulärem System, neurodegenerative Störungen, Autismus, durch eine Chemotherapie induziertes Erbrechen, Hypertonie, Migräne, Kopfschmerzen, Clusterkopfschmerzen, sexuelle Dysfunktion, eine Suchtstörung und ein Entzugssyndrom, eine Anpassungsstörung, eine altersbedingte Lern- und mentale Störung, Anorexia nervosa, Apathie, eine Aufmerksamkeitdefizitstörung aufgrund allgemeiner medizinischer Zustände, eine Aufmerksamkeitsdefizithyperaktivitätsstörung, eine Verhaltensstörung, bipolare Störung, Bulimia nervosa, chronisches Erschöpfungssyndrom, eine Anpassungsstörung im Sozialverhalten, Cyclothymiestörung, Dysthymiestörung, Fibromyalgie und andere somatoforme Störungen, eine allgemeine Angststörung, eine Inhalationsstörung, eine Intoxikationsstörung, Bewegungsstörung, gegnerische Trotzstörung, periphere Neuropathie, post-traumatische Belastungsstörung, prämenstruelle Dysphoriestörung, psychotische Störung, affektive Psychose, jahreszeitlich bedingte affektive Störung, Schlafstörung, spezielle Entwicklungsstörung, Agitationsstörung, ein selektives Serotoninwiederaufnahmehemmungssyndrom oder eine Tic-Störung, sind.

39. Verbindung nach Anspruch 36, wobei die Therapie die Behandlung von Angst, Fettsucht, Depression oder einer stressbezogenen Erkrankung ist.

40. Verwendung einer Verbindung nach einem der Ansprüche 31 bis 34 zur Herstellung eines Medikaments zur Behandlung oder Prävention einer Erkrankung oder Störung des Zentralnervensystems.

41. Verwendung nach Anspruch 40, wobei die Erkrankung oder Störung des Zentralnervensystems Fettsucht, Depression, Schizophrenie, eine schizoide Störung, eine schizoaffektive Störung, ein paranoides Syndrom, eine stressbezogene Erkrankung, Panikstörung, Phobie, obsessive Zwangsstörung, post-traumatisches Belastungssyndrom, eine Immundepression, ein belastungsinduziertes Problem mit dem Harnwege-, Magen-Darm- oder kardiovaskulärem System, neurodegenerative Störungen, Autismus, durch eine Chemotherapie induziertes Erbrechen, Hypertonie, Migräne, Kopfschmerzen, Clusterkopfschmerzen, sexuelle Dysfunktion, eine Suchtstörung und ein Entzugssyndrom, eine Anpassungsstörung, eine altersbedingte Lern- und mentale Störung, Anorexia nervosa, Apathie, eine Aufmerksamkeitdefizitstörung aufgrund allgemeiner medizinischer Zustände, eine Aufmerksamkeitsdefizithyperaktivitätsstörung, eine Verhaltensstörung, bipolare Störung, Bulimia nervosa, chronisches Erschöpfungssyndrom, eine Anpassungsstörung im Sozialverhalten, Cyclothymiestörung, Dysthymiestörung, Fibromyalgie und andere somatoforme Störungen, eine allgemeine Angststörung, eine Inhalationsstörung, eine Intoxikationsstörung, Bewegungsstörung, gegnerische Trotzstörung, periphere Neuropathie, post-traumatische Belastungsstörung, prämenstruelle Dysphoriestörung, psychotische Störung, affektive Psychose, jahreszeitlich bedingte affektive Störung, Schlafstörung, spezielle Entwicklungsstörung, Agitationsstörung, ein selektives Serotoninwiederaufnahmehemmungssyndrom oder eine Tic-Störung sind.

42. Verwendung nach Anspruch 40, wobei die Erkrankung oder Störung des Zentralnervensystems Angst, Fettsucht, Depression oder eine stressbezogene Erkrankung ist.

43. Verwendung nach Anspruch 40, wobei die Erkrankung oder der Zustand Angst, Fettsucht, Depression, Schizophrenie, eine stressbezogene Erkrankung, eine Panikstörung, Phobie, obsessive Zwangsstörung, ein post-traumatisches Stresssyndrom, eine Immundepression, ein belastungsindiziertes Problem mit dem Magen-Darm- oder kardiovaskulären System oder eine sexuelle Dysfunktion ist.

44. Verbindung der Formel I: 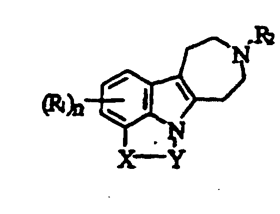 worin
jedes R₁ unabhängig voneinander Hydroxy, Nitro, Halogen, Cyano, Trifluormethyl, Trifluormethoxy, C₁₋₇-Alkyl, C₁₋₇-Alkoxy, C₁₋₇-Alkanoyl, C₁₋₇-Alkoxycarbonyl, C₁₋₇-Alkanoyloxy, Aryl, Heteroaryl, -S(O)ₘNRₐR_{b}, NR_{c}R_{d}, -S(O)ₘRₑ oder - C(=O)NRₐR_{b} bedeutet, wobei jedes C₁₋₇-Alkyl, C₁₋₇-Alkoxy, C₁₋₇-Alkanoyl, C₁₋₇-Alkoxycarbonyl oder C₁₋₇-Alkanoyloxy von R₁ optional partiell ungesättigt ist und optional mit Aryl, Aryloxy, Heteroaryl, Heteroaryloxy, Hydroxy, Nitro, Halogen, Cyano, C₁₋₇-Alkoxy, C₁₋₇-Alkanoyl, C₁₋₇-Alkoxycarbonyl, C₁₋₇-Alkanoyloxy, -S(O)ₘRₑ, -S(O)ₘNRₐR_{b}, NR_{c}R_{d} oder -C(=O)NRₐR_{b} substituiert ist;
R₂ eine geeignete Schutzgruppe ist;
X und Y zusammen eine 2-, 3- oder 4-gliedrige gesättigte oder partiell ungesättigte Kette bilden, die ein oder mehrere Kohlenstoffatome umfasst und optional ein Oxy (-O-), Thio (-S-), Sulfinyl (-SO-), Sulfonyl (S(O)₂-) oder NR_{f} in der Kette umfasst; wobei die Kette an jedem Kohlenstoff optional mit Oxo (=O), Thioxo (=S), -NR_{q}Rᵣ, -S(O)ₚRₛ oder-ORₜ oder mit einem oder zwei Substituenten, die unabhängig voneinander aus der aus C₁₋₇-Alkyl, (C₁₋₇-Alkoxy) C₁₋₇-alkyl, Aryl, (Aryl)C₁₋₇-alkyl, Heteroaryl, (Heteroaryl)C₁₋₇-alkyl und (Aryl)oxy-C₁₋₇-alkyl bestehenden Gruppe ausgewählt sind, substituiert ist; oder wobei die Kette optional an einem Kohlenstoff mit einem 4-, 5- oder 6-gliedrigen spirocyclischen Kohlenstoffring substituiert ist; oder wobei die Kette optional an zwei benachbarten Atomen mit einer 2-, 3-oder 4-gliedrigen Alkylenkette (beispielsweise -CH₂CH₂-, - CH₂CH₂CH₂- oder -CH₂CH₂CH₂CH₂-) unter Bildung eines Rings, der an den X und Y umfassenden Ring kondensiert ist, substituiert ist;
jedes m unabhängig voneinander 0, 1 oder 2 bedeutet;
n 0, 1, 2 oder 3 bedeutet;
p 0, 1 oder 2 bedeutet;
jedes Rₐ und R_{b} unabhängig voneinander Wasserstoff, C₁₋₇-Alkyl, Aryl, (Aryl)C₁₋₇-alkyl, Heteroaryl oder (Heteroaryl)-C₁₋₇-alkyl bedeuten; oder Rₐ und R_{b} zusammen mit dem Stickstoff, an den sie gebunden sind, einen Pyrrolidino-, Piperidino-, Morpholino- oder Thiomorpholinoring bilden;
jedes R_{c} und R_{d} unabhängig voneinander Wasserstoff, C₁₋₇-Alkyl, C₁₋₇-Alkanoyl, C₁₋₇-Alkoxycarbonyl, Aryl, (Aryl)C₁₋₇alkyl, Heteroaryl, (Heteroaryl)C₁₋₇-alkyl, Arylcarbonyl, Heteroarylcarbonyl, Aryloxycarbonyl oder Heteroaryloxycarbonyl bedeuten; oder R_{c} und R_{d} zusammen mit dem Stickstoff, an den sie gebunden sind, einen Pyrrolidino-, Piperidino-, Morpholino- oder Thiomorpholinoring bilden;
jedes Rₑ unabhängig voneinander Wasserstoff, C₁₋₇-Alkyl, Aryl, (Aryl)C₁₋₇-alkyl, Heteroaryl oder (Heteroaryl)C₁₋₇alkyl bedeutet;
R_{f} Wasserstoff, C₁₋₇-Alkyl, Aryl, (Aryl)C₁₋₇-alkyl, Heteroaryl, (Heteroaryl)C₁₋₇-alkyl oder eine Bindung zu einer 2-, 3- oder 4-gliedrigen Alkylenkette, die einen Ring, der an den X- und Y umfassenden Ring kondensiert ist, bildet, bedeutet;
jedes R_{q} und Rᵣ unabhängig voneinander Wasserstoff, C₁₋₇-Alkyl, Aryl, (Aryl)C₁₋₇-alkyl, Heteroaryl oder (Heteroaryl)-C₁₋₇-alkyl bedeuten; oder R_{q} und Rₜ zusammen mit dem Stickstoff, an den sie gebunden sind, einen Pyrrolidino-, Piperidino-, Morpholino- oder Thiomorpholinoring bilden;
Rₛ C₁₋₇-Alkyl, Aryl, (Aryl)C₁₋₇-alkyl, Heteroaryl oder (Heteroaryl)C₁₋₇-alkyl bedeutet; und
Rₜ Wasserstoff, C₁₋₇-Alkyl, Aryl, (Aryl)C₁₋₇-alkyl, Heteroaryl oder (Heteroaryl)C₁₋₇-alkyl bedeutet;
wobei jeder Aryl- oder Heteroarylring von R₁, R₂, X, Y, Rₐ-R_{f} oder R_{q}-Rₜ optional mit einem oder mehreren (beispielsweise 1, 2, 3 oder 4) Substituenten substituiert ist, die unabhängig voneinander aus Halogen, Hydroxy, Cyano, Nitro, Trifluormethyl, Trifluormethoxy, C₁₋₇-Alkyl, C₁₋₇-Alkoxy, Phenyl, Sulfonyl, NRⱼRₖ oder -C(=O)NRⱼRₖ ausgewählt sind; wobei jedes Rⱼ und Rₖ unabhängig voneinander Wasserstoff, C₁₋₇-Alkyl, C₁₋₇-Alkanoyl, C₁₋₇-Alkoxycarbonyl, Aryl, (Aryl)C₁₋₇-alkyl, Arylcarbonyl oder Aryloxycarbonyl bedeuten; oder Rⱼ und Rₖ zusammen mit dem Stickstoff, an den sie gebunden sind, einen Pyrrolidino-, Piperidino-, Morpholinooder Thiomorpholinoring bilden;
oder ein pharmazeutisch akzeptables Salz derselben;
mit dem Vorbehalt, dass Y nicht Oxy, Thio, Sulfinyl oder NR_{f} bedeutet; und
mit dem Vorbehalt, dass X und Y zusammen keine zweigliedrige ungesättigte Kette sind; und
mit dem Vorbehalt, dass kein Kohlenstoff von X und Y an mehr als ein Oxy, Thio, Sulfinyl oder NR_{f} gebunden ist.

45. Verbindung nach Anspruch 1, worin R₂ C₁₋₄-Alkyl, C₁₋₄-Alkanoyl, Arylcarbonyl, (Aryl)C₁₋₂-alkyl, C₁₋₄-Alkoxycarbonyl, Aryloxycarbonyl, Arylsulfonyl oder (Aryl)methoxycarbonyl bedeutet, wobei jedes Aryl optional mit 1, 2 oder 3 Substituenten, die unabhängig voneinander aus C₁₋₄-Alkyl und Trifluormethyl ausgewählt sind, substituiert ist.

46. Verbindung nach Anspruch 1, worin R₂ Methyl, Ethyl, Propyl, Isopropyl, Acetyl, tert.-Butoxycarbonyl, Benzyloxycarbonyl, Benzyl oder p-Toluolsulfonyl bedeutet.

47. Verbindung nach Anspruch 1, nämlich:
10-Benzoyl-5,6,9,10,11,12-hexahydro-4H,8H-azepino[4',5':4,5]pyrrolo[3,2,1-ij]chinolin-4-on;
10-Benzoyl-2-fluor-5,6,9,10,11,12-hexahydro-4H,8H-azepino[4',5':4,5]pyrrolo[3,2,1-ij]chinolin-4-on;
10-Benzoyl-2-chlor-5,6,9,10,11,12-hexahydro-4H,8H-azepino[4',5':4,5]pyrrolo[3,2,1-ij]chinolin-4-on;
10-Benzoyl-6-methyl-5,6,9,10,11,12-hexahydro-4H,8H-azepino[4',5':4,5]pyrrolo[3,2,1-ij]chinolin-4-on;
10-Benzoyl-2-fluor-6-methyl-5,6,9,10,11,12-hexahydro-4H,8Hazepino[4',5':4,5]pyrrolo[3,2,1-ij]chinolin-4-on;
10-Benzoyl-2,3-dichlor-6-methyl-5,6,9,10,11,12-hexahydro-4H,8H-azepino[4',5':4,5]pyrrolo[3,2,1-ij]chinolin-4-on;
10-Benzoyl-6-propyl-5,6,9,10,11,12-hexahydro-4H,8H-azepino[4',5':4,5]pyrrolo[3,2,1-ij]chinolin-4-on;
10-Benzoyl-6-(trifluormethyl)-5,6,9,10,11,12-hexahydro-4H,8H-azepino[4',5':4,5]pyrrolo[3,2,1-ij]chinolin-4-on;
10-Benzoyl-5,6,8,9,20,11,12,12a-octahydro-4H,7aH-azepino[4',5':4,5]pyrrolo[3,2,1-ij]chinolin-4-ol oder
10-Benzoyl-4-methoxy-5,6,8,9,10,11,12,12a-octahydro-4H,7aHazepino[4',5':4,5]pyrrolo[3,2,1-ij]chinolin.

48. Verbindung nach Anspruch 1, nämlich:
Benzyl-5,6,8,9,11,12-hexahydro-4H,10H-azepino[4',5':4,5]-pyrrolo[3,2,1-ij]chinolin-10-carboxylat;
Benzyl-2-methyl-5,6,8,9,11,12-hexahydro-4H,10H-azepino[4',5':4,5]pyrrolo[3,2,1-ij]chinolin-10-carboxylat;
10-Benzoyl-1-methoxy-5,6,8,9,10,11,12-hexahydro-4H,8Hazepino[4',5':4,5]pyrrolo[3,2,1-ij]chinolin;
Benzyl-2-fluor-5,6,8,9,11,12-hexahydro-4H,10H-azepino[4',5':4,5]pyrrolo[3,2,1-ij]chinolin-10-carboxylat;
Benzyl-6-methyl-5,6,8,9,11,12-hexahydro-4H,10H-azepino[4',5':4,5]pyrrolo[3,2,1-ij]chinolin-10-carboxylat;
Benzyl-5-methyl-5,6,8,9,11,12-hexahydro-4H,10H-azepino[4',5':4,5]pyrrolo[3,2,1-ij]chinolin-10-carboxylat;
Benzyl-4-methyl-5,6,8,9,11,12-hexahydro-4H,10H-azepino[4',5':4,5]pyrrolo[3,2,1-ij]chinolin-10-carboxylat;
(+)-Benzyl-6-methyl-5,6,8,9,11,12-hexahydro-4H,10H-azepino[4',5':4,5]pyrrolo[3,2,1-ij]chinolin-10-carboxylat;
(-)-Benzyl-6-methyl-5,6,8,9,11,12-hexahydro-4H,10H-azepino[4',5':4,5]pyrrolo[3,2,1-ij]chinolin-10-carboxylat;
(+)-Benzyl-5-methyl-5,6,8,9,11,12-hexahydro-4H,10H-azepino[4',5':4,5]pyrrolo[3,2,1-ij]chinolin-10-carboxylat;
(-)-Benzyl-5-methyl-5,6,8,9,11,12-hexahydro-4H,10H-azepino[4',5':4,5]pyrrolo[3,2,1-ij]chinolin-10-carboxylat;
(+)-Benzyl-4-methyl-5,6,8,9,11,12-hexahydro-4H,10H-azepino[4',5':4,5]pyrrolo[3,2,1-ij]chinolin-10-carboxylat;
(-)-Benzyl-4-methyl-5,6,8,9,11,12-hexahydro-4H,10H-azepino[4',5':4,5]pyrrolo[3,2,1-ij]chinolin-10-carboxylat;
Benzyl-2-methyl-1,2,7,8,10,11-hexahydro-9H-azepino[4,5-b] [1,4]oxazino[2,3,4-hi]indol-9-carboxylat;
Benzyl-2,2-dimethyl-1,2,7,8,10,11-hexahydro-9H-azepino[4,5-b] [1,4]oxazino[2,3,4-hi]indol-9-carboxylat;
Benzyl-4-fluor-1,2,7,8,10,11-he xahydro-9H-azepino[4,5-b] [1,4]oxazino[2,3,4-hi]indol-9-carboxylat;
Benzyl-4-chlor-1,2,7,8,10,11-hexahydro-9H-azepino[4,5-b] [1,4]oxazino[2,3,4-hi]indol-9-carboxylat;
Benzyl-5-fluor-1,2,7,8,10,11-hexahydro-9H-azepino[4,5-b] [1,4]oxazino[2,3,4-hi]indol-9-carboxylat;
Benzyl-5-chlor-1,2,7,8,10,11-hexahydro-9H-azepino[4,5-b] [1,4]oxazino[2,3,4-hi]indol-9-carboxylat;
Benzyl-5-methyl-1,2,7,8,10,11-hexahydro-9H-azepino[4,5-b] [1,4]oxazino[2,3,4-hi]indol-9-carboxylat;
Benzyl-6-fluor-1,2,7,8,10,11-hexahydro-9H-azepino[4,5-b] [1,4]oxazino[2,3,4-hi]indol-9-carboxylat;
Benzyl-6-chlor-1,2,7,8,10,11-hexahydro-9H-azepino[4,5-b] [1,4]oxazino[2,3,4-hi]indol-9-carboxylat;
Benzyl-6-methyl-1,2,6b,7,8,10,11,11a-octahydro-9Hazepino[4,5-b] [1,4]oxazino[2,3,4-hi]indol-9-carboxylat;
Benzyl-6-(trifluormethyl)-1,2,7,8,10,11-hexahydro-9Hazepino[4,5-b] [1,4]oxazino[2,3,4-hi]indol-9-carboxylat;
Benzyl-5,6-difluor-1,2,7,8,10,11-hexahydro-9H-azepino[4,5-b] [1,4]oxazino[2,3,4-hi]indol-9-carboxylat;
Benzyl-5-chlor-6-fluor-1,2,7,8,10,11-hexahydro-9Hazepino[4,5-b] [1,4]oxazino[2,3,4-hi]indol-9-carboxylat;
Benzyl-5-fluor-6-chlor-1,2,7,8,10,11-hexahydro-9Hazepino[4,5-b] [1,4]oxazino[2,3,4-hi]indol-9-carboxylat;
Benzyl-5,6-dichlor-1,2,7,8,10,11-hexahydro-9H-azepino[4,5-b][1,4]oxazino[2,3,4-hi]indol-9-carboxylat;
Benzyl-4,6-dichlor-1,2,7,8,10,11-hexahydro-9H-azepino[4,5-b] [1,4]oxazino[2,3,4-hi]indol-9-carboxylat;
7-Benzoyl-4,5,8,9,10,11-hexahydro-7H-azepino[4,5-b]pyrrolo[3,2,1-hi]indol;
7-Benzoyl-2-fluor-4,5,8,9,10,11-hexahydro-7H-azepino[4,5-b]pyrrolo[3,2,1-hi]indol;
7-Benzoyl-2-methoxy-4,5,8,9,10,11-hexahydro-7H-azepino[4,5-b]pyrrolo[3,2,1-hi]indol;
Benzyl-5-methyl-4,5,8,9,10,11-hexahydro-7H-azepino[4,5-b]-pyrrolo[3,2,1-hi]-7-carboxylat;
Benzyl-4,5-dimethyl-4,5,8,9,10,11-hexahydro-7H-azepino[4,5-b]pyrrolo[3,2,1-hi]-7-carboxylat;
Benzyl-2,3,4,5,8b,9,10,11,12,12a-decahydro-1H-azepino[4',5':4,5]pyrrolo[3,2,1-jk]carbazol-7-carboxylat;
Benzyl-1-chlor-2-fluor-4,5,8,9,10,11-hexahydro-7H-azepino[4,5-b]pyrrolo[3,2,1-hi]indol-7-carboxylat;
Benzyl-2-chlor-4,5,8,9,10,11-hexahydro-7H-azepino[4,5-b]-pyrrolo[3,2,1-hi]indol-7-carboxylat;
Benzyl-1-chlor-4,5,8,9,10,11-hexahydro-7H-azepino[4,5-b]-pyrrolo[3,2,1-hi]indol-7-carboxylat;
10-Benzoyl-5,6,9,10,11,12-hexahydro-4H,8H-azepino[4',5':4,5]pyrrolo[3,2,1-ij]chinolin-4-on;
10-Benzoyl-2-fluor-5,6,9,10,11,12-hexahydro-4H,8H-azepino[4',5':4,5]pyrrolo[3,2,1-ij]chinolin-4-on;
10-Benzoyl-2-chlor-5,6,9,10,11,12-hexahydro-4H,8H-azepino[4',5':4,5]pyrrolo[3,2,1-ij]chinolin-4-on;
10-Benzoyl-6-methyl-5,6,9,10,11,12-hexahydro-4H,8H-azepino[4',5':4,5]pyrrolo[3,2,1-ij]chinolin-4-on;
10-Benzoyl-2-fluor-6-methyl-5,6,9,10,11,12-hexahydro-4H,8Hazepino[4',5':4,5]pyrrolo[3,2,1-ij]chinolin-4-on;
10-Benzoyl-2,3-dichlor-6-methyl-5,6,9,10,11,12-hexahydro-4H,8H-azepino[4',5':4,5]pyrrolo[3,2,1-ij]chinolin-4-on;
10-Benzoyl-6-propyl-5,6,9,10,11,12-hexahydro-4H,8H-azepino[4',5':4,5]pyrrolo[3,2,1-ij]chinolin-4-on;
10-Benzoyl-6-(trifluormethyl)-5,6,9,10,11,12-hexahydro-4H,8H-azepino[4',5':4,5]pyrrolo[3,2,1-ij]chinolin-4-on;
10-Benzoyl-5,6,8,9,10,11,12,12a-octahydro-4H,7aH-azepino[4',5':4,5]pyrrolo[3,2,1-ij]chinolin-4-ol und
10-Benzoyl-4-methoxy-5,6,8,9,10,11,12,12a-octahydro-4H,7aHazepino[4',5':4,5]pyrrolo[3,2,1-ij]chinolin.

49. Verfahren zur Herstellung einer Verbindung der Formel (I), worin R₂ Wasserstoff bedeutet, das das Entschützen einer entsprechenden Verbindung der Formel (I), worin R₂ eine Schutzgruppe ist, umfasst.

50. Verfahren nach Anspruch 49, wobei die Schutzgruppe Benzyloxycarbonyl oder Benzoyl ist.

## Revendications

1. Composé de formule I dans laquelle : 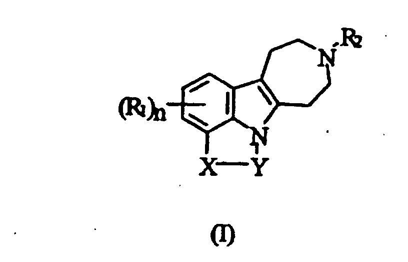 dans laquelle,
chaque groupe R₁ représente indépendamment un groupe hydroxy, nitro, halogéno, cyano, trifluorométhyle, trifluorométhoxy, alkyle en C₁ à C₇, alcoxy en C₁ à C₇, alcanoyle en C₁ à C₇, (alcoxy en C₁ à C₇)carbonyle, alcanoyloxy en C₁ à C₇, aryle, hétéroaryle, -S(O)ₘNRₐR_{b}, NR_{c}R_{d}, -S(O)ₘRₑ, ou -C(=O)NRₐR_{b}, dans lequel n'importe quel groupe alkyle en C₁ à C₇, alcoxy en C₁ à C₇, alcanoyle en C₁ à C₇, (alcoxy en C₁ à C₇)carbonyle ou alcanoyloxy en C₁ à C₇ de R₁ est facultativement partiellement insaturé et est facultativement substitué avec un substituant aryle, aryloxy, hétéroaryle, hétéroaryloxy, hydroxy, nitro, halogéno, cyano, alcoxy en C₁ à C₇, alcanoyle en C₁ à C₇, (alcoxy en C₁ à C₇)carbonyle, alcanoyloxy en C₁ à C₇, -S(O)ₘRₑ, -S(O)ₘNRₐR_{b}, NR_{c}R_{d} ou -C(=O)NRₐR_{b} ;
R₂ représente un atome d'hydrogène, un groupe alkyle en C₁ à C₇, alcanoyle en C₁ à C₇, arylcarbonyle, aryle, (aryl) (alkyle en C₁ à C₇), (alcoxy en C₁ à C₇)carbonyle, aryloxycarbonyle, arylsulfonyle ou (aryl)(alcoxy en C₁ à C₇)carbonyle ;
X et Y, conjointement, représentent une chaîne bi-, tri- ou tétragonale saturée ou partiellement insaturée comprenant un ou plusieurs atomes de carbone et comprenant facultativement un groupe oxy (-O-), thio (-S-), sulfinyle (-SO-), sulfonyle (S(O)₂-) ou NR_{f} dans la chaîne ; la chaîne étant facultativement substituée sur chaque atome de carbone avec un substituant oxo (=O), thioxo (=S), -NR_{q}Rᵣ, -S(O)ₚRₛ ou -ORₜ, ou avec un ou deux substituants choisis indépendamment dans le groupe consistant en des substituants alkyle en C₁ à C₇, (alcoxy en C₁ à C₇) (alkyle en C₁ à C₇), aryle, (aryl) (alkyle en C₁ à C₇), hétéroaryle, (hétéroaryl) (alkyle en C₁ à C₇) et (aryl) oxy (alkyle en C₁ à C₇) ; ou la chaîne étant facultativement substituée sur un atome de carbone avec un noyau carboné spirocyclique tétra-, penta- ou hexagonal ; ou la chaîne étant facultativement substituée sur deux atomes de carbone adjacents avec une chaîne alkylène de 2, 3 ou 4 membres (par exemple -CH₂CH₂-, -CH₂CH₂CH₂-, -CH₂CH₂CH₂CH₂-) formant un noyau qui est condensé avec le noyau comprenant X et Y ;
chaque indice m est indépendamment égal à 0, 1 ou 2 ;
n est égal à 0, 1, 2 ou 3 ;
p est égal à 0, 1 ou 2 ;
chacun des groupes Rₐ et R_{b} représente indépendamment un atome d'hydrogène, un groupe alkyle en C₁ à C₇, aryle, (aryl)(alkyle en C₁ à C₇), hétéroaryle ou (hétéroaryl) (alkyle en C₁ à C₇) ; ou bien Rₐ et R_{b}, conjointement avec l'atome d'azote auquel ils sont fixés, forment un noyau pyrrolidino, pipéridino, morpholino ou thiomorpholino ;
chacun des groupes R_{c} et R_{d} représente indépendamment un atome d'hydrogène, un groupe alkyle en C₁ à C₇, alcanoyle en C₁ à C₇, (alcoxy en C₁ à C₇)carbonyle, aryle, (aryl) (alkyle en C₁ à C₇), hétéroaryle, (hétéroaryl) (alkyle en C₁ à C₇), arylcarbonyle, hétéroarylcarbonyle, aryloxycarbonyle ou hétéroaryloxy-carbonyle ; ou bien R_{c} et R_{d}, conjointement avec l'atome d'azote auquel ils sont fixés, forment un noyau pyrrolidino, pipéridino, morpholino ou thiomorpholino ;
chaque groupe Rₑ représente indépendamment un atome d'hydrogène, un groupe alkyle en C₁ à C₇, aryle, (aryl) (alkyle en C₁ à C₇), hétéroaryle ou (hétéroaryl) (alkyle en C₁ à C₇) ;
R_{f} représente un atome d'hydrogène, un groupe alkyle en C₁ à C₇, aryle, (aryl) (alkyle en C₁ à C₇), hétéroaryle ou (hétéroaryl) (alkyle en C₁ à C₇) ; ou bien représente une liaison à une chaîne alkylène de 2, 3 ou 4 membres qui forme un noyau qui est condensé avec le noyau comprenant X et Y ;
chacun des groupes R_{q} et Rᵣ représente indépendamment un atome d'hydrogène, un groupe alkyle en C₁ à C₇, aryle, (aryl) (alkyle en C₁ à C₇), hétéroaryle ou (hétéroaryl) (alkyle en C₁ à C₇) ; ou bien R_{q} et Rᵣ, conjointement avec l'atome d'azote auquel ils sont fixés, forment un noyau pyrrolidino, pipéridino, morpholino ou thiomorpholino ;
Rₛ représente un groupe alkyle en C₁ à C₇, aryle, (aryl) (alkyle en C₁ à C₇), hétéroaryle ou (hétéroaryl) (alkyle en C₁ à C₇) ; et
Rₜ représente un atome d'hydrogène, un groupe alkyle en C₁ à C₇, aryle, (aryl)(alkyle en C₁ à C₇), hétéroaryle ou (hétéroaryl) (alkyle en C₁ à C₇) ;
n'importe quel noyau aryle ou hétéroaryle de R₁, R₂, X, Y, Rₐ-R_{f} ou R_{q}-Rₜ étant facultativement substitué avec un ou plusieurs (par exemple 1, 2, 3 ou 4) substituants choisis indépendamment entre des substituants halogéno, hydroxy, cyano, nitro, trifluorométhyle, trifluorométhoxy, alkyle en C₁ à C₇, alcoxy en C₁ à C₇, phényle, sulfonyle, NRⱼRₖ ou -C(=O)NRⱼRₖ ; dans lesquels chacun des groupes Rⱼ et Rₖ représente indépendamment un atome d'hydrogène, un groupe alkyle en C₁ à C₇, alcanoyle en C₁ à C₇, (alcoxy en C₁ à C₇)carbonyle, aryle, (aryl) (alkyle en C₁ à C₇), arylcarbonyle ou aryloxycarbonyle ; ou bien Rⱼ et Rₖ, conjointement avec l'atome d'azote auquel ils sont fixés, forment un noyau pyrrolidino, pipéridino, morpholino ou thiomorpholino ;
ou un de ses sels pharmaceutiquement acceptables ;
sous réserve que Y ne représente pas un groupe oxy, thio, sulfinyle ou NR_{f} ; et
sous réserve que X et Y, conjointement, ne représentent pas une chaîne insaturée de 2 membres ; et
sous réserve qu'aucun atome de carbone de X et Y ne soit lié à plus d'un groupe oxy, thio, sulfinyle ou NR_{f}.

2. Composé suivant la revendication 1, dans lequel chaque groupe R₁ représente indépendamment un groupe hydroxy, nitro, halogéno, cyano, trifluorométhyle, trifluorométhoxy, alkyle en C₁ à C₇, alcoxy en C₁ à C₇, alcanoyle en C₁ à C₇, (alcoxy en C₁ à C₇)carbonyle, alcanoyloxy en C₁ à C₇, aryle, hétéroaryle, -S(O)ₘNRₐR_{b}, NR_{c}R_{d}, - S(O)ₘRₑ, ou -C(=O)NRₐR_{b} dans lequel n'importe quel groupe alkyle en C₁ à C₇, alcoxy en C₁ à C₇, alcanoyle en C₁ à C₇, (alcoxy en C₁ à C₇)carbonyle ou alcanoyloxy en C₁ à C₇ de R₁ est facultativement substitué avec un substituant aryle, aryloxy, hétéroaryle, hétéroaryloxy, hydroxy, nitro, halogéno, cyano, alcoxy en C₁ à C₇, alcanoyle en C₁ à C₇, (alcoxy en C₁ à C₇)carbonyle, alcanoyloxy en C₁ à C₇, -S(O)ₘRₑ, -S(O)ₘNRₐR_{b}, NR_{c}R_{d} ou -C(=O)NRₐR_{b}.

3. Composé suivant la revendication 1, dans lequel chaque groupe R₁ représente indépendamment un groupe hydroxy, nitro, halogéno, cyano, trifluorométhyle, trifluorométhoxy, alkyle en C₁ à C₇, alcoxy en C₁ à C₇, alcanoyle en C₁ à C₇, (alcoxy en C₁ à C₇)carbonyle, alcanoyloxy en C₁ à C₇, aryle, hétéroaryle, -S(O)ₘNRₐR_{b}, NR_{c}R_{d}, - S(O)ₘRₑ, ou -C(=O)NRₐR_{b} dans lequel n'importe quel groupe alkyle en C₁ à C₇ ou alcoxy en C₁ à C₇ de R₁ est facultativement substitué avec un substituant aryle, aryloxy, hétéroaryle, hétéroaryloxy, hydroxy, nitro, halogéno, cyano, alcoxy en C₁ à C₇, alcanoyle en C₁ à C₇, (alcoxy en C₁ à C₇) carbonyle, alcanoyloxy en C₁ à C₇, -S(O)ₘRₑ, -S(O)ₘNRₐR_{b}, NR_{c}R_{d} ou -C(=O)NRₐR_{b}.

4. Composé suivant la revendication 1, dans lequel chaque groupe R₁ représente indépendamment un groupe hydroxy, nitro, halogéno, cyano, trifluorométhyle, trifluorométhoxy, alkyle en C₁ à C₇, alcoxy en C₁ à C₇, alcanoyle en C₁ à C₇, (alcoxy en C₁ à C₇)carbonyle, alcanoyloxy en C₁ à C₇, aryle, hétéroaryle, -S(O)ₘNRₐR_{b}, NR_{c}R_{d}, -S(=O)ₘRₑ ou -C(=O)NRₐR_{b}, dans lequel n'importe quel groupe alkyle en C₁ à C₇ est facultativement substitué avec un substituant aryle, aryloxy, hétéroaryle, hétéroaryloxy, hydroxy, nitro, halogéno, cyano, alcoxy en C₁ à C₇, alcanoyle en C₁ à C₇, (alcoxy en C₁ à C₇)carbonyle, alcanoyloxy en C₁ à C₇, -S(O)ₘRₑ, -S(O)ₘNRₐR_{b}, NR_{c}R_{d} ou -C(=O)NRₐR_{b}.

5. Composé suivant la revendication 1, dans lequel chaque groupe R₁ représente indépendamment un groupe hydroxy, nitro, halogéno, cyano, trifluorométhyle, trifluorométhoxy, alkyle en C₁ à C₇, alcoxy en C₁ à C₇, alcanoyle en C₁ à C₇, (alcoxy en C₁ à C₇)carbonyle, alcanoyloxy en C₁ à C₇, aryle, hétéroaryle, -S(O)ₘNRₐR_{b}, NR_{c}R_{d}, -S(O)ₘRₑ ou -C(=O)NRₐR_{b}.

6. Composé suivant la revendication 1, dans lequel chaque groupe R₁ représente indépendamment un groupe alkyle en C₁ à C₇, alcoxy en C₁ à C₇, trifluorométhyle ou halogéno.

7. Composé suivant la revendication 1, dans lequel n est égal à 1 et R₁ représente un groupe alkyle en C₁ à C₇, alcoxy en C₁ à C₇ ou halogéno.

8. Composé suivant la revendication 1, dans lequel n est égal à 1 et R₁ représente un groupe méthyle, méthoxy, chloro ou fluoro.

9. Composé suivant la revendication 1, dans lequel R₂ représente un atome d'hydrogène.

10. Composé suivant la revendication 1, dans lequel n est égal à 1, 2 ou 3.

11. Composé suivant la revendication 1, dans lequel n est égal à 0.

12. Composé suivant la revendication 1, dans lequel X et Y, conjointement, représentent une chaîne saturée ou partiellement insaturée de 2, 3 ou 4 membres comprenant un ou plusieurs atomes de carbone et comprenant facultativement un groupe oxy (-O-), thio (-S-), sulfinyle (-SO-), sulfonyle (S(O)₂-) ou NR_{f} dans la chaîne ; la chaîne étant facultativement substituée sur chaque atome de carbone avec un substituant oxo (=O), thioxo (=S), -NR_{q}Rᵣ, -S(O)ₚRₛ ou -ORₜ, ou avec un ou deux substituants choisis indépendamment dans le groupe consistant en des substituants alkyle en C₁ à C₇, (alcoxy en C₁ à C₇)(alkyle en C₁ à C₇), aryle, (aryl) (alkyle en C₁ à C₇), hétéroaryle, (hétéroaryl) (alkyle en C₁ à C₇) et (aryl)oxy(alkyle en C₁ à C₇).

13. Composé suivant la revendication 1, dans lequel X et Y, conjointement, représentent une chaîne saturée ou partiellement insaturée de 2, 3 ou 4 membres comprenant un ou plusieurs atomes de carbone et comprenant facultativement un groupe oxy (-O-), thio (-S-), sulfinyle (-SO-), sulfonyle (S(O)₂-) ou NR_{f} dans la chaîne ; la chaîne étant facultativement substituée sur chaque atome de carbone avec un substituant oxo (=O), thioxo (=S), -NR_{q}Rᵣ, -S(O)ₚRₛ ou -ORₜ.

14. Composé suivant la revendication 1, dans lequel X et Y, conjointement, représentent une chaîne saturée ou partiellement insaturée de 2, 3 ou 4 membres comprenant un ou plusieurs atomes de carbone et comprenant facultativement un groupe oxy (-O-), thio (-S-), sulfinyle (-SO-), sulfonyle (S(O)₂-) ou NR_{f} dans la chaîne ; la chaîne étant facultativement substituée sur chaque atome de carbone avec un ou deux substituants choisis indépendamment dans le groupe consistant en des substituants alkyle en C₁ à C₇, (alcoxy en C₁ à C₇) (alkyle en C₁ à C₇), aryle, (aryl) (alkyle en C₁ à C₇), hétéroaryle, (hétéroaryl) (alkyle en C₁ à C₇) et (aryl)oxy(alkyle en C₁ à C₇).

15. Composé suivant la revendication 1, dans lequel X et Y, conjointement, représentent une chaîne de 2, 3 ou 4 membres comprenant un ou plusieurs atomes de carbone et comprenant facultativement un groupe oxy, thio, sulfinyle, sulfonyle ou NR_{f} dans la chaîne ; la chaîne étant facultativement substituée sur chaque atome de carbone avec un substituant oxo (=O), hydroxy, aryloxy, hétéroaryloxy ou alcoxy en C₁ à C₇, ou avec un ou deux substituants choisis indépendamment dans le groupe consistant en des substituants alkyle en C₁ à C₇, (alcoxy en C₁ à C₇) (alkyle en C₁ à C₇), aryle, (aryl) (alkyle en C₁ à C₇), hétéroaryle, (hétéroaryl)(alkyle en C₁ à C₇) et (aryl)oxy(alkyle en C₁ à C₇) ; et la chaîne étant facultativement substituée sur deux atomes adjacents avec une chaîne alkylène de 2, 3 ou 4 membres formant un noyau qui est condensé avec le noyau comprenant X et Y.

16. Composé suivant la revendication 1, dans lequel X et Y, conjointement, représentent une chaîne carbonée de 2, 3 ou 4 membres, la chaîne étant facultativement substituée sur chaque atome de carbone avec un substituant oxo, hydroxy, (aryl) oxy, hétéroaryl (oxy) ou alcoxy en C₁ à C₇, ou avec un ou deux substituants choisis indépendamment dans le groupe consistant en des substituants alkyle en C₁ à C₇, (alcoxy en C₁ à C₇) (alkyle en C₁ à C₇), aryle, (aryl) (alkyle en C₁ à C₇), hétéroaryle, (hétéroaryl) (alkyle en C₁ à C₇) et (aryl)oxy(alkyle en C₁ à C₇).

17. Composé suivant la revendication 1, dans lequel X et Y, conjointement, représentent une chaîne carbonée de 2 ou 3 membres facultativement substituée sur chaque atome de carbone avec un substituant oxo ou hydroxy, ou avec un ou deux substituants alkyle en C₁ à C₇.

18. Composé suivant la revendication 1, dans lequel X représente un groupe -O-, -S- ou -C(R_{g})(Rₕ)-, dans lequel R_{g} et Rₕ représentent chacun indépendamment un atome d'hydrogène, un groupe alkyle en C₁ à C₇, (alcoxy en C₁ à C₇) (alkyle en C₁ à C₇), aryle, (aryl)(alkyle en C₁ à C₇), hétéroaryle, (hétéroaryl) (alkyle en C₁ à C₇) ou (aryl) oxy (alkyle en C₁ à C₇), ou bien R_{g} et Rₕ, conjointement, représentent un groupe oxo.

19. Composé suivant la revendication 1, dans lequel Y représente un groupe -C(R_{g}) (Rₕ)-, -C(R_{g})(Rₕ)C(R_{g})(Rₕ)-, -C(R_{g})(Rₕ)C(R_{g})(Rₕ)C(R_{g})(Rₕ)-, -C(R_{g})(Rₕ)C(=O)-, -C(R_{g})(Rₕ)C(R_{g})(Rₕ)C(=O)-, -C(=O)C(R_{g})(Rₕ)- ou -C(=O)C(R_{g})(Rₕ)C(R_{g})(Rₕ)-, et chacun des groupes R_{g} et Rₕ représente indépendamment un atome d'hydrogène ou un groupe alkyle en C₁ à C₇.

20. Composé suivant la revendication 1, dans lequel X représente un groupe -O-, -S- ou -C(R_{g})(Rₕ)- ; et Y représente un groupe -C(R_{g})(Rₕ)C(=O)- ou -C(R_{g})(Rₕ)C(R_{g})(Rₕ)-, dans lequel chacun des groupes R_{g} et Rₕ représente indépendamment un atome d'hydrogène ou un groupe alkyle en C₁ à C₇.

21. Composé suivant la revendication 1, dans lequel X représente un groupe -O- ou -S- ; et Y représente un groupe -C(R_{g})(Rₕ)C(=O)-, -C(=O)C(R_{g})(Rₕ)- ou -C(R_{g})(Rₕ)C(R_{g})(Rₕ)-, dans lequel chacun des groupes R_{g} et Rₕ représente indépendamment un atome d'hydrogène ou un groupe alkyle en C₁ à C₇.

22. Composé suivant la revendication 1, dans lequel X et Y, conjointement, représentent un groupe -CH(R_{g})CH₂-, -CH(R_{g})CH(R_{g})CH₂-, -CH(R_{g})CH(R_{g})CH(R_{g})CH₂-, - C(R_{g})=C(R_{g})CH₂-, -CH=CHCH(R_{g})CH₂-, -CH(R_{g})CH=CHCH₂, -O-CH₂CH₂-, -O-CH₂CH(R_{g})CH₂-, -S-CH₂CH₂-, -S-CH₂CH(R_{g})CH₂-, -S(O)-CH₂CH₂-, -S(O)-CH₂CH(R_{g})CH₂-, -S(O)₂-CH₂CH₂-, -S(O)₂-CH₂CH(R_{g})CH₂-, -NR_{f}-CH₂CH₂-, -NR_{f}-CH₂CH(R_{g})CH₂-, -CH₂C(=O)-, - CH(R_{g})CH₂C(=O)-, -CH(R_{g})CH(R_{g})CH₂C(=O)-, -CH₂OC(=O)-, -CH(R_{g})CH₂OC(=O)-, -OCH₂C(=O)- ou -OCH₂CH₂C(=O)- ; dans lequel chaque groupe R_{g} représente indépendamment un groupe -NR_{q}Rᵣ, -S(O)ₚRₛ ou -ORₜ.

23. Composé suivant la revendication 1, dans lequel X et Y, conjointement, représentent un groupe -CH(R_{g})CH(R_{g})-, -CH(R_{g})CH(R_{g})CH(R_{g})-, -CH(R_{g})CH(R_{g})CH(R_{g})CH(R_{g})-, -CH=CHCH(R_{g})-, -C(R_{g})=C(R_{g})CH(R_{g})CH(R_{g})-, -CH(R_{g})C(R_{g})=C(R_{g})CH(R_{g})-, -O-CH(R_{g})CH(R_{g})-, -O-CH(R_{g})CH(R_{g})CH(R_{g})-, -S-CH(R_{g})CH(R_{g})-, -S-CH(R_{g})CH(R_{g})CH(R_{g})-, -S(O)-CH(R_{g})CH(R_{g})-, -S(O)-CH(R_{g})CH(R_{g})CH(R_{g})-, -S(O)₂-CH(R_{g})CH(R_{g})-, -S(O)₂-CH(R_{g})CH(R_{g})CH(R_{g})-, NR_{f}-CH(R_{g})CH(R_{g})-, -NR_{f}-CH(R_{g})CH(R_{g})CH(R_{g})-, -CH(R_{g})C(=O)-, -CH(R_{g})CH(R_{g})C(=O)-, -CH(R_{g})CH(R_{g})CH(R_{g})C(=O)-, -CH(R_{g})OC(=O)-, -CH(R_{g})CH(R_{g})OC(=O)-, - OCH(R_{g})C(=O)- ou -OCH(R_{g})CH(R_{g})C(=O)- ; dans lequel chaque groupe R_{g} représente indépendamment un atome d'hydrogène, un groupe alkyle en C₁ à C₇, aryle, (aryl) (alkyle en C₁ à C₇), (aryl)oxy, hétéroaryl(oxy) ou (aryl)oxy(alkyle en C₁ à C₇).

24. Composé suivant la revendication 1, dans lequel X et Y, conjointement, représentent un groupe -CH(R_{g})CH(R_{g})-, -CH(R_{g})CH(R_{g})CH(R_{g})-, -C(R_{g})=C(R_{g})CH(R_{g})-, - O-CH(R_{g})CH(R_{g})-, -S-CH(R_{g})CH(R_{g})-, -S(O)-CH(R_{g})CH(R_{g})-, - S(O)₂-CH(R_{g})CH(R_{g})-, -NR_{f}-CH(R_{g})CH(R_{g})-, -CH(R_{g})C(=O)-, -CH(R_{g})CH(R_{g})C(=O)-, -CH(R_{g})OC(=O)-, -OCH(R_{g})C(=O)-; dans lequel chaque groupe R_{g} représente indépendamment un atome d'hydrogène ou un groupe alkyle en C₁ à C₇.

25. Composé suivant la revendication 1, dans lequel X et Y, conjointement, représentent un groupe -CH(R_{g})CH₂-, -CH(R_{g})CH(R_{g})CH₂ ou -O-CH₂CH₂-, dans lequel chaque groupe R_{g} représente indépendamment un groupe -NR_{q}Rᵣ, -S(O)ₚRₛ, -ORₜ, alkyle en C₁ à C₇, (alcoxy en C₁ à C₇)(alkyle en C₁ à C₇), aryle, (aryl) (alkyle en C₁ à C₇), hétéroaryle, (hétéroaryl)(alkyle en C₁ à C₇) et (aryl) oxy (alkyle en C₁ à C₇).

26. Composé suivant la revendication 1, dans lequel X et Y, conjointement, représentent un groupe -C(=O)CH₂-, -CH₂C(=O)-, -C(=S)CH₂-, -CH₂C(=S)-, -C(=O)CH₂CH₂-, -CH₂C(=O)CH₂-, -CH₂CH₂C(=O)-, -C(=S)CH₂CH₂-, -CH₂C(=S)CH₂- ou -CH₂CH₂C(=S)-.

27. Composé suivant la revendication 1, dans lequel X et Y, conjointement, représentent un groupe -CH(R_{g})CH(R_{g})-, -CH(R_{g})CH(R_{g})CH(R_{g})-, -C(R_{g})=C(R_{g})CH(R_{g})-, - O-CH(R_{g})CH(R_{g})-, -S-CH(R_{g})CH(R_{g})-, -S(O)-CH(R_{g})CH(R_{g})-, -S(O)₂-CH(R_{g})CH(R_{g})-, NR_{f}-CH(R_{g})CH(R_{g})- ou -CH (R_{g})CH(R_{g})C(=O)- ; dans lequel chaque groupe R_{g} représente indépendamment un atome d'hydrogène ou un groupe alkyle en C₁ à C₇ ou bien, conjointement avec un groupe R_{g} sur un atome de carbone adjacent, forme un noyau carbocyclique tétra-, penta- ou hexagonal condensé.

28. Composé suivant la revendication 1, dans lequel X et Y, conjointement, représentent un groupe -CH(R_{g})CH(R_{g})-, -CH(R_{g})CH(R_{g})CH(R_{g})-, -O-CH(R_{g})CH(R_{g})-, -S-CH(R_{g})CH(R_{g})- ; dans lequel chaque groupe R_{g} représente indépendamment un atome d'hydrogène, un groupe alkyle en C₁ à C₇, aryle ou (aryl) (alkyle en C₁ à C₇).

29. Composé suivant la revendication 1, dans lequel X et Y, conjointement, représentent un groupe -CH₂CH₂CH₂-, -CH₂CH₂C(CH₃)H-, -CH₂C(CH₃)HCH₂-, -C(CH₃)HCH₂CH₂-, -CH₂CH₂-, -CH₂C(CH₃)H-, -C(CH₃)HC(CH₃)H-, -CH(R_{g})CH(R_{g})-, -O-CH₂CH₂-, -O-C(CH₃)HCH₂-,
ou -S-CH₂CH₂-.

30. Composé suivant la revendication 1, dans lequel X et Y, conjointement, représentent un groupe -CH₂CH₂-, -CH₂CH₂CH₂-, -CH=CHCH₂-, -O-CH₂CH₂-, -S-CH₂CH₂-, -S(O)-CH₂CH₂-, -S(O)₂-CH₂CH₂-, -NR_{f}-CH₂CH₂-, CH₂C(=O)-, -CH₂CH₂C(=O)-, -CH₂OC(=O)-, -OCH₂C(=O)-.

31. Composé suivant l'une quelconque des revendications 1 à 30, dans lequel R₂ représente un atome d'hydrogène.

32. Composé suivant la revendication 1, qui est :
5,6,9,10,11,12-hexahydro-4H,8H-azépino[4',5':4,5]-pyrrolo[3,2,1-ij]quinoléine ;
2-méthyl-5,6,9,10,11,12-hexahydro-4H,8H-azépino[4',5':4,5]pyrrolo[3,2,1-ij]quinoléine ;
1-méthoxy-5,6,9,10,11,12-hexahydro-4H,8H-azépino[4',5':4,5]pyrrolo[3,2,1-ij]quinoléine ;
2-fluoro-5,6,9,10,11,12-hexahydro-4H,8H-azépino[4',5':4,5]pyrrolo[3,2,1-ij]quinoléine ;
6-méthyl-5,6,9,10,11,12-hexahydro-4H,8H-azépino[4',5':4,5]pyrrolo[3,2,1-ij]quinoléine ;
5-méthyl-5,6,9,10,11,12-hexahydro-4H,8H-azépino[4',5':4,5]pyrrolo[3,2,1-ij]quinoléine ;
4-méthyl-5,6,9,10,11,12-hexahydro-4H,8H-azépino[4',5':4,5]pyrrolo[3,2,1-ij]quinoléine ;
(+)-6-méthyl-5,6,9,10,11,12-hexahydro-4H,8H-azépino[4',5':4,5]pyrrolo[3,2,1-ij]quinoléine ;
(-)-6-méthyl-5,6,9,10,11,12-hexahydro-4H,8H-azépino[4',5':4,5]pyrrolo[3,2,1-ij]quinoléine ;
(+)-5-méthyl-5,6,9,10,11,12-hexahydro-4H,8H-azépino[4',5':4,5]pyrrolo[3,2,1-ij]quinoléine ;
(-)-5-méthyl-5,6,9,10,11,12-hexahydro-4H,8H-azépino[4',5':4,5]pyrrolo[3,2,1-ij]quinoléine ;
(+)-4-méthyl-5,6,9,10,11,12-hexahydro-4H,8H-azépino[4',5':4,5]pyrrolo[3,2,1-ij]quinoléine ;
(-)-4-méthyl-5,6,9,10,11,12-hexahydro-4H,8H-azépino[4',5':4,5]pyrrolo[3,2,1-ij]quinoléine ;
le 4,5,8,9,10,11-hexahydro-7H-azépino[4,5-b]pyrrolo[3,2,1-hi]indole ;
le 2-fluoro-4,5,8,9,10,11-hexahydro-7H-azépino[4,5-b]-pyrrolo[3,2,1-hi]indole ;
le 2-méthoxy-4,5,8,9,10,11-hexahydro-7H-azépino[4,5-b]pyrrolo[3,2,1-hi]indole ;
le 5-méthyl-4,5,8,9,10,11-hexahydro-7H-azépino[4,5-b]-pyrrolo[3,2,1-hi]indole ;
le 4,5-diméthyl-4,5,8,9,10,11-hexahydro-7H-azépino[4,5-b]pyrrolo[3,2,1-hi]indole ;
le 2,3,4,5,8b,9,10,11,12,12a-décahydro-1H-azépino[4',5':4,5]-pyrrolo[3,2,1-jk]carbazole ;
le 6-méthyl-1,2,8,9,10,11-hexahydro-7H-azépino[4,5-b]-[1,4]oxazino[2,3,4-hi]indole ;
le 1,2,8,9,10,11-hexahydro-7H-azépino[4,5-b][1,4]-oxazino[2,3,4-hi]indole ;
le 6-chloro-1,2,8,9,10,11-hexahydro-7H-azépino[4,5-b]-[1,4]oxazino[2,3,4-hi]indole ;
le 5-fluoro-1,2,8,9,10,11-hexahydro-7H-azépino[4,5-b]-[1,4]oxazino[2,3,4-hi]indole ;
le 5-méthyl-1,2,8,9,10,11-hexahydro-7H-azépino[4,5-b]-[1,4]oxazino[2,3,4-hi]indole ;
le 6-fluoro-1,2,8,9,10,11-hexahydro-7H-azépino[4,5-b]-[1,4]oxazino[2,3,4-hi]indole ;
le 2-méthyl-1,2,8,9,10,11-hexahydro-7H-azépino[4,5-b]-[1,4]oxazino[2,3,4-hi]indole ;
le 1,2,8,9,10,11-hexahydro-7H-azépino[4,5-b] [1,4]thiazino[2,3,4-hi]indole ;
le 2,2-diméthyl-1,2,8,9,10,11-hexahydro-7H-azépino[9,5-b] [1,4]oxazino[2,3,4-hi]indole ;
le 4-fluoro-1,2,8,9,10,11-hexahydro-7H-azépino[4,5-b]-[1,4]oxazino[2,3,4-hi]indole ;
le 4-chloro-1,2,8,9,10,11-hexahydro-7H-azépino[4,5-b]-[1,4]oxazino[2,3,4-hi]indole ;
le 5-chloro-1,2,8,9,10,11-hexahydro-7H-azépino[4,5-b]-[1,4]oxazino[2,3,4-hi]indole ;
le 6-(trifluorométhyl)-1,2,8,9,10,11-hexahydro-7H-azépino[4,5-b] [1,4]oxazino[2,3,4-hi]indole ;
le 5,6-difluoro-1,2,8,9,10,11-hexahydro-7H-azépino[4,5-b] [1,4]oxazino[2,3,4-hi]indole ;
le 5-chloro-6-fluoro-1,2,8,9,10,11-hexahydro-7H-azépino[4,5-b] [1,4]oxazino[2,3,4-hi]indole ;
le 6-chloro-5-fluoro-1,2,8,9,10,11-hexahydro-7H-azépino[4,5-b][1,4]oxazino[2,3,4-hi]indole ;
le 5,6-dichloro-1,2,8,9,10,11-hexahydro-7H-azépino[4,5-b] [1,4]oxazino[2,3,4-hi]indole ;
le 4,6-dichloro-1,2,8,9,10,11-hexahydro-7H-azépino[4,5-b] [1,4]oxazino[2,3,4-hi]indole ;
le 1-chloro-2-fluoro-4,5,9,10,11-hexahydro-7H-azépino[4,5-b]pyrrolo[3,2,1-hi]indole ;
le 1-chloro-4,5,8,9,10,11-hexahydro-7H-azépino[4,5-b]-pyrrolo[3,2,1-hi]indole ;
le 2-chloro-4,5,8,9,10,11-hexahydro-7H-azépino[4,5-b]-pyrrolo[3,2,1-hi]indole ;
la 5,6,9,10,11,12-hexahydro-4H,8H-azépino[[4',5':4,5]-pyrrolo[3,2,1-ij]quinoléine-4-one ;
la 2-fluoro-5,6,9,10,11,12-hexahydro-4H,8H-azépino[4',5':4,5]pyrrolo[3,2,1-ij]quinoléine-4-one ;
la 2-chloro-5,6,9,10,11,12-hexahydro-4H,8H-azépino[4',5':4,5]pyrrolo[3,2,1-ij] quinoléine-4-one ;
la 6-méthyl-5,6,9,10,11,12-hexahydro-4H,8H-azépino[4',5':4,5]pyrrolo[3,2,1-ij] quinoléine-4-one ;
la 2-fluoro-6-méthyl-5,6,9,10,11,12-hexahydro-4H,8H-azépino[4',5':4,5]pyrrolo[3,2,1-ij]quinoléine-4-one ;
la 2,3-dichloro-6-méthyl-5,6,9,10,11,12-hexahydro-4H,8H-azépino[4',5':4,5]pyrrolo[3,2,1-ij]quinoléine-4-one ;
la 6-propyl-5,6,9,10,11,12-hexahydro-4H,8H-azépino[4',5':4,5]pyrrolo[3,2,1-ij]quinoléine-4-one ;
la 6- (trifluorométhyl)-5,6,9,10,11,12-hexahydro-4H,8H-azépino[4',5':4,5]pyrrolo[3,2,1-ij]quinoléine-4-one ;
la 5, 6, 8, 9,10,11,12,12a-octahydro-4H,7aH-azépino[4',5':4,5]pyrrolo[3,2,1-ij]quinoléine-4-ol ;
la 4-méthoxy-5,6,8,9,10,11,12,12a-octahydro-4H,7aH-azépino[4',5':4,5]pyrrolo[3,2,1-ij]quinoléine ;
la 10-benzoyl-4-phénoxy-5,6,9,10,11,12-hexahydro-4H,8H-azépino[4',5':4,5]pyrrolo[3,2,1-ij]quinoléine ; ou
la 4-phénoxy-5,6,9,10,11,12-hexahydro-4H,8H-azépino[4',5':4,5]pyrrolo[3,2,1-ij]quinoléine ;
ou un de ses sels pharmaceutiquement acceptables.

33. Composé 1,2,8,9,10,11-hexahydro-7H-azépino[4,5-b] [1,4]oxazino[2,3,4-hi]indole ; ou un de ses sels pharmaceutiquement acceptables.

34. Composé suivant la revendication 1, qui est :
le 4-bromo-1,2,8,9,10,11-hexahydro-7H-azépino[4,5-b] [1,4]-oxazino[2,3,4-hi]indole ;
le 5-bromo-1,2,8,9,10,11-hexahydro-7H-azépino[4,5-b] [1,4]-oxazino[2,3,4-hi]indole ;
le 6-bromo-1,2,8,9,10,11-hexahydro-7H-azépino[4,5-b] [1,4]-oxazino[2,3,4-hi]indole ;
le 5,6-dibromo-1,2,8,9,10,11-hexahydro-7H-azépino[4,5-b]-[1,4]oxazino[2,3,4-hi]indole ;
le 4,5-dibromo-1,2,8,9,10,11-hexahydro-7H-azépino[4,5-b]-[1,4]oxazino[2,3,4-hi]indole ;
le 4-méthoxy-1,2,8,9,10,11-hexahydro-7H-azépino[4,5-b]-[1,4]oxazino[2,3,4-hi]indole ;
le 5-méthoxy-1,2,8,9,10,11-hexahydro-7H-azépino[4,5-b]-[1,4]oxazino[2,3,4-hi]indole ;
le 6-méthoxy-1,2,8,9,10,11-hexahydro-7H-azépino[4,5-b]-[1,4]oxazino[2,3,4-hi]indole ;
le 4-(trifluorométhyl)-1,2,8,9,10,11-hexahydro-7H-azépino[4,5-b] [1,4]oxazino[2,3,4-hi]indole ;
le 5-(trifluorométhyl)-1,2,8,9,10,11-hexahydro-7H-azépino[4,5-b] [1,4]oxazino[2,3,4-hi]indole ;
le 4-benzyloxy-1,2,8,9,10,11-hexahydro-7H-azépino[4,5-b]-[1,4]oxazino[2,3,4-hi]indole ;
le 5-benzyloxy-1,2,8,9,10,11-hexahydro-7H-azépino[4,5-b]-[1,4]oxazino[2,3,4-hi]indole ;
le 6-benzyloxy-1,2,8,9,10,11-hexahydro-7H-azépino[4,5-b]-[1,4]oxazino[2,3,4-hi]indole ;
le 4-fluoro-1,2,8,9,10,11-hexahydro-7H-azépino[4,5-b] [1,4]-thiazino[2,3,4-hi]indole ;
le 5-fluoro-1,2,8,9,10,11-hexahydro-7H-azépino[4,5-b] [1,4]-thiazino[2,3,4-hi]indole ;
le 6-fluoro-1,2,8,9,10,11-hexahydro-7H-azépino[4,5-b] [1,4]-thiazino[2,3,4-hi]indole ;
le 4-chloro-1,2,8,9,10,11-hexahydro-7H-azépino[4,5-b] [1,4]-thiazino[2,3,4-hi]indole ;
le 5-chloro-1,2,8,9,10,11-hexahydro-7H-azépino[4,5-b] [1,4]-thiazino[2,3,4-hi]indole ;
le 6-chloro-1,2,8,9,10,11-hexahydro-7H-azépino[4,5-b] [1,4]-thiazino[2,3,4-hi]indole ;
le 4,5-difluoro-1,2,8,9,10,11-hexahydro-7H-azépino[4,5-b]-[1,4]thiazino[2,3,4-hi]indole ;
le 5,6-difluoro-1,2,8,9,10,11-hexahydro-7H-azépino[4,5-b]-[1,4]thiazino[2,3,4-hi]indole ;
le 4,6-difluoro-1,2,8,9,10,11-hexahydro-7H-azépino[4,5-b]-[1,4]thiazino[2,3,4-hi]indole ;
le 4-chloro-5-fluoro-1,2,8,9,10,11-hexahydro-7H-azépino[4,5-b] [1,4]-thiazino[2,3,4-hi]indole ;
le 4-chloro-6-fluoro-1,2,8,9,10,11-hexahydro-7H-azépino[4,5-b] [1,4]-thiazino[2,3,4-hi]indole ;
le 5-chloro-6-fluoro-1,2,8,9,10,11-hexahydro-7H-azépino[4,5-b] [1,4]-thiazino[2,3,4-hi]indole ;
le 6-chloro-5-fluoro-1,2,8,9,10,11-hexahydro-7H-azépino[4,5-b] [1,4]-thiazino[2,3,4-hi]indole ;
le 4-méthyl-1,2,8,9,10,11-hexahydro-7H-azépino[4,5-b] [1,4]-thiazino[2,3,4-hi]indole ;
le 5-méthyl-1,2,8,9,10,11-hexahydro-7H-azépino[9,5-b] [1,4]-thiazino[2,3,4-hi]indole ;
le 6-méthyl-1,2,8,9,10,11-hexahydro-7H-azépino[4,5-b] [1,4]-thiazino[2,3,4-hi]indole ;
le 4-méthoxy-1,2,8,9,10,11-hexahydro-7H-azépino[4,5-b]-[1,4]thiazino[2,3,4-hi]indole ;
le 5-méthoxy-1,2,8,9,10,11-hexahydro-7H-azépino[4,5-b]-[1,4]thiazino[2,3,4-hi]indole ;
le 6-méthoxy-1,2,8,9,10,11-hexahydro-7H-azépino[4,5-b]-[1,4]thiazino[2,3,4-hi]indole ;
le 4-(trifluorométhyl)-1,2,8,9,10,11-hexahydro-7H-azépino[4,5-b] [1,4]thiazino[2,3,4-hi]indole ;
le 5-(trifluorométhyl)-1,2,8,9,10,11-hexahydro-7H-azépino[4,5-b] [1,4]thiazino[2,3,4-hi]indole ;
le 6-(trifluorométhyl)-1,2,8,9,10,11-hexahydro-7H-azépino[4,5-b] [1,4]thiazino[2,3,4-hi]indole ;
le 4-benzyloxy-1,2,8,9,10,11-hexahydro-7H-azépino[4,5-b]-[1,4]thiazino[2,3,4-hi]indole ;
le 5-benzyloxy-1,2,8,9,10,11-hexahydro-7H-azépino[4,5-b]-[1,4]thiazino[2,3,4-hi]indole ;
le 6-benzyloxy-1,2,8,9,10,11-hexahydro-7H-azépino[4,5-b]-[1,4]thiazino[2,3,4-hi]indole ;
le 1-fluoro-4,5,8,9,10,11-hexahydro-7H-azépino[4,5-b]pyrrolo[3,2,1-hi]indole ;
le 3-fluoro-4,5,8,9,10,11-hexahydro-7H-azépino[4,5-b]pyrrolo[3,2,1-hi]indole ;
le 1-bromo-4,5,8,9,10,11-hexahydro-7H-azépino[4,5-b]pyrrolo[3,2,1-hi]indole ;
le 2-bromo-4,5,8,9,10,11-hexahydro-7H-azépino[4,5-b]pyrrolo[3,2,1-hi]indole ;
le 3-bromo-4,5,8,9,10,11-hexahydro-7H-azépino[4,5-b]pyrrolo[3,2,1-hi]indole ;
le 3-chloro-1,4,5,8,9,10,11-hexahydro-7H-azépino[4,5-b]pyrrolo[3,2,1-hi]indole ;
le 2-chloro-1-fluoro-4,5,8,9,10,11-hexahydro-7H-azépino[4,5-b]-pyrrolo[3,2,1-hi]indole ;
le 1-méthoxy-4,5,8,9,10,11-hexahydro-7H-azépino[4,5-b]pyrrolo[3,2,1-hi]indole ;
le 3-méthoxy-4,5,8,9,10,11-hexahydro-7H-azépino[4,5-b]pyrrolo[3,2,1-hi]indole ;
le 4-méthyl-4,5,8,9,10,11-hexahydro-7H-azépino[4,5-b]pyrrolo[3,2,1-hi]indole ;
le 1- (trifluorométhyl)-4,5,8,9,10,11-hexahydro-7H-azépino[4,5-b]pyrrolo[3,2,1-hi]indole ;
le 2-(trifluorométhyl)-4,5,8,9,10,11-hexahydro-7H-azépino[4,5-b]pyrrolo[3,2,1-hi]indole ;
le 3- (trifluorométhyl)-4,5,8,9,10,11-hexahydro-7H-azépino[4,5-b]pyrrolo[3,2,1-hi]indole ;
la 4-benzyloxy-5,6,8,9,10,11,12,12a-octahydro-4H,7aH-azépino[4',5':4,5]pyrrolo[3,2,1-ij]quinoléine ;
la 4- (3-chlorophénoxy)-5,6,9,10,11,12-hexahydro-4H,8H-azépino[4',5':4,5]pyrrolo[3,2,1-ij]quinoléine ;
la 4- (2-chlorophénoxy)-5,6,9,10,11,12-hexahydro-4H,8H-azépino[4',5':4,5]pyrrolo[3,2,1-ij]quinoléine ;
la 4-(4-méthoxyphénoxy)-5,6,9,10,11,12-hexahydro-4H,8H-azépino[4',5':4,5]pyrrolo[3,2,1-ij]quinoléine ;
la 4-(3-méthoxyphénoxy)-5,6,9,10,11,12-hexahydro-4H,8H-azépino[4',5':4,5]pyrrolo[3,2,1-ij]quinoléine ;
la 4- (2-méthoxyphénoxy)-5,6,9,10,11,12-hexahydro-4H,8H-azépino[4',5':4,5]pyrrolo[3,2,1-ij]quinoléine ;
la 4-(4-bromo-2-méthoxyphénoxy)-5,6,9,10,11,12-hexahydro-4H,8H-azépino[4',5':4,5]pyrrolo[3,2,1-ij]quinoléine ;
la 5,6,9,10,11,12-hexahydro-4H,8H-azépino[4',5':4,5]-pyrrolo[3,2,1-ij]quinoléine-4-amine ;
la N-phényl-5,6,9,10,11,12-hexahydro-4H,8H-azépino[4',5':4,5]pyrrolo[3,2,1-ij]quinoléine-4-amine ;
la N-(4-chlorophényl)-5,6,9,10,11,12-hexahydro-4H,8H-azépino[4',5':4,5]pyrrolo[3,2,1-ij]quinoléine-4-amine ;
la N-(3-chlorophényl)-5,6,9,10,11,12-hexahydro-4H,8H-azépino[4',5':4,5]pyrrolo[3,2,1-ij]quinoléine-4-amine ;
la N- (2-chlorophényl) -5,6,9,10,11,12-hexahydro-4H, 8H-azépino[4',5':4,5]pyrrolo[3,2,1-ij]quinoléine-4-amine ;
la N-(4-méthoxyphényl)-5,6,9,10,11,12-hexahydro-4H,8H-azépino[4',5':4,5]pyrrolo[3,2,1-ij]quinoléine-4-amine ;
la N-(3-méthoxyphényl)-5,6,9,10,11,12-hexahydro-4H,8H-azépino[4',5':4,5]pyrrolo[3,2,1-ij]quinoléine-4-amine ;
la N-(2-méthoxyphényl)-5,6,9,10,11,12-hexahydro-4H,8H-azépino[4',5':4,5]pyrrolo[3,2,1-ij]quinoléine-4-amine ;
la N-(4-bromo-2-méthoxyphényl)-5,6,9,10,11,12-hexahydro-4H,8H-azépino[4',5':4,5]pyrrolo[3,2,1-ij]quinoléine-4-amine ;
la 5,6,9,10,11,12-hexahydro-4H,8H-azépino[4',5':4,5]pyrrolo[3,2,1-ij]quinoléine-4-thione ;
la 4-(phénylsulfonyl)-5,6,9,10,11,12-hexahydro-4H,8H-azépino[4',5':4,5]pyrrolo[3,2,1-ij]quinoléine ;
la 4-(4-chlorophénylsulfonyl)-5,6,9,10,11,12-hexahydro-4H,8H-azépino[4',5':4,5]pyrrolo[3,2,1-ij]quinoléine ;
la 4-(3-chlorophénylsulfonyl)-5,6,9,10,11,12-hexahydro-4H,8H-azépino[4',5':4,5]pyrrolo[3,2,1-ij]quinoléine ;
la 4-(2-chlorophénylsulfonyl)-5,6,9,10,11,12-hexahydro-4H,8H-azépino[4',5':4,5]pyrrolo[3,2,1-ij]quinoléine ;
la 4-(4-méthoxyphénylsulfonyl)-5,6,9,10,11,12-hexahydro-4H,8H-azépino[4',5':4,5]pyrrolo[3,2,1-ij]quinoléine ;
la 4-(3-méthoxyphénylsulfonyl)-5,6,9,10,11,12-hexahydro-4H,8H-azépino[4',5':4,5]pyrrolo[3,2,1-ij]quinoléine ;
la 4-(2-méthoxyphénylsulfonyl)-5,6,9,10,11,12-hexahydro-4H,8H-azépino[4',5':4,5]pyrrolo[3,2,1-ij]quinoléine ;
la 4-(4-bromo-2-méthoxyphénylsulfonyl)-5,6,9,10,11,12-hexahydro-4H,8H-azépino[4',5':4,5]pyrrolo[3,2,1-ij]quinoléine ;
le 5,6,8,9,10,11,12,12a,-octahydro-4H,7aH-azépino[4',5':4,5]pyrrolo[3,2,1-ij]quinoléine-5-ol ;
la 5-méthoxy-5,6,8,9,10,11,12,12a,-octahydro-4H,7aH-azépino[4',5':4,5]pyrrolo[3,2,1-ij]quinoléine ;
la 5-benzyloxy-5,6,8,9,10,11,12,12a,-octahydro-4H,7aH-azépino[4',5':4,5]pyrrolo[3,2,1-ij]quinoléine ;
la 5-phénoxy-5,6,9,10,11,12-hexahydro-4H,8H-azépino[4',5':4,5]pyrrolo[3,2,1-ij]quinoléine ;
la 5-(4-chlorophénoxy)-5,6,9,10,11,12-hexahydro-4H,8H-azépino[4',5':4,5]pyrrolo[3,2,1-ij]quinoléine ;
la 5-(3-chlorophénoxy)-5,6,9,10,11,12-hexahydro-4H,8H-azépino[4',5':4,5]pyrrolo[3,2,1-ij]quinoléine ;
la 5-(2-chlorophénoxy)-5,6,9,10,11,12-hexahydro-4H,8H-azépino[4',5':9,5]pyrrolo[3,2,1-ij]quinoléine ;
la 5-(4-méthoxyphénoxy)-5,6,9,10,11,12-hexahydro-4H,8H-azépino[4',5':4,5]pyrrolo[3,2,1-ij]quinoléine ;
la 5-(3-méthoxyphénoxy)-5,6,9,10,11,12-hexahydro-4H,8H-azépino[4',5':4,5]pyrrolo[3,2,1-ij]quinoléine ;
la 5-(2-méthoxyphénoxy)-5,6,9,10,11,12-hexahydro-4H,8H-azépino[4',5':4,5]pyrrolo[3,2,1-ij]quinoléine ;
la 5-(4-bromo-2-méthoxyphénoxy)-5,6,9,10,11,12-hexahydro-4H,8H-azépino[4',5':4,5]pyrrolo[3,2,1-ij]quinoléine ;
la 5,6,9,10,11,12-hexahydro-4H,8H-azépino[4',5':4,5]pyrrolo[3,2,1-ij]quinoléine-5-amine ;
la N-phényl-5,6,9,10,11,12-hexahydro-4H,8H-azépino[4',5':4,5]-pyrrolo[3,2,1-ij]quinoléine-5-amine ;
la N-(4-chlorophényl)-5,6,9,10,11,12-hexahydro-4H,8H-azépino[4',5':4,5]pyrrolo[3,2,1-ij]quinoléine-5-amine ;
la N-(3-chlorophényl)-5,6,9,10,11,12-hexahydro-4H,8H-azépino[4',5':4,5]pyrrolo[3,2,1-ij]quinoléine-5-amine ;
la N-(2-chlorophényl)-5,6,9,10,11,12-hexahydro-4H,8H-azépino[4',5':4,5]pyrrolo[3,2,1-ij]quinoléine-5-amine ;
la N-(4-méthoxyphényl)-5,6,9,10,11,12-hexahydro-4H,8H-azépino[4',5':4,5]pyrrolo[3,2,1-ij]quinoléine-5-amine ;
la N-(3-méthoxyphényl)-5,6,9,10,11,12-hexahydro-4H,8H-azépino[4',5':4,5]pyrrolo[3,2,1-ij]quinoléine-5-amine ;
la N-(2-méthoxyphényl)-5,6,9,10,11,12-hexahydro-4H,8H-azépino[4',5':4,5]pyrrolo[3,2,1-ij]quinoléine-5-amine ;
la N-(4-bromo-2-méthoxyphényl)-5,6,9,10,11,12-hexahydro-4H,8H-azépino[4',5':4,5]pyrrolo[3,2,1-ij]quinoléine-5-amine ;
la 5,6,9,10,11,12-hexahydro-4H,8H-azépino[4',5':4,5]pyrrolo[3,2,1-ij]quinoléine-5-thione ;
la 5- (phénylsulfonyl)-5,6,9,10,11,12-hexahydro-4H,8H-azépino[4',5':4,5]pyrrolo[3,2,1-ij]quinoléine ;
la 5-(4-chlorophénylsulfonyl)-5,6,9,10,11,12-hexahydro-4H,8H-azépino[4',5':4,5]pyrrolo[3,2,1-ij]quinoléine ;
la 5-(3-chlorophénylsulfonyl)-5,6,9,10,11,12-hexahydro-4H,8H-azépino[4',5':4,5]pyrrolo[3,2,1-ij]quinoléine ;
la 5-(2-chlorophénylsulfonyl)-5,6,9,10,11,12-hexahydro-4H,8H-azépino[4',5':4,5]pyrrolo[3,2,1-ij]quinoléine ;
la 5-(4-méthoxyphénylsulfonyl)-5,6,9,10,11,12-hexahydro-4H,8H-azépino[4',5':4,5]pyrrolo[3,2,1-ij]quinoléine ;
la 5-(3-méthoxyphénylsulfonyl)-5,6,9,10,11,12-hexahydro-4H,8H-azépino[4',5':4,5]pyrrolo[3,2,1-ij]quinoléine ;
la 5-(2-méthoxyphénylsulfonyl)-5,6,9,10,11,12-hexahydro-4H,8 H-azépino[4',5':4,5]pyrrolo[3,2,1-ij]quinoléine ;
la 5-(4-bromo-2-méthoxyphénylsulfonyl)-5,6,9,10,11,12-hexahydro-4H,8H-azépino[4',5':4,5]pyrrolo[3,2,1-ij]quinoléine ;
la 4-(4-chlorophénoxy)-5,6,9,10,11,12-hexahydro-4H,8H-azépino[4',5':4,5]pyrrolo[3,2,1-ij]quinoléine ;
la 1-[2-(4-fluorophénoxy)éthoxy]-5,6,9,10,11,12-hexahydro-4H,8H-azépino[4',5':4,5]pyrrolo[3,2,1-ij]quinoléine ;
la 1-[2-(3-fluorophénoxy)éthoxy]-5,6,9,10,11,12-hexahydro-4H,8H-azépino[4',5':4,5]pyrrolo[3,2,1-ij]quinoléine ;
la 1-[2-(2-fluorophénoxy)éthoxy]-5,6,9,10,11,12-hexahydro-4H,8H-azépino[4',5':4,5]pyrrolo[3,2,1-ij]quinoléine ;
la 1-[2-(4-chlorophénoxy)éthoxy]-5,6,9,10,11,12-hexahydro-4H,8H-azépino[4',5':4,5]pyrrolo[3,2,1-ij]quinoléine ;
la 1-[2-(3-chlorophénoxy)éthoxy]-5,6,9,10,11,12-hexahydro-4H,8H-azépino[4',5':4,5]pyrrolo[3,2,1-ij]quinoléine ;
la 1-[2-(2-chlorophénoxy)éthoxy]-5,6,9,10,11,12-hexahydro-4H,8H-azépino[4',5':4,5]pyrrolo[3,2,1-ij]quinoléine ;
la 1-[2-(4-bromophénoxy)éthoxy]-5,6,9,10,11,12-hexahydro-4H,8H-azépino[4',5':4,5]pyrrolo[3,2,1-ij]quinoléine ;
la 1-[2-(3-bromophénoxy)éthoxy]-5,6,9,10,11,12-hexahydro-4H,8H-azépino[4',5':4,5]pyrrolo[3,2,1-ij]quinoléiné ;
la 1-[2-(2-bromophénoxy)éthoxy]-5,6,9,10,11,12-hexahydro-4H,8H-azépino[4',5':4,5]pyrrolo[3,2,1-ij]quinoléine ;
la 1-[2-(4-méthoxyphénoxy)éthoxy]-5,6,9,10,11,12-hexahydro-4H,8H-azépino[4',5':4,5]pyrrolo[3,2,1-ij]quinoléine ;
la 1-[2-(3-méthoxyphénoxy)éthoxy]-5,6,9,10,11,12-hexahydro-4H,8H-azépino[4',5':4,5]pyrrolo[3,2,1-ij]quinoléine ;
la 1-[2-(2-méthoxyphénoxy)éthoxy]-5,6,9,10,11,12-hexahydro-4H,8H-azépino[4',5':4,5]pyrrolo[3,2,1-ij]quinoléine ;
la 1-[2-(4-méthylphénoxy)éthoxy]-5,6,9,10,11,12-hexahydro-4H,8H-azépino[4',5':4,5]pyrrolo[3,2,1-ij]quinoléine ;
la 1-[2-(3-méthylphénoxy)éthoxy]-5,6,9,10,11,12-hexahydro-4H,8H-azépino[4',5':4,5]pyrrolo[3,2,1-ij]quinoléine ;
la 1-[2-(2-méthylphénoxy)éthoxy]-5,6,9,10,11,12-hexahydro-4H,8H-azépino[4',5':4,5]pyrrolo[3,2,1-ij]quinoléine ;
la 1-[2-(1-naphtyloxy)éthoxy]-5,6,9,10,11,12-hexahydro-4H,8H-azépino[4',5':4,5]pyrrolo[3,2,1-ij]quinoléine ;
la 1-[2-(2-naphtyloxy)éthoxy]-5,6,9,10,11,12-hexahydro-4H,8H-azépino[4',5':4,5]pyrrolo[3,2,1-ij]quinoléine ;
la 1-[2-([1,1'-biphényl]-4-yloxy)éthoxy]-5,6,9,10,11,12-hexahydro-4H,8H-azépino[4',5':4,5]pyrrolo[3,2,1-ij]quinoléine ;
la 1-[2-([1,1'-biphényl]-3-yloxy)éthoxy]-5,6,9,10,11,12-hexahydro-4H,8H-azépino[4',5':4,5]pyrrolo[3,2,1-ij]quinoléine ;
la 1-[2-([1,1'-biphényl]-2-yloxy)éthoxy]-5,6,9,10,11,12-hexahydro-4H,8H-azépino[4',5':4,5]pyrrolo[3,2,1-ij]quinoléine ;
la 1-{2-[4-(trifluorométhoxy)phénoxy]éthoxy}-5,6,9,10,11,12-hexahydro-4H,8H-azépino[4',5':4,5]pyrrolo[3,2,1-ij]quinoléine ;
la 1-{2-[3-(trifluorométhoxy)phénoxy]éthoxy}-5,6,9,10,11,12-hexahydro-4H,8H-azépino[4',5':4,5]pyrrolo[3,2,1-ij]quinoléine ;
la 1-{2-[2-(trifluorométhoxy)phénoxy]éthoxy}-5,6,9,10,11,12-hexahydro-4H,8H-azépino[4',5':4,5]pyrrolo[3,2,1-ij]quinoléine ;
la 1-{2-[4-(trifluorométhyl)phénoxy]éthoxy}-5,6,9,10,11,12-hexahydro-4H,8H-azépino[4',5':4,5]pyrrolo[3,2,1-ij]quinoléine ;
la 1-{2-[3-(trifluorométhyl)phénoxy]éthoxy}-5,6,9,10,11,12-hexahydro-4H,8H-azépino[4',5':4,5]pyrrolo[3,2,1-ij]quinoléine ; ou
la 1-{2-[2-(trifluorométhyl)phénoxy]éthoxy}-5,6,9,10,11,12-hexahydro-4H,8H-azépino[4',5':4,5]pyrrolo[3,2,1-ij]quinoléine ; ou un de ses sels pharmaceutiquement acceptables.

35. Composition pharmaceutique comprenant un composé suivant l'une quelconque des revendications 31 à 34 et un excipient pharmaceutiquement acceptable.

36. Composé suivant l'une quelconque des revendications 31 à 34, destiné à être utilisé dans un diagnostic médical ou en thérapeutique.

37. Composé suivant la revendication 36, la thérapeutique étant le traitement d'une maladie ou d'un trouble du système nerveux central.

38. Composé suivant la revendication 37, la maladie ou le trouble du système nerveux central étant l'obésité, la dépression, la schizophrénie, un trouble schizophréniforme, un trouble schizo-affectif, les délires, une maladie due au stress, la panique, une phobie, le syndrome obsessionnel compulsif, le syndrome de stress post-traumatique, une dépression du système immunitaire, un problème induit par un stress au niveau du système urinaire, gastro-intestinal ou cardiovasculaire, des troubles neurodégénératifs, l'autisme, les vomissements induits par la chimiothérapie, l'hypertension, la migraine, les céphalées, les céphalées vasculaires de Horton, un dysfonctionnement sexuel, des troubles d'accoutumance et le syndrome de sevrage, un trouble d'adaptation, un trouble de l'apprentissage et trouble mental en association avec l'âge, l'anorexie mentale, l'apathie, un trouble de déficit d'attention dû à un état médical général, un trouble d'hyperactivité avec déficit d'attention, une perturbation comportementale, un trouble bipolaire, la boulimie mentale, le syndrome de fatigue chronique, un trouble de la conduite, un trouble cyclothymique, un trouble dysthymique, la fibromyalgie et d'autres troubles somatoformes ; le syndrome d'anxiété généralisée, un trouble provoqué par inhalation, un trouble d'intoxication, un trouble du mouvement, un trouble de défiance par opposition, la neuropathie périphérique, le syndrome de stress post-traumatique, un trouble dysphorique prémenstruel, un trouble psychotique, un trouble de l'humeur, un trouble affectif saisonnier, un trouble du sommeil, un trouble de développement spécifique, un trouble d'agitation, le syndrome d'inhibiteur de réabsorption de sérotonine sélective ou les tics.

39. Composé suivant la revendication 36, dans lequel la thérapeutique est le traitement de l'anxiété, de l'obésité, de la dépression ou d'une maladie due à un stress.

40. Utilisation d'un composé suivant l'une quelconque des revendications 31 à 34 pour la préparation d'un médicament destiné au traitement ou à la prévention d'une maladie ou d'un trouble du système nerveux central.

41. Utilisation suivant la revendication 40, dans laquelle la maladie ou le trouble du système nerveux central est l'obésité, la dépression, la schizophrénie, un trouble schizophréniforme, un trouble schizo-affectif, les délires, une maladie due au stress, la panique, une phobie, le syndrome obsessionnel compulsif, le syndrome de stress post-traumatique, une dépression du système immunitaire, un problème induit par un stress au niveau du système urinaire, gastro-intestinal ou cardiovasculaire, des troubles neurodégénératifs, l'autisme, les vomissements induits par la chimiothérapie, l'hypertension, la migraine, les céphalées, les céphalées vasculaires de Horton, un dysfonctionnement sexuel, des troubles d'accoutumance et le syndrome de sevrage, un trouble d'adaptation, un trouble de l'apprentissage et trouble mental en association avec l'âge, l'anorexie mentale, l'apathie, un trouble de déficit d'attention dû à un état médical général, un trouble d'hyperactivité avec déficit d'attention, une perturbation comportementale, un trouble bipolaire, la boulimie mentale, le syndrome de fatigue chronique, un trouble de la conduite, un trouble cyclothymique, un trouble dysthymique, la fibromyalgie et d'autres troubles somatoformes ; le syndrome d'anxiété généralisée, un trouble provoqué par inhalation, un trouble d'intoxication, un trouble du mouvement, un trouble de défiance par opposition, la neuropathie périphérique, le syndrome de stress post-traumatique, un trouble dysphorique prémenstruel, un trouble psychotique, un trouble de l'humeur, un trouble affectif saisonnier, un trouble du sommeil, un trouble de développement spécifique, un trouble d'agitation, le syndrome d'inhibiteur de réabsorption de sérotonine sélective ou les tics.

42. Utilisation suivant la revendication 40, dans laquelle la maladie ou le trouble du système nerveux central est l'anxiété, l'obésité, la dépression ou une maladie due à un stress.

43. Utilisation suivant la revendication 40, dans laquelle la maladie ou le trouble du système nerveux central est l'anxiété, l'obésité, la dépression, la schizophrénie, une maladie due à un stress, la panique, une phobie, le syndrome obsessionnel compulsif, le syndrome de stress post-traumatique, une dépression du système immunitaire, un problème induit par un stress au niveau du système gastro-intestinal ou cardiovasculaire ou un dysfonctionnement sexuel.

44. Composé de formule I : 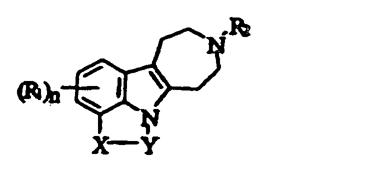 dans laquelle
chaque groupe R₁ représente indépendamment un groupe hydroxy, nitro, halogéno, cyano, trifluorométhyle, trifluorométhoxy, alkyle en C₁ à C₇, alcoxy en C₁ à C₇, alcanoyle en C₁ à C₇ (alcoxy en C₁ à C₇)carbonyle, alcanoyloxy en C₁ à C₇, aryle, hétéroaryle, -S(O)ₘNRₐR_{b}, NR_{c}R_{d}, -S(O)ₘRₑ, ou -C(=O)NRₐR_{b}, dans lequel n'importe quel groupe alkyle en C₁ à C₇, alcoxy en C₁ à C₇, alcanoyle en C₁ à C₇, (alcoxy en C₁ à C₇)carbonyle ou alcanoyloxy en C₁ à C₇ de R₁ est facultativement partiellement insaturé et est facultativement substitué avec un substituant aryle, aryloxy, hétéroaryle, hétéroaryloxy, hydroxy, nitro, halogéno, cyano, alcoxy en C₁ à C₇, alcanoyle en C₁ à C₇, (alcoxy en C₁ à C₇)carbonyle, alcanoyloxy en C₁ à C₇, -S(O)ₘRₑ, -S(O)ₘNRₐR_{b}, NR_{c}R_{d} ou -C(=O)NRₐR_{b} ;
R₂ représente un groupe protecteur convenable ;
X et Y, conjointement, représentent une chaîne saturée ou partiellement insaturée de 2,3 ou 4 membres comprenant un ou plusieurs atomes de carbone et comprenant facultativement un groupe oxy (-O-), thio (-S-), sulfinyle (-SO-), sulfonyle (S(O)₂-) ou NR_{f} dans la chaîne ; la chaîne étant facultativement substituée sur chaque atome de carbone avec un substituant oxo (=O), thioxo (=S), -NR_{q}Rᵣ, -S(O)ₚRₛ ou -ORₜ, ou avec un ou deux substituants choisis indépendamment dans le groupe consistant en des substituants alkyle en C₁ à C₇, (alcoxy en C₁ à C₇)(alkyle en C₁ à C₇), aryle, (aryl) (alkyle en C₁ à C₇), hétéroaryle, (hétéroaryl) (alkyle en C₁ à C₇) et (aryl) oxy (alkyle en C₁ à C₇) ; ou la chaîne étant facultativement substituée sur un atome de carbone avec un noyau carboné spirocyclique tétra-, penta- ou hexagonal ; ou la chaîne étant facultativement substituée sur deux atomes de carbone adjacents avec une chaîne alkylène de 2, 3 ou 4 membres (par exemple -CH₂CH₂-, -CH₂CH₂CH₂- ou -CH₂CH₂CH₂CH₂-) formant un noyau qui est condensé avec le noyau comprenant X et Y ;
chaque indice m est indépendamment égal à 0, 1 ou 2 ;
n est égal à 0, 1, 2 ou 3 ;
p est égal à 0, 1 ou 2 ;
chacun des groupes Rₐ et R_{b} représente indépendamment un atome d'hydrogène, un groupe alkyle en C₁ à C₇, aryle, (aryl) (alkyle en C₁ à C₇), hétéroaryle ou (hétéroaryl) (alkyle en C₁ à C₇) ; ou bien Rₐ et R_{b}, conjointement avec l'atome d'azote auquel ils sont fixés, forment un noyau pyrrolidino, pipéridino, morpholino ou thiomorpholino ;
chacun des groupes R_{c} et R_{d} représente indépendamment un atome d'hydrogène, un groupe alkyle en C₁ à C₇, alcanoyle en C₁ à C₇, (alcoxy en C₁ à C₇) carbonyle, aryle, (aryl) (alkyle en C₁ à C₇), hétéroaryle, (hétéroaryl) (alkyle en C₁ à C₇), arylcarbonyle, hétéroarylcarbonyle, aryloxycarbonyle ou hétéroaryloxycarbonyle ; ou bien R_{c} et R_{d}, conjointement avec l'atome d'azote auquel ils sont fixés, forment un noyau pyrrolidino, pipéridino, morpholino ou thiomorpholino ;
chaque groupe Rₑ représente indépendamment un atome d' hydrogène, un groupe alkyle en C₁ à C₇, aryle, (aryl) (alkyle en C₁ à C₇), hétéroaryle ou (hétéroaryl) (alkyle en C₁ à C₇) ;
R_{f} représente un atome d'hydrogène, un groupe alkyle en C₁ à C₇, aryle, (aryl) (alkyle en C₁ à C₇), hétéroaryle ou (hétéroaryl) (alkyle en C₁ à C₇) ; ou bien représente une liaison à une chaîne alkylène de 2, 3 ou 4 membres qui forme un noyau qui est condensé avec le noyau comprenant X et Y ;
chacun des groupes R_{q} et Rᵣ représente indépendamment un atome d'hydrogène, un groupe alkyle en C₁ à C₇, aryle, (aryl) (alkyle en C₁ à C₇), hétéroaryle ou (hétéroaryl) (alkyle en C₁ à C₇) ; ou bien R_{q} et Rᵣ, conjointement avec l'atome d'azote auquel ils sont fixés, forment un noyau pyrrolidino, pipéridino, morpholino ou thiomorpholino ;
Rₛ représente un groupe alkyle en C₁ à C₇, aryle, (aryl) (alkyle en C₁ à C₇), hétéroaryle ou (hétéroaryl) (alkyle en C₁ à C₇) ; et
Rₜ représente un atome d'hydrogène, un groupe alkyle en C₁ à C₇, aryle, (aryl) (alkyle en C₁ à C₇), hétéroaryle ou (hétéroaryl) (alkyle en C₁ à C₇) ;
n'importe quel noyau aryle ou hétéroaryle de R₁, R₂, X, Y, Rₐ-R_{f} ou R_{q}-Rₜ étant facultativement substitué avec un ou plusieurs (par exemple 1, 2, 3 ou 4) substituants choisis indépendamment entre des substituants halogéno, hydroxy, cyano, nitro, trifluorométhyle, trifluorométhoxy, alkyle en C₁ à C₇, alcoxy en C₁ à C₇, phényle, sulfonyle, NRⱼRₖ ou -C(=O)NRⱼRₖ ; dans lesquels chacun des groupes Rⱼ et Rₖ représente indépendamment un atome d'hydrogène, un groupe alkyle en C₁ à C₇, alcanoyle en C₁ à C₇, (alcoxy en C₁ à C₇) carbonyle, aryle, (aryl) (alkyle en C₁ à C₇), arylcarbonyle ou aryloxycarbonyle; ou bien Rⱼ et Rₖ, conjointement avec l'atome d'azote auquel ils sont fixés, forment un noyau pyrrolidino, pipéridino, morpholino ou thiomorpholino ;
ou un de ses sels pharmaceutiquement acceptables ;
sous réserve que Y ne représente pas un groupe oxy, thio, sulfinyle ou NR_{f} ; et
sous réserve que X et Y, conjointement, ne représentent pas une chaîne insaturée de 2 membres ; et
sous réserve qu'aucun atome de carbone de X et Y ne soit lié à plus d'un groupe oxy, thio, sulfinyle ou NR_{f}.

45. Composé suivant la revendication 1, dans lequel R₂ représente un groupe alkyle en C₁ à C₄, alcanoyle en C₁ à C₄, arylcarbonyle, (aryl) (alkyle en C₁ ou C₂), (alcoxy en C₁ à C₄)carbonyle, aryloxycarbonyle, arylsulfonyle ou (aryl)-méthoxycarbonyle, dans lequel n'importe quel groupe aryle est facultativement substitué avec 1, 2 ou 3 substituants choisis indépendamment entre des substituants alkyle en C₁ à C₄ et trifluorométhyle.

46. Composé suivant la revendication 1, dans lequel R₂ représente un groupe méthyle, éthyle, propyle, isopropyle, acétyle, tertiobutoxycarbonyle, benzyloxycarbonyle, benzyle ou p-tuluènesulfonyle.

47. Composé suivant la revendication 1, qui est :
la 10-benzoyl-5,6,9,10,11,12-hexahydro-4H,8H-azépino[4',5':4,5]pyrrolo[3,2,1-ij]quinoléine-4-one ;
la 10-benzoyl-2-fluoro-5,6,9,10,11,12-hexahydro-4H,8H-azépino[4',5':4,5]pyrrolo[3,2,1-ij]quinoléine-4-one ;
la 10-benzoyl-2-chloro-5,6,9,10,11,12-hexahydro-4H,8H-azépino[4',5':4,5]pyrrolo[3,2,1-ij]quinoléine-4-one ;
la 10-benzoyl-6-méthyl-5,6,9,10,11,12-hexahydro-4H,8H-azépino[4',5':4,5]pyrrolo[3,2,1-ij]quinoléine-4-one ;
la 10-benzoyl-2-fluoro-6-méthyl-5,6,9,10,11,12-hexahydro-4H,8H-azépino[4',5':4,5]pyrrolo[3,2,1-ij]quinoléine-4-one ;
la 10-benzoyl-2,3-dichloro-6-méthyl-5,6,9,10,11,12-hexahydro-4H,8H-azépino[4',5':4,5]pyrrolo[3,2,1-ij]quinoléine-4-one ;
la 10-benzoyl-6-propyl-5,6,9,10,11,12-hexahydro-4H,8H-azépino[4',5':4,5]pyrrolo[3,2,1-ij]quinoléine-4-one ;
la 10-benzoyl-6- (trifluorométhyl) -5,6,9,10,11,12-hexahydro-4H,8H-azépino[4',5':4,5]pyrrolo[3,2,1-ij]quinoléine-4-one ;
le 10-benzoyl-5,6,8,9,10,11,12,12a-octahydro-4H,7aH-azépino[4',5':4,5]pyrrolo[3,2,1-ij]quinoléine-4-ol ; ou
la 10-benzoyl-4-méthoxy-5,6,8,9,10,11,12,12a-octahydro-4H,7aH-azépino[4',5':4,5]pyrrolo[3,2,1-ij]quinoléine.

48. Composé suivant la revendication 1, qui est :
le 5,6,8,9,11,12-hexahydro-4H,10H-azépino[4',5':4,5]pyrrolo[3,2,1-ij]quinoléine-10-carboxylate de benzyle ;
le 2-méthyl-5,6,8,9,11,12-hexahydro-9H,10H-azépino[4',5':4,5]pyrrolo[3,2,1-ij]quinoléine-10-carboxylate de benzyle ;
la 10-benzoyl-1-méthoxy-5,6,8,9,11,12-hexahydro-4H, 8H-azépino[4',5':4,5]pyrrolo[3,2,1-ij]quinoléine ;
le 2-fluoro-5,6,8,9,11,12-hexahydro-4H,10H-azépino[4',5':4,5]pyrrolo[3,2,1-ij]quinoléine-10-carboxylate de benzyle ;
le 6-méthyl-5,6,8,9,11,12-hexahydro-4H,10H-azépino[4',5':4,5]pyrrolo[3,2,1-ij]quinoléine-10-carboxylate de benzyle ;
le 5-méthyl-5,6,8,9,11,12-hexahydro-4H,10H-azépino[4',5':4,5]pyrrolo[3,2,1-ij]quinoléine-10-carboxylate de benzyle ;
le 4-méthyl-5,6,8,9,11,12-hexahydro-4H,10H-azépino[4',5':4,5]pyrrolo[3,2,1-ij]quinoléine-10-carboxylate de benzyle ;
le 6-méthyl-5,6,8,9,11,12-hexahydro-4H,10H-azépino[4',5':4,5]-pyrrolo[3,2,1-ij]quinoléine-10-carboxylate de (+)-benzyle ;
le 6-méthyl-5,6,8,9,11,12-hexahydro-4H,10H-azépino[4',5':4,5]-pyrrolo[3,2,1-ij]quinoléine-10-carboxylate de (-)-benzyle ;
le 5-méthyl-5,6,8,9,11,12-hexahydro-4H,10H-azépino[4',5':4,5]-pyrrolo[3,2,1-ij]quinaléine-10-carboxylate de (+)-benzyle ;
le 5-méthyl-5,6,8,9,11,12-hexahydro-4H,10H-azépino[4',5':4,5]-pyrrolo[3,2,1-ij]quinoléine-10-carboxylate de (-)-benzyle ;
le 4-méthyl-5,6,8,9,11,12-hexahydro-4H,10H-azépino[4',5':4,5]-pyrrolo[3,2,1-ij]quinoléine-10-carboxylate de (+)-benzyle ;
le 4-méthyl-5,6,8,9,11,12-hexahydro-4H,10H-azépino[4',5':4,5]-pyrrolo[3,2,1-ij]quinoléine-10-carboxylate de (-)-benzyle ;
le 2-méthyl-1,2,7,8,10,11-hexahydro-9H-azépino[4,5-b] [1,4]-oxazino[2,3,4-hi]indole-9-carboxylate de benzyle ;
le 2,2-diméthyl-1,2,7,8,10,11-hexahydro-9H-azépino[4,5-b]-[1,4]oxazino[2,3,4-hi]indole-9-carboxylate de benzyle ;
le 4-fluoro-1,2,7,8,10,11-hexahydro-9H-azépino[4,5-b] [1,4]-oxazino[2,3,4-hi]indole-9-carboxylate de benzyle ;
le 4-chloro-1,2,7,8,10,11-hexahydro-9H-azépino[4,5-b] [1,4]-oxazino[2,3,4-hi]indole-9-carboxylate de benzyle ;
le 5-fluoro-1,2,7,8,10,11-hexahydro-9H-azépino[4,5-b] [1,4]-oxazino[2,3,4-hi]indole-9-carboxylate de benzyle ;
le 5-chloro-1,2,7,8,10,11-hexahydro-9H-azépino[4,5-b] [1,4]-oxazino[2,3,4-hi]indole-9-carboxylate de benzyle ;
le 5-méthyl-1,2,7,8,10,11-hexahydro-9H-azépino[4,5-b] [1,4]-oxazino[2,3,4-hi]indole-9-carboxylate de benzyle ;
le 6-fluoro-1,2,7,8,10,11-hexahydro-9H-azépino[4,5-b] [1,4]-oxazino[2,3,4-hi]indole-9-carboxylate de benzyle ;
le 6-chloro-1,2,7,8,10,11-hexahydro-9H-azépino[4,5-b] [1,4]-oxazino[2,3,4-hi]indole-9-carboxylate de benzyle ;
le 6-méthyl-1,2,6b,7,8,10,11,11a-octahydro-9H-azépino[4,5-b] [1,4]oxazino[2,3,4-hi]indole-9-carboxylate de benzyle ;
le 6- (trifluorométhyl) -1,2,7,8,10, 11-hexahydro-9H-azépino[4,5-b] [1,4]oxazino[2,3,4-hi]indole-9-carboxylate de benzyle ;
le 5,6-difluoro-1,2,7,8,10,11-hexahydro-9H-azépino[4,5-b]-[1,4]oxazino[2,3,4-hi]indole-9-carboxylate de benzyle ;
le 5-chloro-6-fluoro-1,2,7,8,10,11-hexahydro-9H-azépino[4,5-b] [1,4]-oxazino[2,3,4-hi]indole-9-carboxylate de benzyle ;
le 5-fluoro-6-chloro-1,2,7,8,10,11-hexahydro-9H-azépino[4,5-b] [1,4]-oxazino[2,3,4-hi]indole-9-carboxylate de benzyle ;
le 5, 6-dichloro-1,2,7,8,10,11-hexahydro-9H-azépino[4,5-b] [1,4]-oxazino[2,3,4-hi]indole-9-carboxylate de benzyle ;
le 4, 6-dichloro-1,2,7,8,10,11-hexahydro-9H-azépino[4,5-b] [1,4]-oxazino[2,3,4-hi]indole-9-carboxylate de benzyle ;
le 7-benzoyl-4,5,8,9,10,11-hexahydro-7H-azépino[4,5-b]-pyrrolo[3,2,1-hi]indole ;
le 7-benzoyl-2-fluoro-4,5,8,9,10,11-hexahydro-7H-azépino[4,5-b]pyrrolo[3,2,1-hi]indole ;
le 7-benzoyl-2-méthoxy-4,5,8,9,10,11-hexahydro-7H-azépino[4,5-b]pyrrolo[3,2,1-hi]indole ;
le 5-méthyl-4,5,8,9,10,11-hexahydro-7H-azépino[4,5-b]pyrrolo[3,2,1-hi]-7-carboxylate de benzyle ;
le 4,5-diméthyl-4,5,8,9,10,11-hexahydro-7H-azépino[4,5-b]-pyrrolo[3,2,1-hi]-7-carboxylate de benzyle ;
le 2,3,4,5,8b,9,10,11,12,12a-décahydro-1H-azépino[4',5':4,5]pyrrolo[3,2,1-jk]carbazole-7-carboxylate de benzyle ;
le 1-chloro-2-fluoro-4,5,8,9,10,11-hexahydro-7H-azépino[4,5-b]pyrrolo[3,2,1-hi]indole-7-carboxylate de benzyle ;
le 2-chloro-4,5,8,9,10,11-hexahydro-7H-azépino[4,5-b]pyrrolo[3,2,1-hi]indole-7-carboxylate de benzyle ;
le 1-chloro-4,5,8,9,10,11-hexahydro-7H-azépino[4,5-b]pyrrolo[3,2,1-hi]indole-7-carboxylate de benzyle ;
la 10 benzoyl-5,6,9,10,11,12-hexahydro-4H,8H-azépino[4',5':4,5]pyrrolo[3,2,1-ij]quinoléine-4-one ;
la 10 benzoyl-2-fluoro-5,6,9,10,11,12-hexahydro-4H,8H-azépino[4',5':4,5]pyrrolo[3,2,1-ij]quinoléine-4-one ;
la 10 benzoyl-2-chloro-5,6,9,10,11,12-hexahydro-4H,8H-azépino[4',5':4,5]pyrrolo[3,2,1-ij]quinoléine-4-one ;
la 10 benzoyl-6-méthyl-5,6,9,10,11,12-hexahydro-4H,8H-azépino[4',5':4,5]pyrrolo[3,2,1-ij]quinoléine-4-one ;
la 10 benzoyl-2-fluoro-6-méthyl-5,6,9,10,11,12-hexahydro-4H,8H-azépino[4',5':4,5]pyrrolo[3,2,1-ij]quinoléine-4-one ;
la 10 benzoyl-2,3-dichloro-6-méthyl-5,6,9,10,11,12-hexahydro-4H,8H-azépino[4',5':4,5]pyrrolo[3,2,1-ij]quinoléine-4-one ;
la 10 benzoyl-6-propyl-5,6,9,10,11,12-hexahydro-4H,8H-azépino[4',5':4,5]pyrrolo[3,2,1-ij]quinoléine-4-one ;
la 10 benzoyl-6-(trifluorométhyl)-5,6,9,10,11,12-hexahydro-4H,8H-azépino[4',5':4,5]pyrrolo[3,2,1-ij]quinoléine-4-one ;
le 10 benzoyl-5,6,8,9,10,11,12,12a-octahydro-4H,7aH-azépino[4',5':4,5]pyrrolo[3,2,1-ij]quinoléine-4-ol ; et
la 10 benzoyl-4-méthoxy-5,6,8,9,10,11,12,12a-octahydro-4H,7aH-azépino[4',5':4,5]pyrrolo[3,2,1-ij]quinoléine.

49. Procédé pour la préparation d'un composé de formule (I) dans laquelle R₂ représente un atome d'hydrogène, comprenant la suppression de la protection d'un composé correspondant de formule (I) dans laquelle R₂ représente un groupe protecteur.

50. Procédé suivant la revendication 49, dans lequel le groupe protecteur est un groupe benzyloxycarbonyle ou benzoyle.
